# EUROPEAN PATENT SPECIFICATION

(11) **EP 4 147 053 B1**
(45) Date of publication and mention of the grant of the patent: **28.01.2026**
(21) Application number: 21723277.6
(22) Date of filing: 07.05.2021
(51) Int. Cl.: G01N 33/574, C07K 16/00

(54) **ANTXR1 AS A BIOMARKER OF IMMUNOSUPPRESSIVE FIBROBLAST POPULATIONS AND ITS USE FOR PREDICTING RESPONSE TO IMMUNOTHERAPY**
ANTXR1 ALS BIOMARKER VON IMMUNOSUPPRESSIVEN FIBROBLASTPOPULATIONEN UND DESSEN VERWENDUNG ZUR VORHERSAGE DER REAKTION AUF EINE IMMUNTHERAPIE
ANTXR1 ENTANT QUE BIOMARQUEUR DES POPULATIONS DE FIBROBLASTES IMMUNOSUPPRESSEURS ET SON UTILISATION POUR LA PRÉDICTION DE LA RÉPONSE À L'IMMUNOTHÉRAPIE

(30) Priority: 07.05.2020 EP 20305454
(43) Date of publication of application: 15.03.2023
(60) Divisional application: 25225280.4
(73) Proprietor: Institut Curie, 75248 Paris Cedex 05 (FR); Institut National de la Santé et de la Recherche Médicale (INSERM), 75013 Paris (FR)
(72) Inventor: MECHTA-GRIGORIOU, Fatima, 94100 SAINT MAUR DES FOSSES (FR); KIEFFER, Yann, 94700 MAISONS-ALFORT (FR); VINCENT-SALOMON, Anne, 75013 PARIS (FR); HOCINE, Rachid, Hocine, MANHATTAN, NEW YORK 10021 (US)
(74) Representative: Cabinet Becker et Associés
(86) International application number: PCT/EP2021/062090
(87) International publication number: WO 2021/224438

(56) References cited:
- CHRISTOPHER SZOT ET AL: "Tumor stroma-targeted antibody-drug conjugate triggers localized anticancer drug release", THE JOURNAL OF CLINICAL INVESTIGATION, vol. 128, no. 7, 4 June 2018 (2018-06-04), GB, pages 2927 - 2943, XP055724470, ISSN: 0021-9738, DOI: 10.1172/JCI120481
- ANA COSTA ET AL: "Fibroblast Heterogeneity and Immunosuppressive Environment in Human Breast Cancer", CANCER CELL, vol. 33, no. 3, 1 March 2018 (2018-03-01), US, pages 463 - 479.e10, XP055724537, ISSN: 1535-6108, DOI: 10.1016/j.ccell.2018.01.011
- ANNE-MARIE GIVEL ET AL: "miR200-regulated CXCL12[beta] promotes fibroblast heterogeneity and immunosuppression in ovarian cancers", NATURE COMMUNICATIONS, vol. 9, no. 1, 13 March 2018 (2018-03-13), XP055724717, DOI: 10.1038/s41467-018-03348-z

## Description

### Field of the Invention

The present invention relates to the field of medicine, in particular of oncology. It provides new markers for immunosuppressive cell populations and their uses.

### Background of the Invention

With 9.6 million deaths in 2018, cancer is the second leading cause of death worldwide. The prevalence of cancer is also extremely high as more than 15 million new cases are diagnosed each year, and the number of new cases is expected to rise by about 70% over the next 2 decades.

Many treatment options exist nowadays for cancer, including for example surgery, chemotherapy, radiation therapy, hormonal therapy, targeted therapy and palliative care. Particular cancer treatments, such as antibody-drug conjugates (ADCs) have been described in the art (Szot et al., J Clin Invest. 2018;18(7):2927-2943). The choice of the best adapted treatment for a patient depends on the type, location and grade of the cancer as well as the patient's health and preferences.

In the last few decades, immunotherapy has become an important part of cancer treatment strategies. Cancer immunotherapy relies on the use of the immune system to treat cancer. Among the diversity of immunotherapy treatments that have been developed over time, immune checkpoint inhibitor therapies are particularly promising. However, despite encouraging results, many advanced cancer patients do not respond to immune checkpoint inhibitors, and little is known about the mechanisms of primary resistance. Particularly, some cancers develop immunosuppressive microenvironments that are responsible of the development of resistance to immunotherapy.

Identification of biomarkers that may reliably discriminate responder and non-responder patients before initiating therapy is hence needed to select patients who are likely to benefit from immuno-oncology drugs.

Cancer-Associated Fibroblasts (CAFs) are abundant components in cancer and play key pro-tumorigenic functions. It is now recognized that CAFs are heterogeneous and that distinct CAFs subsets can be defined based on expression of specific markers. For now, 4 CAFs subsets, referred to as CAF-S1 to -S4, have been identified in human breast and ovarian cancers (Costa A, et al. Cancer Cell 2018;33(3):463-79 e10, and Givel et al., Nature Communications (2018) 9:1056). While CAF-S2 and CAF-S3 fibroblasts are also detected in healthy tissues and could be reminiscent of normal fibroblasts, CAF-S1 and CAF-S4 myofibroblasts cells are restricted to cancer and metastatic lymph nodes. Both CAF-S1 and CAF-S4 promote metastases through complementary mechanisms. In contrast, while CAFs-S1 promote immunosuppression in human cancer, CAFs-S4 do not. CAFs subpopulations remain heterogeneous and there is thus a need to identify CAFs having specific roles in primary resistance to immunotherapy in cancer patients, in particular to identify new markers for detecting immunosuppressive CAFs in cancers.

There is also a persistent need to develop new strategies to overcome immunosuppressive environments, thereby making immunotherapy, and in particular immune checkpoint inhibitor treatments, more effective and available for all patients.

The present invention seeks to meet these and other needs.

### Summary of the invention

Cancer is a systemic disease encompassing multiple components of both tumor cells themselves and host stromal cells. It is now clear that stromal cells in a tumor microenvironment play an important role in cancer development. Cancer stroma includes fibroblasts, in particular Cancer Associated Fibroblasts (CAFs), vascular endothelial cells, immune cells and the extracellular matrix. CAFs are the most abundant component of tumor stroma, they make up the bulk of cancer stroma and affect tumor microenvironment such that they promote cancer initiation, angiogenesis, invasion and metastasis.

The inventors discovered new subpopulations of CAFs that play crucial roles in the establishment of an immunosuppressive microenvironment at the tumor site. The inventors used scRNA-seq to address the heterogeneity of the CAF-S1 immunosuppressive subpopulation and identified 8 different CAF-S1 clusters. Among them, 3 CAF-S1 clusters (1, 2, 5) belong to the inflammatory ("iCAF") subgroup and 5 CAF-S1 clusters (0, 3, 4, 6, 7) to the myofibroblastic ("myCAF") subgroup, iCAF and myCAF subgroups having been previously described in pancreatic cancer (Ohlund D, et al. J Exp Med 2017;214(3):579-96).

The 8 CAF-S1 clusters identified by the inventors are characterized by high expression of genes coding extra-cellular matrix (ECM) proteins (cluster 0), detoxification pathway (cluster 1), interleukin-signaling (cluster 2), Transforming Growth Factor β (TGFβ) signaling pathway (cluster 3), wound healing (cluster 4), interferon y (cluster 5), interferon αβ (cluster 6) and acto-myosin pathway (cluster 7). Accordingly, the inventors annotated them as follows: ecm-myCAF (cluster 0), detox-iCAF (cluster 1), IL-iCAF (cluster 2), TGFβ-myCAF (cluster 3), wound-myCAF (cluster 4), IFN y -iCAF (cluster 5), IFN αβ -myCAF (cluster 6) and acto-myCAF (cluster 7).

The existence of these CAF-S1 clusters were validated in head and neck squamous cell carcinoma (HNSCC) and in non-small cell lung cancer (NSCLC) by analyzing publicly available scRNA-seq data, demonstrating the relevance of these CAF-S1 clusters across different cancer types.

In addition, the inventors uncovered that the abundance of two CAF-S1 clusters of the myCAF subgroup, namely ecm-myCAF (cluster 0) and TGFβ-myCAF (cluster 3), is significantly correlated with an immunosuppressive environment, whereas the content in detox-iCAF and IL-iCAF is not.

Indeed, ecm-myCAF (cluster 0) and TGFβ-myCAF (cluster 3) clusters are enriched in tumors with high levels of PD-1⁺, CTLA-4⁺ and TIGIT⁺ CD4⁺ T lymphocytes (themselves enriched in Tregs), and low fraction of CD8⁺ T lymphocytes. Interestingly, ecm-myCAF (cluster 0) specific signature contains the LRRC15 gene that has been recently identified as determinant of patient response to immunotherapy in pancreatic cancer (Dominguez et al., Cancer Discov 2020;10(2):232-53).

In contrast to ecm-myCAF (cluster 0) and TGFβ-myCAF (cluster 3), wound-myCAF (cluster 4) are not associated with an immunosuppressive environment, but correlated with a high global infiltration by T lymphocytes. Hence, as the wound-myCAF cluster (cluster 4) is enriched in tumors from patients who do not respond to immunotherapy, it might serve as a new surrogate marker of primary resistance to immunotherapies in highly infiltrated tumors, which are usually sensitive to this type of treatment. Thus, assessing the content in specific CAF-S1 clusters in tumors at time of diagnosis provide an additive value for predicting primary resistance to immune checkpoint inhibitors.

The inventors particularly show that, at diagnosis, these specific CAF-S1 clusters are associated with primary resistance to immunotherapies in both melanoma and NSCLC patients. In particular, ecm-myCAFs (cluster 0) increase the fraction of FOXP3^{high} T cells and stimulate both PD-1 and CTLA-4 protein levels at the surface of CD4⁺ CD25⁺ T lymphocytes, which in turn increase the proportion of TGFβ-myCAF (cluster 3). This uncovers a positive feedback loop between the immunosuppressive ecm-myCAF (cluster 0) and TGFβ-myCAF (cluster 3) CAF-S1 clusters and Tregs that promote immunosuppression and is involved in resistance to immunotherapies. These data thus support the development of strategies combining PD-1 and/or CTLA-4 blockade with therapies targeting specific CAF-S1 clusters components to overcome primary resistance to immune checkpoint blockade.

Accordingly, the inventors' data support the identification of a new subset of patients having ANTXR1⁺FAP⁺ CAFs in a tumor sample, these patients showing an immunosuppression and having a resistance or a low response to immunotherapies. Therefore, in this particular subset of patients, an agent targeting ANTXR1⁺ CAFs so as to suppress or reduce the immunosuppressive action of ANTXR1⁺ FAP⁺ CAFs can lead to a therapeutic benefit. In addition, the combination of this agent with an immunotherapeutic agent may prevent the occurrence of a resistance to the immunotherapeutic agent or restore the response. In addition, even if FAP is expressed by all CAF-S1 cells, ANTXR1⁺ FAP⁺ patients will have a greater therapeutic benefit of a treatment with a FAP inhibitor, as ANTXR1⁺ FAP⁺ CAF are immunosuppressive and associated with immunotherapy resistance, while ANTXR1⁺ FAP⁻ CAF are not. In this context, the benefit/risk balance is in favor of this treatment in this particular subset of patients, especially in combination with an immunotherapy.

Finally, the inventors identified ANTXR1 as a specific marker of CAF-S1 clusters 0, 3 and 4, allowing to identify CAF associated with immunosuppressive effect.

The object of the present invention is defined by the claims.

In a first aspect, the invention concerns an *in vitro* method for detecting immunosuppressive fibroblasts in a cancer sample from a subject suffering from cancer, wherein the method comprises detecting Anthrax Toxin Receptor 1 (ANTXR1) and Fibroblast Activation Protein (FAP) positive fibroblasts in the cancer sample from said patient, the presence of ANTXR1⁺ fibroblasts being indicative of immunosuppressive CAF.

In a second aspect, the invention concerns an *in vitro* method for predicting a response of a subject suffering from cancer to an immunotherapeutic agent, wherein the method comprises detecting ANTXR1⁺FAP⁺ fibroblasts in a cancer sample from said patient, wherein ANTXR1⁺FAP⁺ fibroblasts in the cancer sample are predictive of the responsiveness of said patient to an immunotherapeutic agent.

In a third aspect, the invention concerns an immune checkpoint inhibitor for use in the treatment of a cancer in a patient, wherein the patient presents a cancer sample having (a) low number or percentage of ANTXR1⁺FAP⁺ fibroblasts; or (b) no ANTXR1⁺FAP⁺ fibroblasts.

In particular, the method for predicting the response of a subject to the immunotherapeutic agent further comprises determining the percentage of ANTXR1⁺FAP⁺ fibroblast and the percentage of ANTXR1⁺FAP⁺ fibroblast is the number of ANTXR1⁺FAP⁺ fibroblasts cells on the total number of fibroblasts in the cancer sample or on the total number of cells in the cancer sample.

Preferably, the ANTXR1⁺ FAP⁺ fibroblasts are FAP⁺ANTXR1⁺ Cancer associated fibroblasts (CAFs).

Even more preferably, the ANTXR1⁺ fibroblasts are selected from the group consisting of FAP⁺ ANTXR1⁺ LAMP5⁻ SDC1⁺ fibroblasts, preferably CAFs; FAP⁺ ANTXR1⁺ LAMP5⁺ SDC1^{+/-} fibroblasts, preferably CAFs; and FAP⁺ANTXR1⁺ SDC1⁻ LAMP5⁻ CD9⁺ fibroblasts, preferably CAFs.

In a fourth aspect, the invention concerns the use of ANTXR1 as a biomarker for the identification of immunosuppressive FAP⁺CAFs. The invention also concerns the use of ANTXR1 in FAP⁺CAFs population, as a biomarker in a tumor of a subject suffering from cancer, for predicting the response of the subject to an immunotherapy.

In a fifth aspect, the invention concerns a FAP inhibitor for use in the treatment of a cancer in a patient, wherein the patient has ANTXR1⁺FAP⁺ fibroblasts, preferably CAFs, in a tumor sample from the patient, wherein the FAP inhibitor is selected in the group consisting of talabostat, PT-100, linagliptin (Tradjenta), FAP inhibitors with a N-(4-quinolinoyl)-Gly-(2-cyanopyrrolidine) scaffold, such as FAPI-02, FAPI-04 and FAPI-46, a peptide-targeted radionuclide such as FAP-2286 and any combination thereof. In a particular aspect, the FAP inhibitor is for use in combination with an immunotherapeutic agent.

In particular, the immunotherapeutic agent is selected from the group consisting of therapeutic treatments that stimulate the patient's immune system to attack the malignant tumor cells, immunization of the patient with tumoral antigens, molecules stimulating the immune system such as cytokines, therapeutic antibodies, adoptive T cell therapy, CAR cell therapy, immune checkpoint inhibitor, and any combination thereof, preferably an immune checkpoint inhibitor.

Preferably, the checkpoint inhibitor is selected from the group consisting of an antibody directed against cytotoxic T lymphocyte associated protein (CTLA-4), programmed cell death protein 1 (PD-1), programmed cell death ligand (PD-L1), T cell immunoreceptor with Ig and ITIM domains (TIGIT), Lymphocyte-activation gene 3 (LAG-3), T-cell immunoglobulin and mucin-domain containing-3 (TIM-3), Band T-lymphocyte attenuator (BLTA), IDO1, or any combination thereof, preferably selected from the group consisting of an antibody directed against CTLA-4, PD-1, PD-L1 and TIGIT or any combination thereof, more preferably an antibody directed against PD-1 or CTLA-4 and the combination thereof. Particularly, the immunotherapeutic agent is selected from the group consisting of ipilimumab, nivolumab, BGB-A317, pembrolizumab, atezolizumab, avelumab, or durvalumab, BMS-986016, and epacadostat, or any combination thereof.

In particular, the cancer is selected from the group consisting of prostate cancer, lung cancer, Non-Small Cell Lung Carcinoma (NSCLC), breast cancer, gastric cancer, kidney cancer, ovarian cancer, hepatocellular cancer, osteosarcoma, melanoma, hypopharynx cancer, esophageal cancer, endometrial cancer, cervical cancer, pancreatic cancer, liver cancer, colon or colorectal cancer, adenocarcinoma, sarcoma, non-Squamous cell carcinoma, Squamous cell carcinoma, neuroendocrine tumors, muscle cancer, adrenal cancer, thyroid cancer, uterine cancer, skin cancer, melanoma, metastatic melanoma, bladder cancer, head and neck cancer, preferably the cancer is selected from the group consisting of head and neck cancer, breast cancer, ovary cancer, NSCLC, melanoma and metastatic melanoma, more preferably the cancer is selected from the group consisting of head and neck cancer, NSCLC, melanoma and metastatic melanoma.

### Brief Description of the Drawings

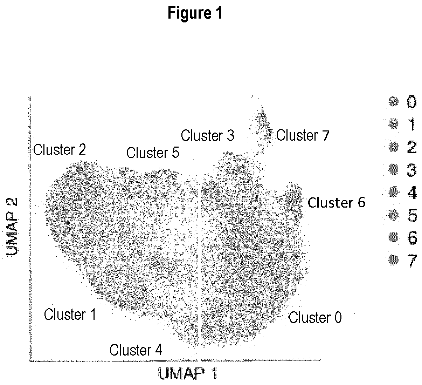

**Figure 1****:** Identification of distinct cellular clusters in CAF-S1 fibroblasts. Uniform Manifold Approximation and Projection (UMAP) of 18 296 CAF-S1 fibroblasts across 7 BC patients allowing the visualization of 8 CAF-S1 clusters (0 to 7). Colors show the different CAF-S1 clusters defined by graph-based clustering method applied on the space defined by the 30 first principal components.

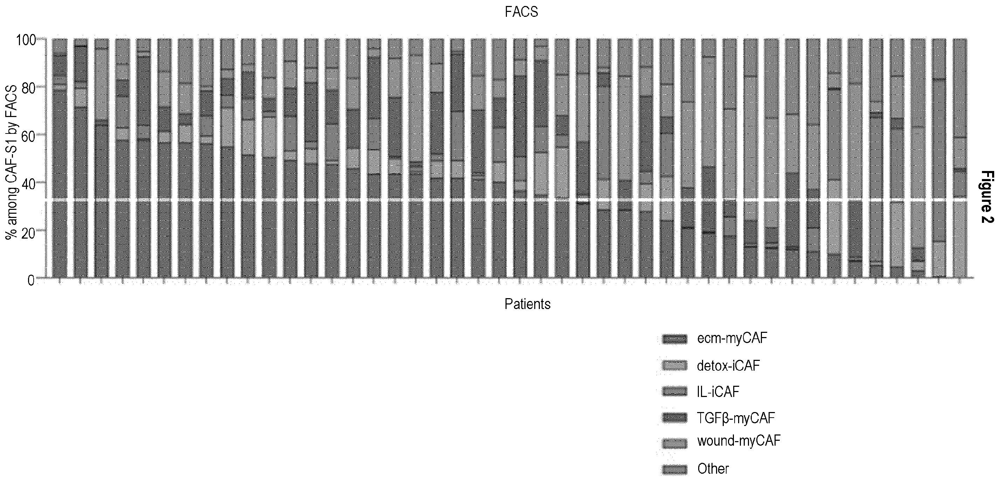

**Figure 2****:** Validation of the 5 most abundant CAF-S1 clusters in distinct cancer types. Percentage (%) of the different clusters among CAF-S1 fibroblasts based on FACS data. Each bar represents one patient (N = 44).

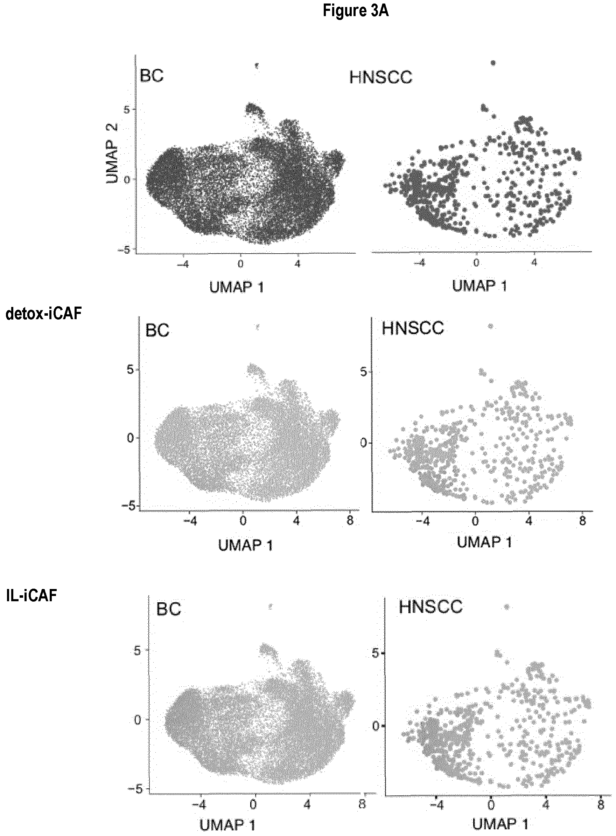

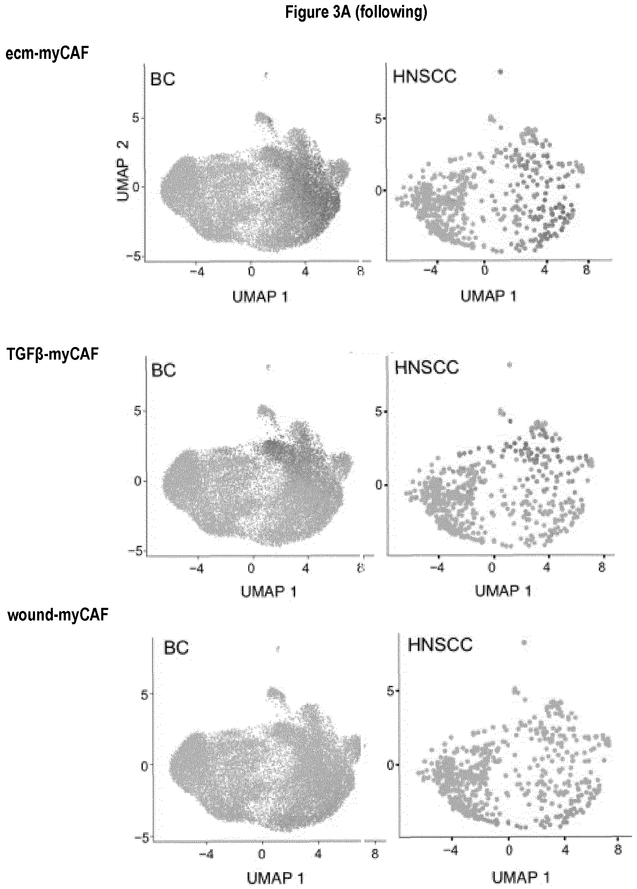

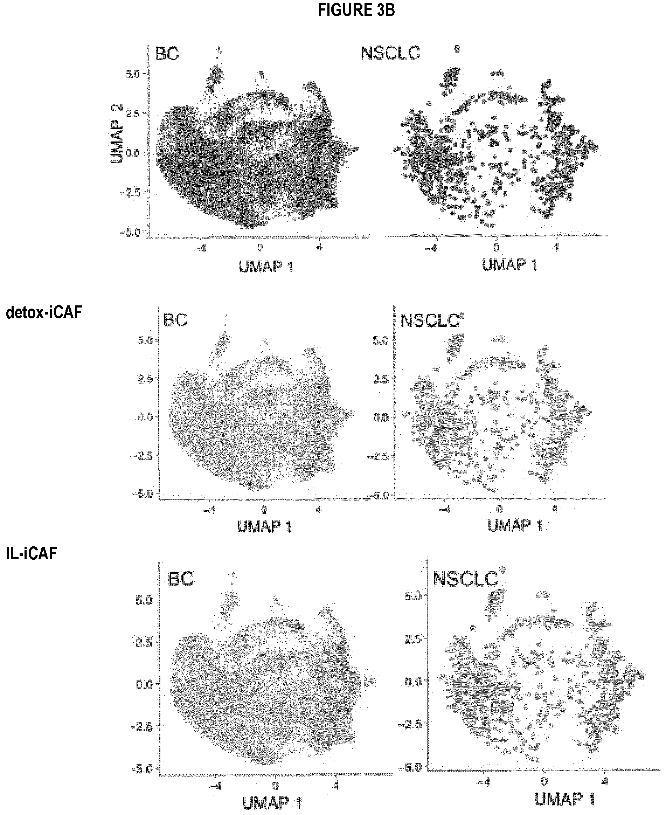

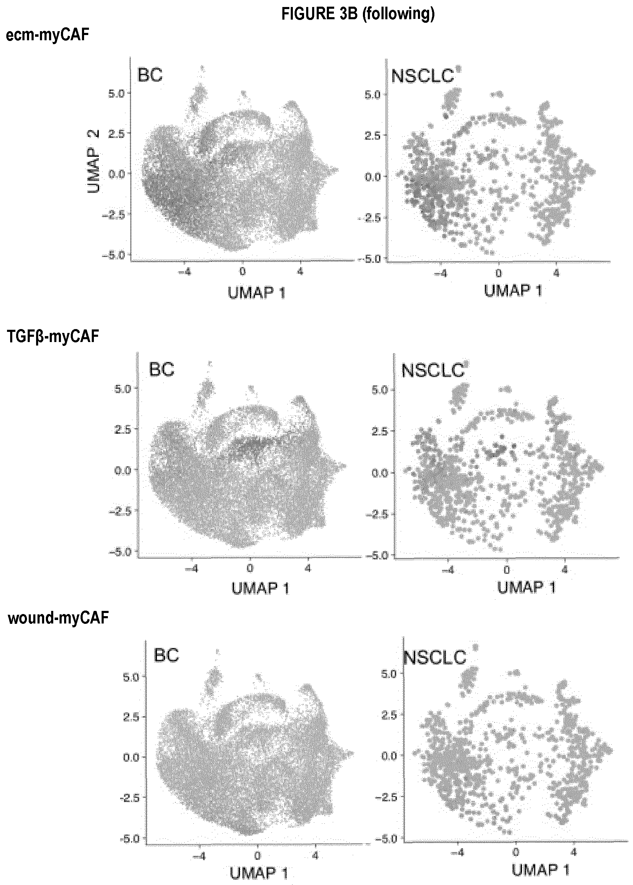

**Figure 3****:** Detection of the CAF-S1 cellular clusters in lung and head and neck cancers. **(A)** UMAP plots combining 18 296 CAF-S1 fibroblasts from BC (in red, upper left panel) and FAP⁺ fibroblasts from HNSCC (data from (30), n = 603 FAP⁺ cells, in blue, upper left panel). A score, calculated as the average z-score of genes that compose specific signature of each CAF-S1 cluster, is applied. **(B)** Same as in **(A)** from NSCLC (data from (31), n = 959 FAP⁺ cells, in blue, upper left panel).

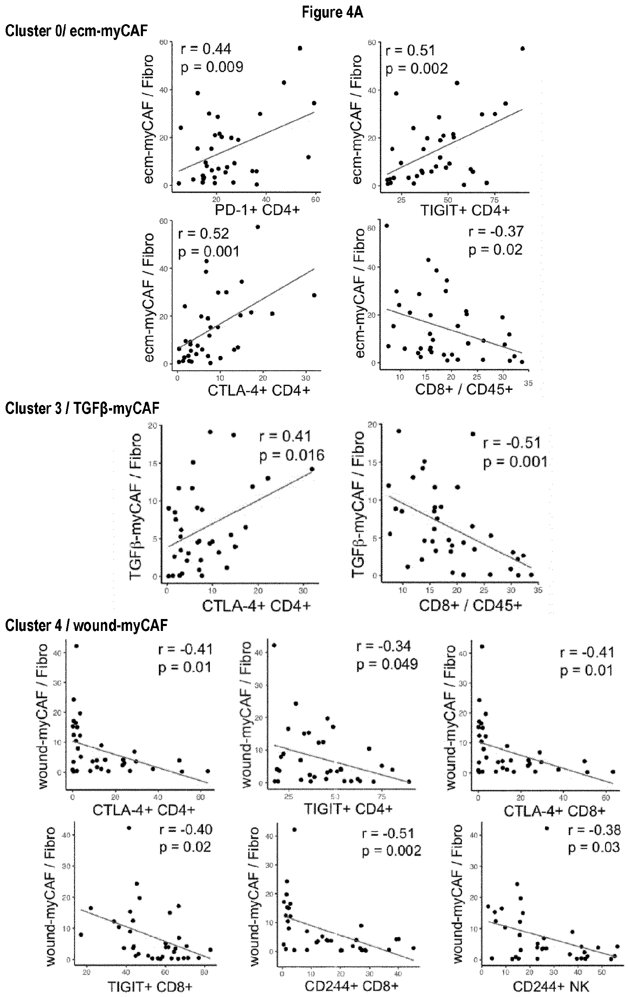

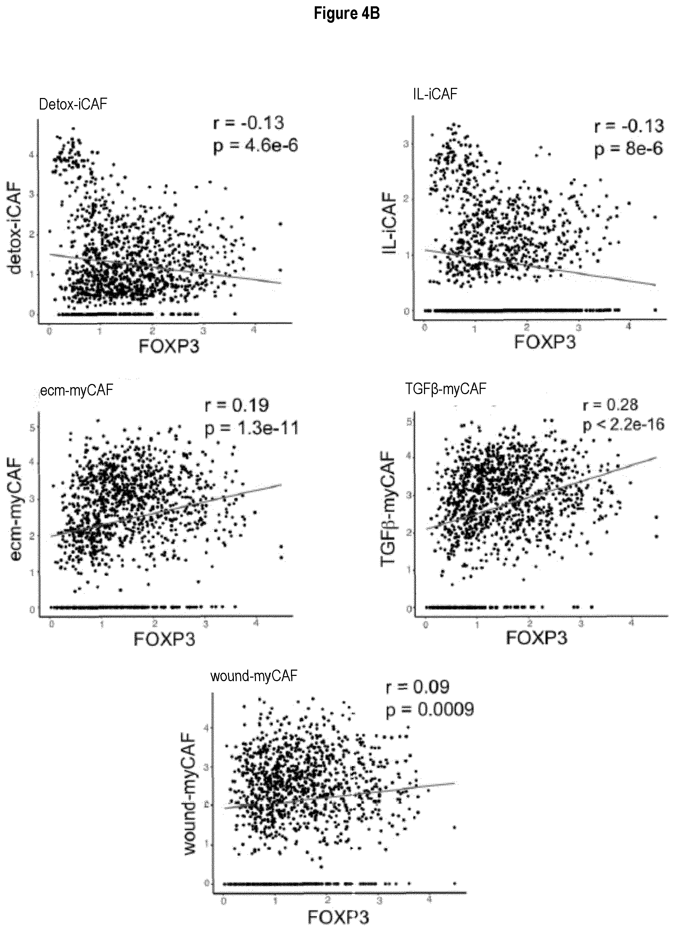

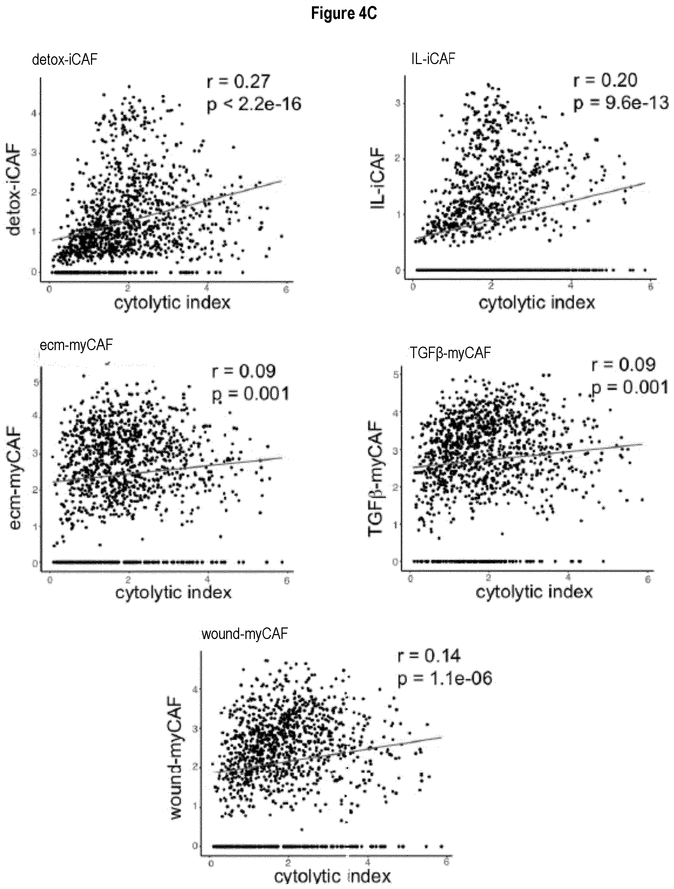

**Figure 4****:** Correlation between CAF-S1 clusters and immune cells in breast cancers. **(A)** Detailed correlation curves between 2 variables, as indicated. Each dot represents one tumor (N = 37). P values from Pearson correlation test. **(B)** Correlation curves between CAF-S1 cluster signatures and FOXP3 in TCGA cohort. Each dot represents one tumor (N = 1221). P values from Pearson correlation test. **(C)** Same as in **(B)** between CAF-S1 cluster signatures and cytolytic index, as defined in Rooney et al., Cell 2015; 160(1-2):48-61.

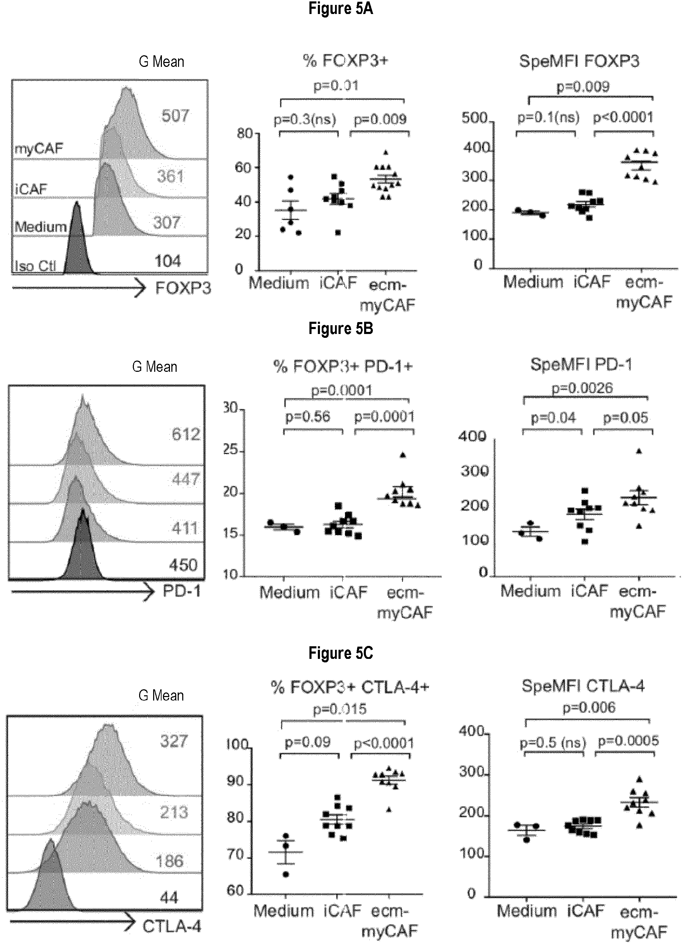

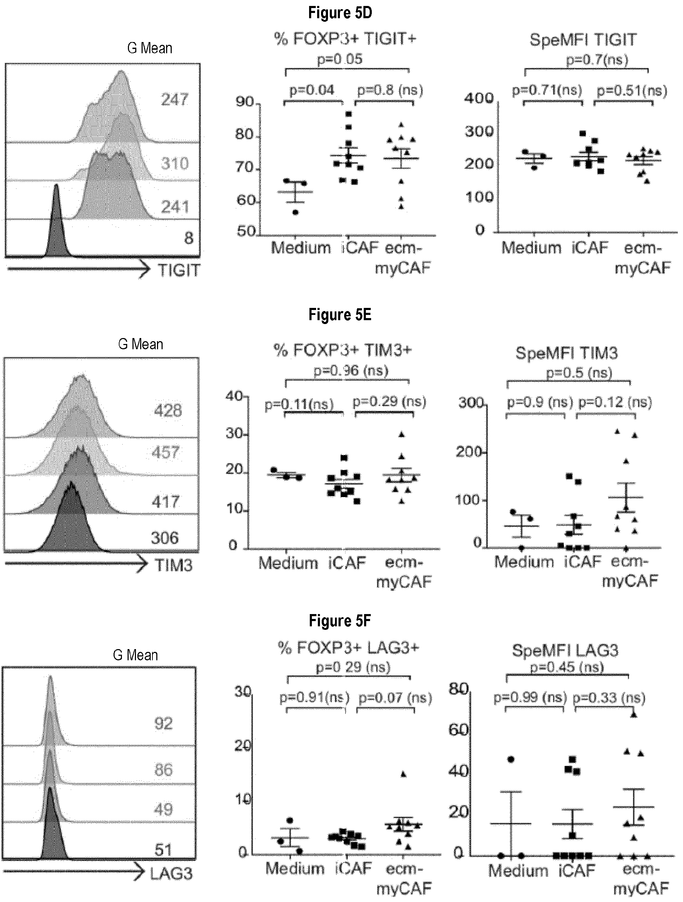

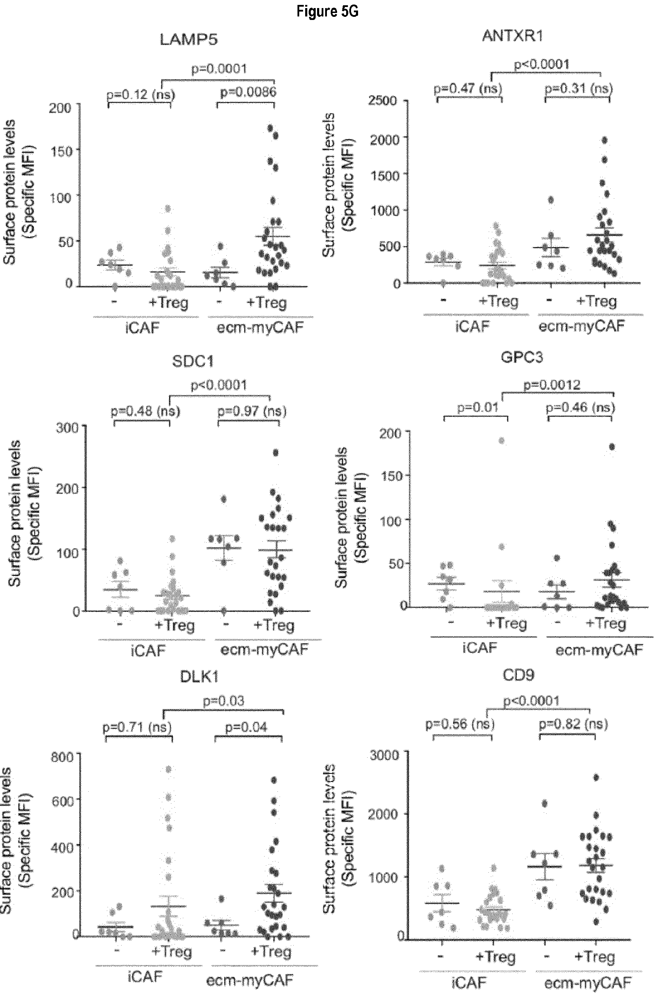

**Figure 5****:** Reciprocal effects of CAF-S1 clusters and Tregs. **(A)** Representative histograms of FOXP3 specific mean fluorescent intensity (speMFI) (Left) either alone (green) or in presence of iCAF (orange) or ecm-myCAF (red). After 24 hours (h) of co-culture with a 10:1 ratio (T:CAF-S1), percentage of FOXP3⁺ cells (middle) and FOXP3 protein level in CD4⁺ CD25⁺ T cells (right) were assessed. P values from Welch's t test (N = 7 CAF-S1 primary cell lines per condition; n = 3 independent experiments). **(B-G)** Same as in **(A)** for the following immune checkpoints, PD-1 **(B),** CTLA-4 **(C),** TIGIT **(D),** TIM3 **(E)** and LAG3 **(F)** in FOXP3⁺ CD4⁺ CD25⁺ Tregs. P values from Welch's t-test. (N = 7 CAF-S1 cell lines per condition; n = 3 independent experiments). **(G)** Impact of CD4⁺ CD25⁺ T lymphocytes on CAF-S1 cluster identity. Dot plots showing protein levels of CAF-S1 clusters markers at the surface of CAF-S1 primary cell lines. For each marker, surface protein level is expressed as specific MFI, calculated as followed: Specific MFI = MFI from the specific antibody - MFI from the isotype control, in absence (-) or presence (+) of CD4⁺ CD25⁺ T cells (N = 7 CAF-S1 cell lines per condition; n = 3 independent experiments). P values from Mann-Whitney test.

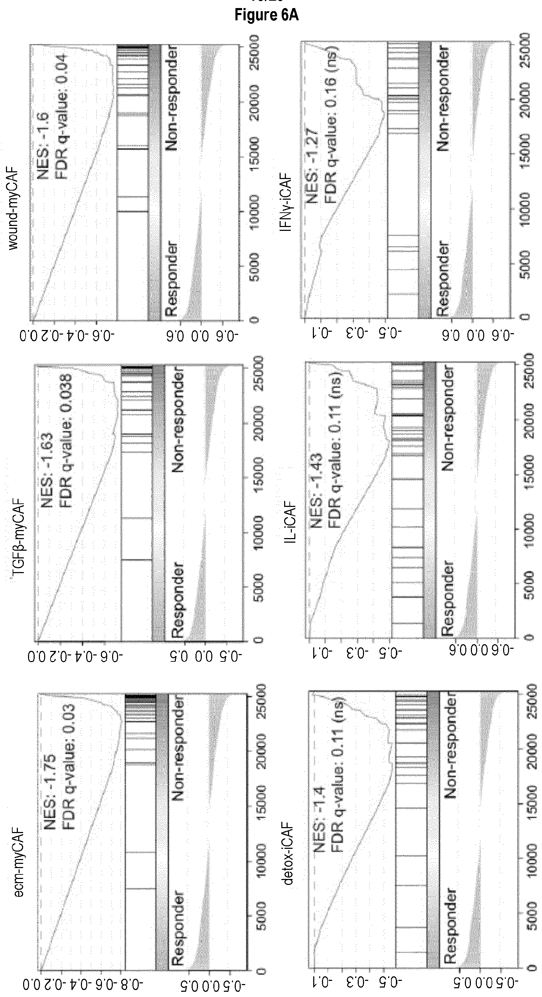

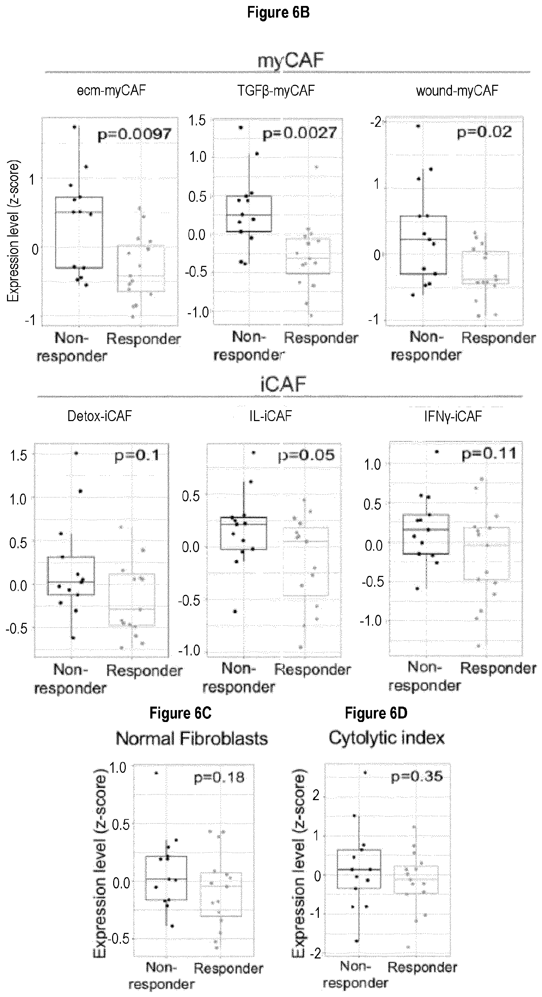

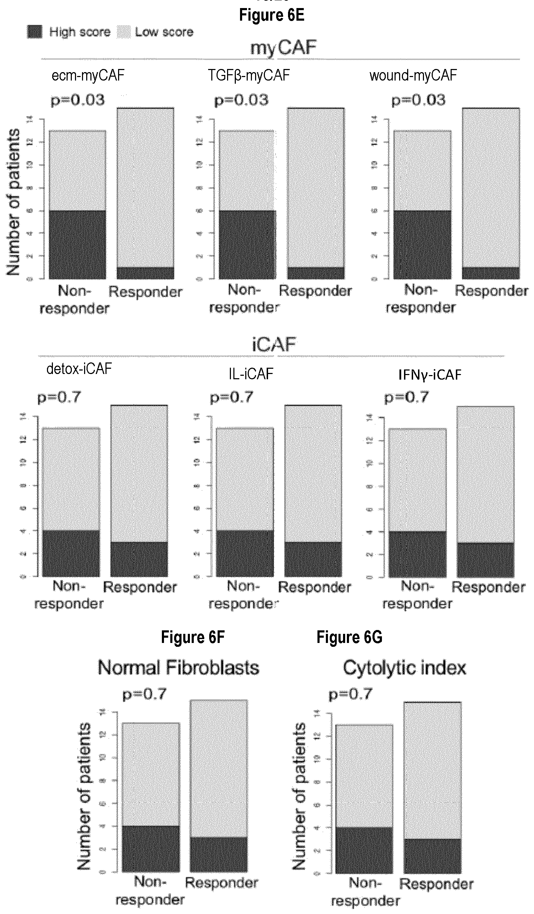

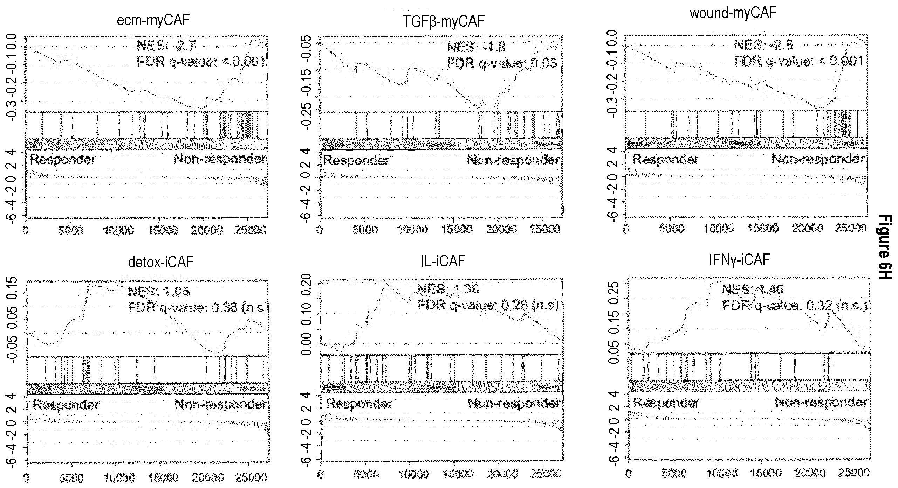

**Figure 6****:** Impact of CAF-S1 clusters on resistance to immunotherapies. **(A)** Gene Set Enrichment Analysis (GSEA) applied on RNA-Seq data from 28 melanoma tumors before anti-PD-1 treatment by using specific signatures from each CAF-S1 cluster showing significant enrichment of CAF-S1 gene signature (top 100 genes) in non-responding (N = 13) patients, compared to responding (N = 15) patients. Cohort from (33).
Down, Same as (Up) for normal fibroblast signature). GSEA analysis shows that clusters 0 (ecm-myCAF), 3 (TGFβ-myCAF) and 4 (wound-myCAF) are significantly associated with non-responders (Up), while clusters 1 (detox-iCAF), 2(IL-iCAF) and 5 (IFN-iCAF) are not (Down). **(B)** Expression assessed by the average z-score of each CAF-S1 cluster signature in responders and non-responders melanoma patients. **(C, D)** Same as in **(B)** using normal fibroblast signature and cytolytic index. **(E)** Responders and non-responders stratified in low- and high-CAF-S1 cluster expression (based on the third quartile of CAF cluster z-score). **(F, G)** Same as in (E) using normal fibroblast signature and cytolytic index. **(H)** Same as in **(A)** analyzing NSCLC cohort of patients.

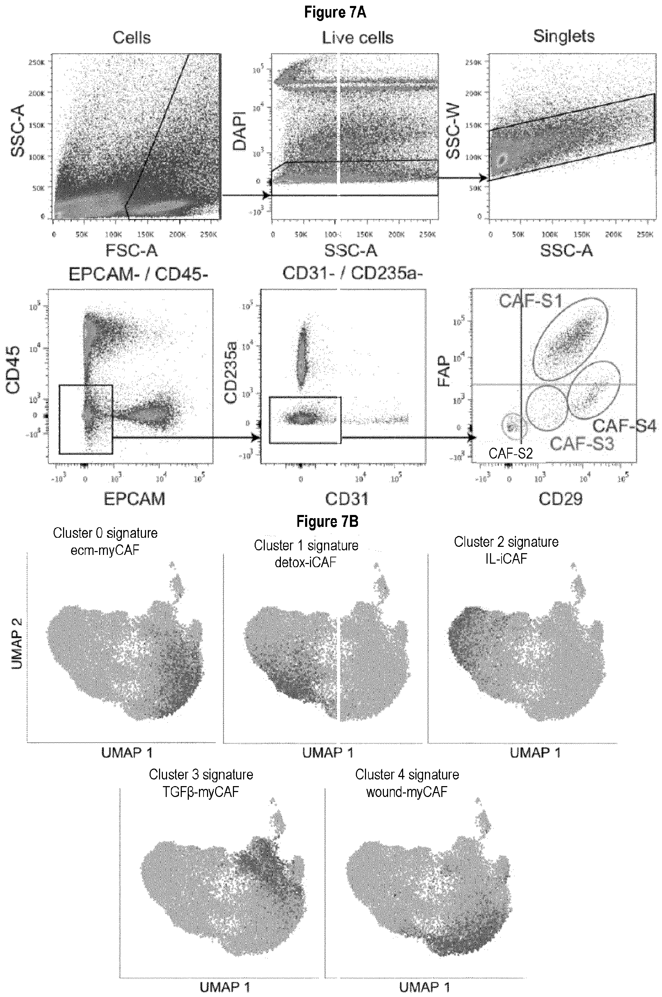

**Figure 7****.** Identification of CAF-S1 clusters. (A) Representative FACS plots showing the gating strategy used to isolate CAF-S1. Cells are first gated on DAPI- EPCAM- CD45- CD31- CD235a- to exclude dead, epithelial, hematopoietic, endothelial and red blood cells, respectively. FAPHigh CD29Med cells are then selected as CAF-S1 fibroblasts. (B) UMAP plots of 18 296 CAF-S1 fibroblasts (as in Fig. 1) showing average z-score of specific gene signatures of the 5 most abundant CAF-S1 clusters.

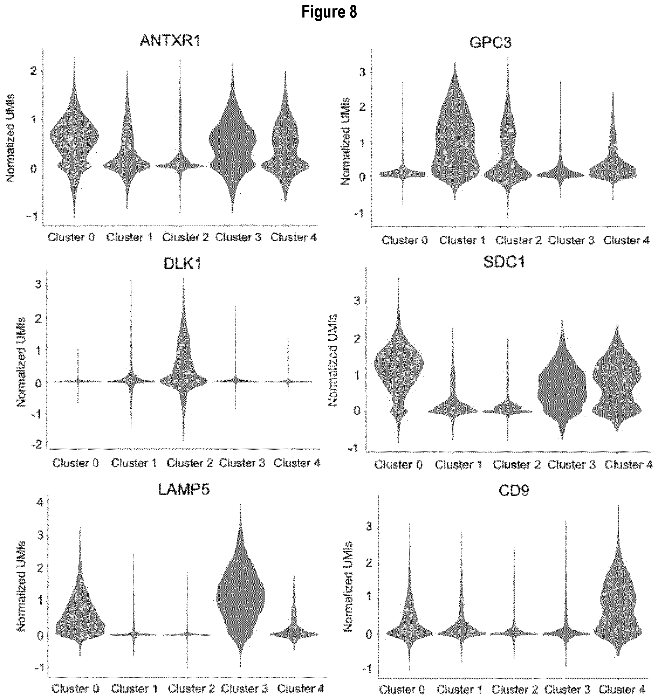

**Figure 8****.** Identification of markers of CAF-S1 clusters for FACS analysis and selection of CAF-S1 from HNSCC and NSCLC scRNA-seq data. Violin plots showing the distribution of expression (RNA levels from scRNA-seq from 18 296 CAF-S1 fibroblasts from 7 BC patients) of 6 genes encoding surface markers specific of each of the 5 most abundant CAF-S1 clusters.

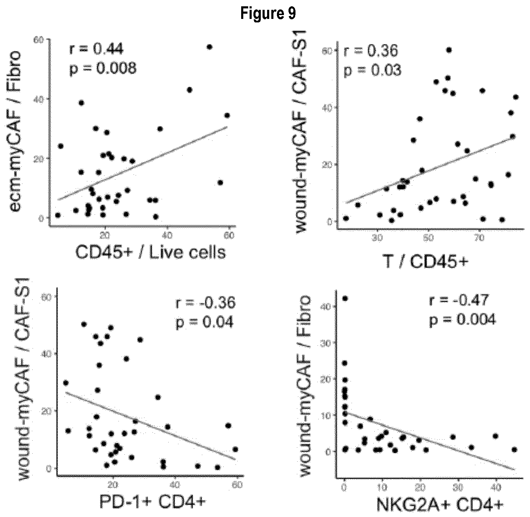

**Figure 9****.** Correlation between CAF-S1 clusters and immune cells. Detailed correlation plots between CAF clusters and immune cells, as indicated (N = 37 BC). P values from Pearson correlation test.

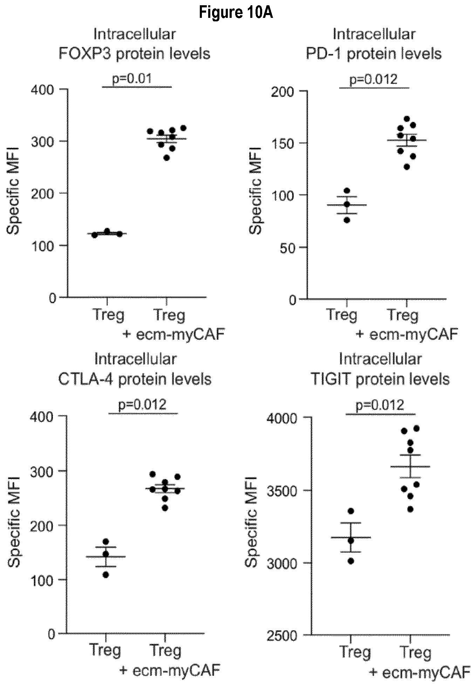

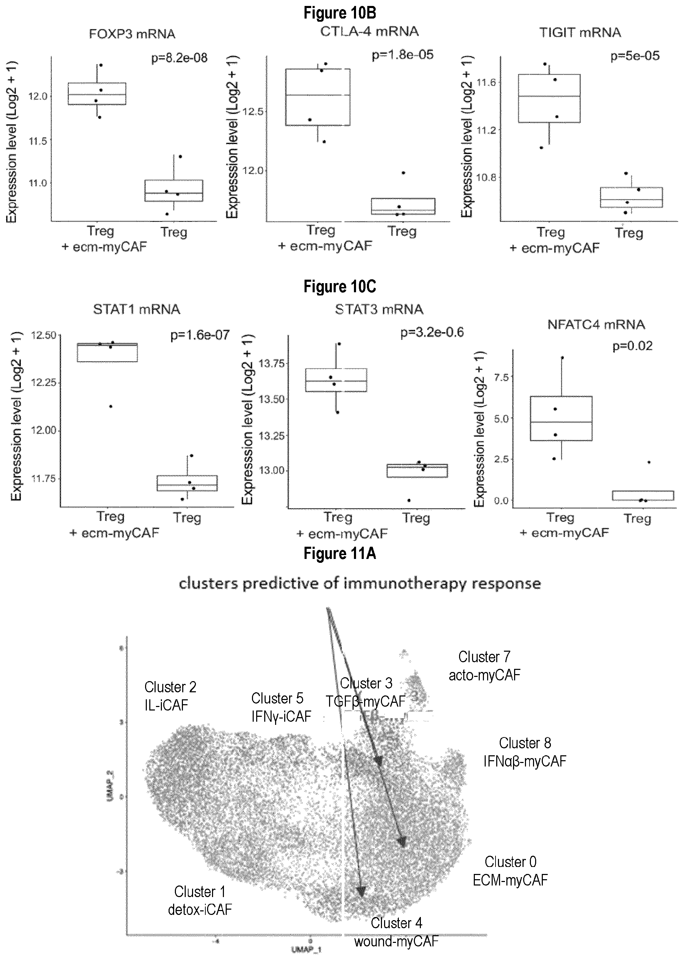

**Figure 10****.** Fibroblasts isolated from juxta-tumors by spreading and maintained in plastic dishes acquire CAF-S1 properties (A) Dot plots showing the intracellular specific mean fluorescent intensity (speMFI) FOXP3, PD-1, CTLA-4 and TIGIT in CD4+ CD25+ T cells cultured alone (Left) or in presence of ecm-myCAF (Right). P values from Mann-Whitney test (N = 4 CAF-S1 primary cell lines). (B) Boxplots showing FOXP3, CTLA-4 and TIGIT mRNA levels in CD4+ CD25+ T cells cultured alone (Right) or in presence of ecm-myCAF (Left). P values from DESeq2 analysis (N = 8). (C) Same as in (B) for STAT and NFAT family members.

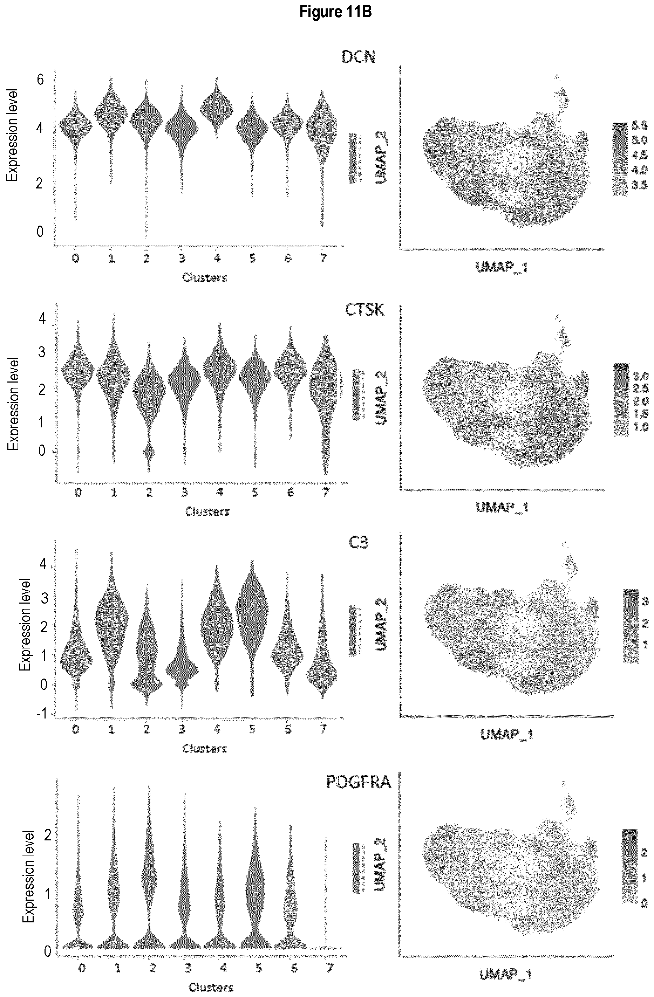

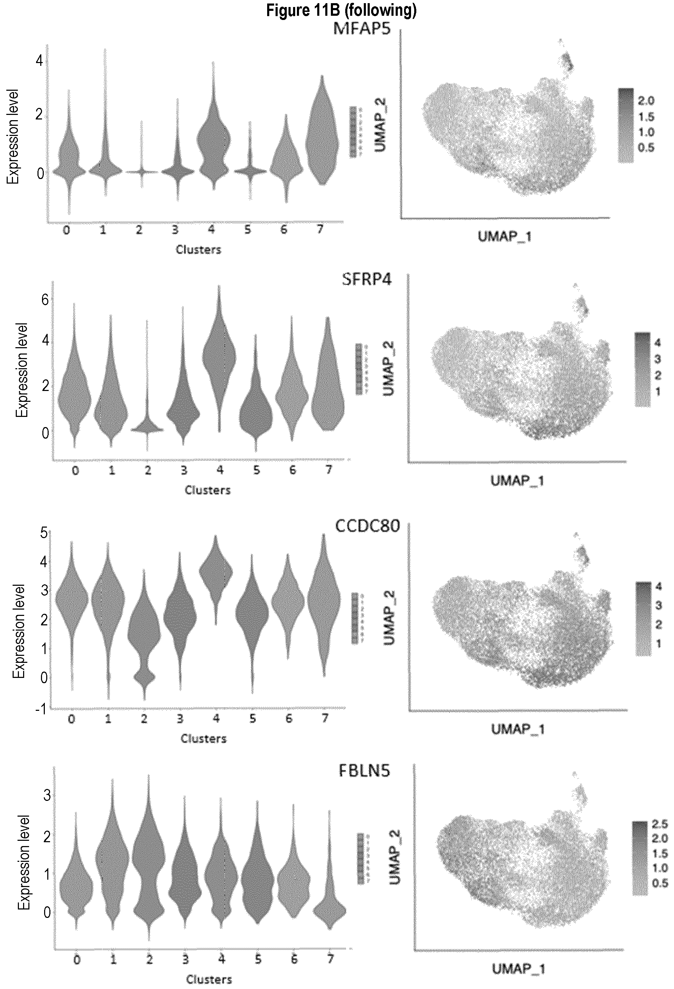

**Figure 11****.** (A) UMAP plots of 18 296 CAF-S1 fibroblasts (as in Fig. 1) allowing the visualization of 8 CAF-S1 clusters (0 to 7). The three predictive clusters of immunotherapy response, namely clusters 0 = ECM-myCAF, cluster 3 = TGFβ-myCAF and cluster 4 = wound-myCAF, are indicated with arrows. (B) Violin plots (left) and UMAP plots (right) showing some of the most differentially expressed genes defining the CAF-S1 signature. Expression of these genes in each CAF-S1 cluster is shown using violin plot and UMAP representations. Among these Top representative CAF-S1 genes, none of them are specifically expressed in clusters 0, 3 and 4 and thus predictive of immunotherapy response.

### Detailed description of the Invention

### Definitions

The term "cancer" or "tumor", as used herein, refers to the presence of cells possessing characteristics typical of cancer-causing cells, such as uncontrolled proliferation, and/or immortality, and/or metastatic potential, and/or rapid growth and/or proliferation rate, and/or certain characteristic morphological features. This term refers to any type of malignancy (primary or metastases) in any type of subject. It may refer to solid tumor as well as hematopoietic tumor.

The term "cancer sample", as used herein, means any sample containing tumor cells and cancer stromal cells, in particular fibroblasts such as Cancer Associated Fibroblasts (CAF), derived from a subject. Cancer tissues are particularly composed of cancer cells and the surrounding cancer stromal cells, including cancer associated fibroblasts (CAFs), vascular endothelial cells, and immune cells, in addition to the extracellular matrix. Preferably, the cancer sample contains nucleic acids and/or proteins. The sample may be treated prior to its use.

As used herein, the term "Cancer Associated Fibroblast" or "carcinoma associated fibroblasts" or "CAF" refers to the fibroblasts present in the stroma of cancers. They are one of the most abundant stromal components with morphology similar to myofibroblasts. CAFs are CD45⁻EpCAM⁻CD31⁻CD29⁺ stromal cells. As used herein the term "immunosuppressive fibroblast" refers to fibroblast that is responsible and/or contribute to immunosuppression in a tumor, i.e. to the partial or complete inhibition or suppression of the immune response of an individual towards cancer cells. Optionally, the fibroblast can be malignant, especially in a sarcoma, or normal.

As used herein the term "immunosuppressive Cancer Associated Fibroblast" or "immunosuppressive CAF" refers to CAF that is responsible and/or contribute to immunosuppression or to an immunosuppressive microenvironment in a tumor, i.e. to the partial or complete inhibition or suppression of the immune response of an individual towards cancer cells. The "tumor microenvironment" or "TME" is the environment around a tumor, including the surrounding blood vessels, immune cells, fibroblasts, signaling molecules and the extracellular matrix (ECM).

The term "immune response" refers to the action of, for example, lymphocytes, antigen presenting cells, phagocytic cells, granulocytes, and soluble macromolecules produced by these cells or the liver (including antibodies, cytokines, and complements) that results in selective damage to, destruction of, or elimination from the human body of invading pathogens, cells or tissues infected with pathogens, cancerous cells, or, in cases of autoimmunity or pathological inflammation, normal human cells or tissues.

As used herein, the terms "Anthrax Toxin Receptor 1", "ANTXR Cell Adhesion Molecule 1","ANTXR", "ANTXR1", "Tumor Endothelial Marker 8", "TEM8", "ATR" or "GAPO" are equivalent and refer to the product of the human ANTXR1 gene, for example such as described under Gene ID: 84168 and UniProt Q9H6X2 references. ANTXR1 protein plays a role in cell attachment and migration.

As used herein, the terms "Fibroblast Activation Protein", "FAP", "Prolyl endopeptidase FAP", "Dipeptidyl peptidase FAP", "Surface-expressed protease", " Sepras", "Serine integral membrane protease", "SIMP", "Integral membrane serine protease", "Post-proline cleaving enzyme" are used interchangeably and refers to the product of the FAP human gene, for example such as described under GenelD: 2191 and UniProt Q12884 references. FAP is a cell surface glycoprotein serine protease that participates in extracellular matrix degradation and involved in many cellular processes including tissue remodeling, fibrosis, wound healing, inflammation and tumor growth.

As used herein, the terms 'Lysosome-associated membrane glycoprotein 5", "Lysosome-associated membrane protein 5", "LAMPS", "Brain and dendritic cell-associated LAMP" and "Brain-associated LAMP-like protein" are equivalent and refer to the product of human LAMP5 gene, for example such as described under GenelD 24141 and UniProt Q9UJQ1 references.

As used herein, the terms "Syndecan-1", "SDC1", "SYND1", "CD138" are used interchangeably herein and refer to the product of human SDC1 gene, for example such as described under GenelD: 6382 and UniProt P18827 references. SDC1 is a cell surface proteoglycan that bears both heparan sulfate and chondroitin sulfate and that links the cytoskeleton to the interstitial matrix.

As used herein, the terms "CD9", "5H9 antigen", "Cell growth-inhibiting gene 2 protein", "Leukocyte antigen MIC3", "Motility-related protein", "MRP-1", "Tetraspanin-29", "Tspan-29" and "p24" are used interchangeably herein and refer to the product of human CD9 gene, for example such as described under GenelD: 928 and UniProt P21926 references. CD9 is an integral membrane protein associated with integrins, which regulates different processes.

"+" refers to a cell expressing a marker. For instance, ANTXR1⁺ refers to a cell expressing ANTXR1. Alternatively, "-" refers to a cell that does not expressing the marker. For instance, ANTXR1⁻ refers to a cell that does not express ANTXR1.

As used herein, the terms "subject", "individual" or "patient" are interchangeable and refer to an animal, preferably to a mammal, even more preferably to a human. However, the term "subject" can also refer to non-human animals, in particular mammals such as dogs, cats, horses, cows, pigs, sheep and non-human primates, among others.

As used herein, the term "marker" or "biomarker" refers to a measurable biological parameter that helps to predict the occurrence of a cancer, the efficiency of a cancer treatment or the presence of immunosuppressive cells.

As used herein, the term "diagnosis" refers to the determination as to whether a subject is likely to be affected by a cancer. The skilled artisan often makes a diagnosis on the basis of one or more diagnosis markers, the presence, absence, or amount of which is indicative of the presence or absence of the cancer. By "diagnosis", it is also intended to refer to the provision of information useful for diagnosis.

As used herein, the term "treatment", "treat" or "treating" refers to any act intended to ameliorate the health status of patients such as therapy, prevention, prophylaxis and retardation of the disease. In certain embodiments, such term refers to the amelioration or eradication of a disease or symptoms associated with a disease. In other embodiments, this term refers to minimizing the spread or worsening of the disease resulting from the administration of one or more therapeutic agents to a subject with such a disease.

As used herein, the term "immunotherapy", "immunotherapeutic agent" or "immunotherapy treatment" refers to a cancer therapeutic treatment using the immune system to reject cancer. The therapeutic treatment stimulates the patient's immune system to attack the malignant tumor cells. It includes immunization of the patient with tumor antigens (e.g. by administering a cancer vaccine), in which case the patient's own immune system is trained to recognize tumor cells as targets to be destroyed, or administration of molecules stimulating the immune system such as cytokines, or administration of therapeutic antibodies as drugs, in which case the patient's immune system is recruited by the therapeutic antibodies to destroy tumor cells. In particular, antibodies are directed against specific antigens such as the unusual antigens that are presented on the surfaces of tumors.

An important part of the immune system is its ability to tell between normal cells in the body and those it sees as "foreign", in particular cancer cells. This lets the immune system attack the cancer cells while leaving the normal cells alone. To do this, the immune system uses "checkpoints", these checkpoints are molecules on certain immune cells that need to be activated (or inactivated) to start an immune response. Cancer cells sometimes find ways to use these checkpoints to avoid being attacked by the immune system. As used herein, the term "immune checkpoint inhibitor treatment" refers to an immunotherapy that targets these checkpoints in order to allow or facilitate the attack of cancer cells by the immune system. The terms "percentage", "quantity," "number", "amount," and "level" are used interchangeably herein and may refer to an absolute quantification of a molecule or a cell in a sample, or to a relative quantification of a molecule or a cell in a sample, i.e., relative to another value such as relative to a reference value as taught herein.

As used herein, a "pharmaceutical composition" refers to a preparation of one or more of the active agents, with optional other chemical components such as physiologically suitable carriers and excipients. The purpose of a pharmaceutical composition is to facilitate administration of the active agent to an organism. Compositions of the present invention can be in a form suitable for any conventional route of administration or use. In one embodiment, a "composition" typically intends a combination of the active agent, e.g., compound or composition, and a naturally-occurring or non-naturally-occurring carrier, inert (for example, a detectable agent or label) or active, such as an adjuvant, diluent, binder, stabilizer, buffers, salts, lipophilic solvents, preservative, adjuvant or the like and include pharmaceutically acceptable carriers. An "acceptable vehicle" or "acceptable carrier" as referred to herein, is any known compound or combination of compounds that are known to those skilled in the art to be useful in formulating pharmaceutical compositions.

As used herein, the terms "active principle", "active ingredient" "active pharmaceutical ingredient", "therapeutic agent", "antitumor compound", and "antitumor agent" are equivalent and refer to a component having a therapeutic effect.

As used herein, the term "therapeutic effect" refers to an effect induced by an active ingredient or by a pharmaceutical composition according to the invention, capable to prevent or to delay the appearance or the development of a cancer, or to cure or to attenuate the effects of a cancer.

"An effective amount" or a "therapeutic effective amount" as used herein refers to the amount of active agent required to confer therapeutic effect on the subject, either alone or in combination with one or more other active agents, e.g. the amount of active agent that is needed to treat the targeted disease or disorder, or to produce the desired effect. The "effective amount" will vary depending on the agent(s), the disease and its severity, the characteristics of the subject to be treated including age, physical condition, size, gender and weight, the duration of the treatment, the nature of concurrent therapy (if any), the specific route of administration and like factors within the knowledge and expertise of the health practitioner. These factors are well known to those of ordinary skill in the art and can be addressed with no more than routine experimentation. It is generally preferred that a maximum dose of the individual components or combinations thereof be used, that is, the highest safe dose according to sound medical judgment.

The terms "kit", "product" or "combined preparation", as used herein, defines especially a "kit of parts" in the sense that the combination partners (a) and (b), as defined in the present application can be dosed independently or by use of different fixed combinations with distinguished amounts of the combination partners (a) and (b), i.e. simultaneously or at different time points. The parts of the kit of parts can then be administered simultaneously or chronologically staggered, that is at different time points for any part of the kit of parts. The ratio of the total amounts of the combination partner (a) to the combination partner (b) to be administered in the combined preparation can be varied. The combination partners (a) and (b) can be administered by the same route or by different routes.

As used herein, the term "simultaneous" refers to a pharmaceutical composition, a kit, a product or a combined preparation according to the invention in which the active ingredients are used or administered simultaneously, i.e. at the same time.

As used herein, the term "sequential" refers to a pharmaceutical composition, a kit, a product or a combined preparation according to the invention in which the active ingredients are used or administered sequentially, i.e. one after the other. Preferably, when the administration is sequential, all the active ingredients are administered in less than about an hour, preferably less than about 10 minutes, even more preferably in less than about a minute.

As used herein, the term "separate" refers to a pharmaceutical composition, a kit, a product or a combined preparation according to the invention in which the active ingredients are used or administered at distinct time of the day. Preferably, when the administration is separate, the active ingredients are administered with an interval of about 1 hour to about 24 hours, preferably with an interval of about 1 hour and 15 hours, more preferably with an interval of about 1 hour and 8 hours, even more preferably with an interval of about 1 hour and 4 hours.

The term "and/or" as used herein is to be taken as specific disclosure of each of the two specified features or components with or without the other. For example, "A and/or B" is to be taken as specific disclosure of each of (i) A, (ii) B and (iii) A and B, just as if each is set out individually.

The term "a" or "an" can refer to one of or a plurality of the elements it modifies (e.g., "a reagent" can mean one or more reagents) unless it is contextually clear either one of the elements or more than one of the elements is described.

The term "about" as used herein in connection with any and all values (including lower and upper ends of numerical ranges) means any value having an acceptable range of deviation of up to +/- 10% (e.g., +/-0.5%, +/-1 %, +/-1 .5%, +/- 2%, +/- 2.5%, +/- 3%, +/- 3.5%, +/- 4%, +/- 4.5%, +/- 5%, +/- 5.5%, +/- 6%, +/-6.5%, +/- 7%, +/- 7.5%, +/- 8%, +/- 8.5%, +/- 9%, +/-9.5%). The use of the term "about" at the beginning of a string of values modifies each of the values (i.e. "about 1, 2 and 3" refers to about 1, about 2 and about 3). Further, when a listing of values is described herein (e.g. about 50%, 60%, 70%, 80%, 85% or 86%) the listing includes all intermediate and fractional values thereof (e.g., 54%, 85.4%).

The methods of the invention as disclosed below may be *in vivo, ex vivo* or *in vitro* methods, preferably *in vitro* or *ex vivo* methods.

### Detection of novel fibroblast subpopulations, in particular CAF subpopulations

In the first aspect, the present invention concerns an *in vitro* method for detecting immunosuppressive fibroblasts, especially Cancer Associated Fibroblast (CAFs), in a cancer sample from a subject suffering from cancer, wherein the method comprises detecting ANTXR1⁺ fibroblasts, especially ANTXR1⁺ CAFs, in the cancer sample from said patient, the presence of ANTXR1⁺ fibroblasts, especially ANTXR1⁺ CAFs being indicative of immunosuppressive fibroblasts, especially immunosuppressive CAFs. The invention then envisioned the use of ANTXR1 as biomarker for the identification of immunosuppressive fibroblasts, especially immunosuppressive CAFs, in a cancer sample from a subject suffering from cancer. The invention also concerns the use of ANTXR1 and immunosuppressive ANTXR1⁺ fibroblasts, especially ANTXR1⁺ CAF, populations as a novel biomarker in the treatment of cancer. Particularly, the invention envisioned the use of immunosuppressive ANTXR1+ fibroblasts, especially ANTXR1⁺ CAFs, populations as a novel biomarker of the response to immunotherapy. The invention also concerns the use of ANTXR1 in fibroblasts, preferably CAFs, population as biomarker in a tumor of a subject suffering from cancer, for predicting the response of the subject to an immunotherapy, in particular whether a subject is susceptible to respond to an immunotherapy.

In one embodiment, the invention concerns an *in vitro* method for detecting immunosuppressive fibroblasts, especially Cancer Associated Fibroblast (CAFs), in a cancer sample from a subject suffering from cancer, wherein the method comprises detecting ANTXR1⁺ fibroblasts, especially ANTXR1⁺CAFs, in the cancer sample from said patient.

Optionally, the method may further comprise a prior step of providing a cancer sample from said patient. Preferably, the CAFs according to the invention belongs to the CAF-S1 subgroup, for example such as described in Costa et al., 2018, Cancer cell Volume 33, Issue 3, Pages 463-479.e10 or in WO 2019/020728. CAF-S1 population particular expresses one or more biomarkers selected from the group consisting of CD29, FAP, αSMA, PDGFRβ and FSP1. The CAF-S1 according to the invention are FAP⁺.

As used herein, the term "FAP⁺ fibroblast" refers to a subpopulation of fibroblasts that express FAP. As used herein, the term "FAP⁺ CAF" or "FAP⁺ Cancer Associated Fibroblast" refers to a subpopulation of CAFs that express FAP. Not all CAFs express FAP. Therefore, in a preferred embodiment, the detection of FAP+ CAFs relies on the detection of CAFs expressing FAP, preferably the detection of FAP mRNA and/or protein. However, other markers specific of FAP⁺ CAFs can also be used to detect them.

As used herein, the term "ANTXR1⁺ fibroblasts" refers to a subpopulation of fibroblasts that express ANTXR1. As used herein, the term "ANTXR1⁺ CAFs" or "ANTXR1⁺ Cancer Associated Fibroblast" refers to a subpopulation of CAFs that express ANTXR1. Not all fibroblasts or CAFs express ANTXR1. Therefore, in a preferred embodiment, the detection of ANTXR1⁺ fibroblasts, especially CAFs, relies on the detection of fibroblasts, especially CAFs, expressing ANTXR1, preferably the detection of ANTXR1 mRNA and/or protein. In a preferred aspect, the detection of ANTXR1 is carried out at the protein level, in particular with anti-ANTXR1 antibodies.

In one aspect, the invention concerns an *in vitro* method for detecting immunosuppressive fibroblasts, especially Cancer Associated Fibroblast (CAFs), in a cancer sample from a subject suffering from cancer, wherein the method comprises detecting FAP⁺ ANTXR1⁺ fibroblasts, especially CAFs, in the cancer sample from said patient.

However, other markers specific of ANTXR1⁺ fibroblasts, especially CAFs, can also be used to detect them. As used herein, the terms "ecm-myCAF", "CAF-S1 cluster 0", "ANTXR1⁺ SDC1⁺ LAMP5⁻ CAF" and "FAP⁺ ANTXR1⁺ SDC1⁺ LAMP5⁻ CAF" are used interchangeably and refer to a subpopulation of CAFs, especially a cluster of CAF subpopulation CAF-S1. Such population expresses FAP, ANTXR1 and SDC1 but does not express LAMP5. Therefore, in a preferred embodiment, the detection of ANTXR1⁺ SDC1⁺ LAMP5⁻ CAFs relies on the detection of CAFs expressing ANTXR1 and SDC1 but not LAMP5. The detection of ANTXR1, LAMP5 and SCD1 can be carried out at mRNA and/or protein level, preferably at the protein level, in particular by antibodies.

As used herein, the terms "TGFb-myCAF", "CAF-S1 cluster 3", "ANTXR1⁺ LAMP5⁺ SDC1⁺ and "FAP⁺ ANTXR1⁺ LAMP5⁺ SDC1^{+/-} "are used interchangeably and refer to a subpopulation of CAFs, especially a cluster of CAF subpopulation CAF-S1. Such population expresses FAP, ANTXR1 and LAMP5 and optionally expresses SDC1. Therefore, in a preferred embodiment, the detection of ANTXR1⁺ LAMP5⁺ SDC1^{+/-} CAFs relies on the detection of CAFs expressing ANTXR1, LAMP5 and optionally SDC1. The detection of ANTXR1, LAMP5 and SCD1 can be carried out at mRNA and/or protein level, preferably at the protein level, in particular by antibodies.

As used herein, the terms "wound-myCAF", "CAF-S1 cluster 4", "ANTXR1⁺ SDC1⁻ LAMP5⁻ CD9⁺" and "FAP⁺ ANTXR1⁺ SDC1⁻ LAMP5⁻ CD9⁺" are used interchangeably and refer to a subpopulation of CAFs, especially a cluster of CAF subpopulation CAF-S1. Such population expresses FAP, ANTXR1 and CD9 but does not express LAMP5 and SDC1. Therefore, in a preferred embodiment, the detection of ANTXR1⁺ SDC1⁻ LAMP5⁻ CD9⁺ CAFs relies on the detection of CAFs expressing ANTXR1 and CD9 but not SDC1 and LAMP5. The detection of ANTXR1, LAMP5, CD9 and SCD1 can be carried out at mRNA and/or protein level, preferably at the protein level, in particular by antibodies.

In one aspect, the invention concerns an *in vitro* method for detecting immunosuppressive fibroblasts, especially Cancer Associated Fibroblast (CAFs), in a cancer sample from a subject suffering from cancer, wherein the method comprises detecting ecm-myCAF (ANTXR1⁺ SDC1⁺ LAMP5⁻), TGFb-myCAF (ANTXR1⁺ LAMP5⁺ SDC1^{+/-}) and/or wound-myCAF (ANTXR1⁺ SDC1⁻ LAMP5⁻ CD9⁺) CAFs in the cancer sample from said patient. Optionally, the method comprises detecting ecm-myCAF (ANTXR1⁺ SDC1⁺ LAMP5⁻) CAFs in the cancer sample from said patient. Optionally, the method comprises detecting TGFb-myCAF (ANTXR1⁺ LAMP5⁺ SDC1^{+/-}) CAFs in the cancer sample from said patient. Optionally; the method comprises detecting wound-myCAF (ANTXR1⁺ SDC1⁻ LAMP5⁻ CD9⁺) CAFs in the cancer sample from said patient. Optionally, the method comprises detecting ecm-myCAF (ANTXR1⁺ SDC1⁺ LAMP5⁻) and TGFb-myCAF (ANTXR1⁺ LAMP5⁺ SDC1^{+/-}) CAFs in the cancer sample from said patient. Optionally, the method comprises detecting ecm-myCAF (ANTXR1⁺ SDC1⁺ LAMP5⁻) and wound-myCAF (ANTXR1⁺ SDC1⁻ LAMP5⁻ CD9⁺) CAFs in the cancer sample from said patient. Optionally, the method comprises detecting TGFb-myCAF (ANTXR1⁺ LAMP5⁺ SDC1^{+/-}) and wound-myCAF (ANTXR1⁺ SDC1⁻ LAMP5⁻ CD9⁺) CAFs in the cancer sample from said patient. Optionally, the method comprises detecting ecm-myCAF (ANTXR1⁺ SDC1⁺ LAMP5⁻), TGFb-myCAF (ANTXR1⁺ LAMP5⁺ SDC1^{+/-}) and wound-myCAF (ANTXR1⁺ SDC1⁻ LAMP5⁻ CD9⁺) CAFs in the cancer sample from said patient.

In an embodiment, a fibroblast, especially a CAF, is considered as expressing FAP, ANTXR1, LAMP5, SDC1 and/or CD9 when the level of the mRNA thereof and/or the level of the protein thereof is significantly different from the level of the corresponding background noise, for example the level measured in the same conditions but in the absence of cells, and/or from the level of the corresponding to a control condition or from RNA or protein of reference, for example the level measured in the same conditions but with cells known for not expressing FAP, ANTXR1, LAMP5, SDC1 and/or CD9 respectively.

The measurement of FAP, ANTXR1, LAMP5, SDC1 and/or CD9 expression level in a fibroblast, especially a CAF, can be implemented by a variety of techniques well known by the skilled person in the art. In particular, the measure of the expression level of FAP, ANTXR1, LAMP5, SDC1 and/or CD9 in CAFs can rely on the detection of FAP, ANTXR1, LAMP5, SDC1 and/or CD9 mRNA (messenger RNA), or protein.

In one aspect, the expression of FAP, ANTXR1, LAMP5, SDC1 and/or CD9 in a fibroblast, especially a CAF, is determined by measuring the expression of their mRNA. Methods for determining the quantity of mRNA in a cell are well known by the man skilled in the art. mRNA can be detected by hybridization (e. g., Northern blot analysis) in particular by the Nanostring method and/or by amplification (e.g., RT-PCR), in particular by quantitative or semi-quantitative RT-PCR. Other methods of Amplification include ligase chain reaction (LCR), transcription-mediated amplification (TMA), strand displacement amplification (SDA) and nucleic acid sequence-based amplification (NASBA).

Real-time quantitative or semi-quantitative RT-PCR is particularly advantageous. Taqman probes specific of the protein of interest transcript may be used. In a preferred embodiment, the expression level of FAP, ANTXR1, LAMP5, SDC1 and/or CD9 and any other protein of interest is determined by measuring the quantity of their mRNA, preferably by quantitative or semi-quantitative RT-PCR or by real-time quantitative or semi-quantitative RT-PCR.

As used herein, the terms "quantitative RT-PCR", "qRT-PCR", "Real time RT-PCR" and "quantitative Real time RT-PCR" are equivalent and can be used interchangeably. Any of a variety of published quantitative RT-PCR protocols can be used (and modified as needed) for use in the present method. Suitable quantitative RT-PCR procedures include but are not limited to those presented in U.S. Pat. No. 5,618,703 and in U.S. Patent Application No. 2005/0048542.

Optionally, ANTXR1, FAP, LAMP5, SDC1 and/or CD9 can be determined on the tumor sample or on a subset of the tumor comprising the fibroblasts, especially CAFs. Accordingly, the tumor can be previously treated before the detection of ANTXR1, FAP, LAMP5, SDC1 and/or CD9 in order to isolate the fibroblasts, especially CAFs, from the other cells of the tumor, in particular the tumor cells. Accordingly, the present invention may relate to a method for detecting immunosuppressive fibroblasts, especially CAFs, in a cancer sample from a subject suffering from cancer, wherein the method comprises isolating fibroblasts, especially CAFs, of the cancer sample from a subject, especially from tumor cells, and detecting ANTXR1⁺ fibroblasts, especially CAFs, in the isolated fibroblasts, especially CAFs respectively.

In another aspect, the expression level of ANTXR1, FAP, LAMP5, SDC1 and/or CD9 in a fibroblast, especially a CAF, is determined by measuring the expression of their respective proteins.

The quantity of a protein may be measured by any method known by the skilled person. Usually, these methods comprise contacting the sample with a binding partner capable of selectively interacting with the protein present in the sample. The binding partner is generally a polyclonal or monoclonal antibody, preferably a monoclonal antibody. Such an antibody can be produced through methods known to the man skilled in the art. This antibody includes in particular those produced by a hybridoma and those produced by genetic engineering using host cells transformed with a recombinant expression vector carrying a gene encoding the antibody. A hybridoma producing monoclonal antibodies can be obtained as following: the protein or immunogenic fragments thereof are used as antigens for immunization according to conventional methods of immunization. The resulting immunocytes are fused with known parent cells according to conventional cell fusion methods and the cells producing the antibodies are thus screened from fused cells using conventional screening methods.

The antibody according to the invention can be labeled and/or fused to a detection entity. Preferably, the antibody according to the invention is labeled or fused to a detection entity.

In a preferred embodiment, the antibody is labeled. The antibody can be labeled with a label selected from the group consisting in a radiolabel, an enzyme label, a fluorescent label, a biotin-avidin label, a chemoluminescent label, and the like. The antibody according to the invention can be labeled by standard labeling techniques well known by the man skilled in the art and labeled antibodies can be visualized using known methods. In particular, labels generally provide signals detectable by fluorescence, chemoluminescence, radioactivity, colorimetry, mass spectrometry, X-ray diffraction or absorption, magnetism, enzymatic activity, or the like.

Preferably, the detectable label may be a luminescent label. For example, fluorescent labels, bioluminescent labels, chemoluminescent labels, and colorimetric labels may be used in the practice of the invention, more preferably a fluorescent label. Preferably, the label is linked at the C-terminal extremity of the antibody.

In another preferred embodiment, the above-mentioned antibody can be fused to a detection entity. The detection entity may be selected from the group consisting of a tag, an enzyme or a fluorescent protein. Preferably, the detection entity is at the C-terminal extremity of the antibody.

The quantity of ANTXR1, FAP, LAMP5, SDC1 and/or CD9 may be measured by semi-quantitative Western blots, enzyme-labeled and mediated immunoassays, such as ELISAs, biotin/avidin type assays, radioimmunoassay, immunohistochemistry, immunoelectrophoresis or immunoprecipitation, protein or antibody arrays, or flow cytometry, such as Fluorescence-activated cell sorting (FACS). The reactions generally include revealing labels such as fluorescent, chemoluminescent, radioactive, enzymatic labels or dye molecules, or other methods for detecting the formation of a complex between the antigen and the antibody or antibodies reacted therewith. Preferably, the protein expression level is assessed by FACS or by immunohistochemistry.

Fluorescence-activated cell sorting (FACS) is a specialized type of flow cytometry. It provides a method for sorting a heterogeneous mixture of biological cells into two or more containers, one cell at a time, based upon the specific light scattering and fluorescent characteristics of each cell. The cell suspension is entrained in the center of a narrow, rapidly flowing stream of liquid. The flow is arranged so that there is a large separation between cells relative to their diameter. A vibrating mechanism causes the stream of cells to break into individual droplets. The system is adjusted so that there is a low probability of more than one cell per droplet. Just before the stream breaks into droplets, the flow passes through a fluorescence measuring station where the fluorescent character of each cell of interest is measured. An electrical charging ring is placed just at the point where the stream breaks into droplets. A charge is placed on the ring based immediately prior to fluorescence intensity being measured, and the opposite charge is trapped on the droplet as it breaks from the stream. The charged droplets then fall through an electrostatic deflection system that diverts droplets into containers based upon their charge. Immunohistochemistry (IHC) refers to the process of selectively imaging antigens (e.g. proteins) in cells of a tissue section by exploiting the principle of antibodies binding specifically to antigens in biological tissues. Visualizing the antibody-antigen interaction can be accomplished in a number of ways, well known by the man skilled in the art. In the most common instance, an antibody is conjugated to an enzyme, such as peroxidase, that can catalyze a color-producing reaction or is tagged by a fluorophore, such as fluorescein or rhodamine. Immunohistochemistry can be divided into two phases: sample preparation and sample labeling.

Preparation of the sample is critical to maintain cell morphology, tissue architecture and the antigenicity of target epitopes. This requires proper tissue collection, fixation and sectioning. A solution of paraformaldehyde is often used to fix tissue, but other methods may be used. The tissue may then be sliced or used whole, dependent upon the purpose of the experiment or the tissue itself. Before sectioning, the tissue sample may be embedded in a medium, like paraffin wax or cryomedia. Sections can be sliced on a variety of instruments, most commonly a microtome, cryostat, or Compresstome tissue slicer. Specimens are typically sliced at a range of 3 µm-50 µm. The slices are then mounted on slides, dehydrated using alcohol washes of increasing concentrations (e.g., 50%, 75%, 90%, 95%, 100%), and cleared using a detergent like xylene before being imaged under a microscope. Depending on the method of fixation and tissue preservation, the sample may require additional steps to make the epitopes available for antibody binding, including deparaffinization and antigen retrieval. For formalin-fixed paraffin-embedded tissues, antigen-retrieval is often necessary, and involves pre-treating the sections with heat or protease. These steps may make the difference between the target antigens staining or not staining. Depending on the tissue type and the method of antigen detection, endogenous biotin or enzymes may need to be blocked or quenched, respectively, prior to antibody staining. Although antibodies show preferential avidity for specific epitopes, they may partially or weakly bind to sites on nonspecific proteins (also called reactive sites) that are similar to the cognate binding sites on the target antigen. To reduce background staining in IHC, samples are incubated with a buffer that blocks the reactive sites to which the primary or secondary antibodies may otherwise bind. Common blocking buffers include normal serum, non-fat dry milk, BSA, or gelatin. Methods to eliminate background staining include dilution of the primary or secondary antibodies, changing the time or temperature of incubation, and using a different detection system or different primary antibody. Quality control should as a minimum include a tissue known to express the antigen as a positive control, and negative controls of tissue known not to express the antigen, as well as the test tissue probed in the same way with omission of the primary antibody (or better, absorption of the primary antibody).

For immunohistochemical detection strategies, antibodies are classified, when necessary, as primary or secondary reagents. Primary antibodies are raised against an antigen of interest and are typically unconjugated (i.e. unlabeled), while secondary antibodies are raised against immunoglobulin of the primary antibody species. The secondary antibody is usually labeled and/or fused to a detection entity as described above.

The direct method is a one-step staining method and involves a labeled antibody reacting directly with the antigen in tissue sections. While this technique utilizes only one antibody and therefore is simple and rapid, the sensitivity is lower due to little signal amplification, in contrast to indirect approaches.

The indirect method involves an unlabeled primary antibody (first layer) that binds to the target antigen in the tissue and a labeled secondary antibody (second layer) that reacts with the primary antibody. The secondary antibody must be raised against the IgG of the animal species in which the primary antibody has been raised. This method is more sensitive than direct detection strategies because of signal amplification due to the binding of several secondary antibodies to each primary antibody if the secondary antibody is conjugated to the fluorescent or enzyme reporter. Further amplification can be achieved if the secondary antibody is conjugated to several biotin molecules, which can recruit complexes of avidin-, streptavidin- or NeutrAvidin protein-bound enzyme.

Preferably, the presence of immunosuppressive CAF is performed by protein expression level assessment by FACS or by immunohistochemistry as described in the experimental part.

Antibodies that can be used to measure FAP expression level in a CAF by FACS or immunohistochemistry are for example: the anti-human FAP antibody of reference #MAB3715 (from R&D systems), ab53066 (abcam), ABIN560844, Vitatex-MABS1001.

Antibodies that can be used to measure ANTXR1 expression level in a CAF by FACS or immunohistochemistry are for example: the anti- ANTXR1-AF405 antibody of reference #NB-100-56585 (from Novus Biological), Human TEM8/ANTXR1 Antibody MAB3886 (R&Dsystem), ABIN252539, ANTXR1 Antibody (15091-1-AP, Thermo Fischer), NB-100-56585 (Novus), MA1-91702 (Thermo Fischer), ab21270 (Abcam), LS-B13896 (Life span bioscience), rb158588 (Biorbyt), bs-5210R (Bioss) or any of these antibody with an alternative label, in particular fluorescent label.

Antibodies that can be used to measure LAMP5 expression level in a CAF by FACS or immunohistochemistry are for example: the anti-LAMP5-PE antibody of reference #130-109-156 (from Miltenyi Biotech).

Antibodies that can be used to measure SDC1 expression level in a CAF by FACS or immunohistochemistry are for example: the anti-SDC1-BUV737 antibody of reference #BD-564393 (from BD Biosciences), ABIN5680139.

Antibodies that can be used to measure CD9 expression level in a CAF by FACS or immunohistochemistry are for example: the anti-CD9-BV711 antibody of reference #BD-743050 (from BD Biosciences), LS-B5962 (LSBio), or AB_2075893 (BioLegend).

In a preferred embodiment, the detection of ANTXR1⁺ CAFs by FACS in a cancer sample of a patient may comprise the following steps:
- exclusion of the non-CAF cells, for example by excluding the CD45⁺ cells, the EpCAM⁺ cells and the CD31⁺ cells and thereby selecting the CD45⁻EpCAM⁻CD31⁻ cells; and/or
- selection of the CAF cells, for example by selecting the CD29⁺ cells and/or the PDGFRb⁺ cells, preferably by selecting the CD29⁺ cells;
- selection of CAF-S1 cells, for example by selecting CD29⁺, FAP⁺, αSMA⁺, PDGFRβ⁺ and/or FSP1⁺ cells, preferably FAP⁺ cells,
- optionally, exclusion of the dead cells, for example by using an intracellular dye such as the violet LIVE/DEAD dye or DAPI and excluding the stained cells; and
- detection of ANTXR1⁺ CAFs in the cells obtained at the precedent step,
- optionally detection of LAMP5, SDC1 and/or CD9 positive or negative cells.
   In another preferred embodiment, the detection of ANTXR1⁺ CAFs by immunohistochemistry in a cancer sample of a patient may comprise the following steps:
- identification of the CAFs in the cancer sample, for example on the basis of morphological criteria;
- detection of the ANTXR1⁺ CAFs among the CAFs, preferably based on ANTXR1 antibodies immunostaining;
- detection of the FAP⁺ CAFs among the CAFs, preferably based on FAP antibodies immunostaining;
- optionally detection of LAMP5, SDC1 and/or CD9 positive or negative cells.

In one aspect, the presence of immunosuppressive fibroblasts, especially CAFs, refers to the quantity of immunosuppressive fibroblasts, especially CAFs, in a tumor sample or any fraction of it, a ratio or percentage of the number of cells of the ANTXR1⁺ fibroblasts, in particular ANTXR1⁺ CAFs on the total number of cells of the sample or any fraction of it, a ratio or percentage of the number of cells of the ANTXR1⁺ fibroblasts, in particular ANTXR1⁺ CAF on the total number of stromal cells of the sample or any fraction of it, a ratio or percentage of the number of cells of ANTXR1⁺ fibroblasts on the total number of fibroblasts in the sample, a ratio or percentage of the number of cells of ANTXR1⁺ fibroblasts on the total number of fibroblasts in the sample, a ratio or percentage of the number of cells of the ANTXR1⁺ CAF on the total number of CAF cells of the sample or any fraction of it, or a ratio or percentage of the number of cells of the ANTXR1⁺ CAF on the total number of CAF-S1 cells of the sample or any fraction of it. Preferably, the term "percentage of ANTXR1+ fibroblasts in a tumor" may refer to any ratio having the number of ANTXR1⁺ fibroblasts as nominator, and a number of reference cells as a denominator. Preferably, the term "percentage of ANTXR1+ CAFs in a tumor" may refer to any ratio having the number of ANTXR1⁺ CAFs cells as nominator, and a number of reference cells as a denominator. In cancer sample, such reference cells can particularly be selected from the group consisting of: all the cells of the cancer sample, cancer cells, stromal cells, CAFs cells and CAF-S1 cells.

A "low percentage of ANTXR1⁺ fibroblasts" or a "low percentage of ANTXR1⁺ fibroblasts" corresponds to less than 20%, 10%, 5%, 2%, 1%, 0.5% or 0.1%.

A "high percentage of ANTXR1⁺ fibroblasts" or a "high percentage of ANTXR1⁺ fibroblasts" corresponds to more than 20%, 30%, 40%, 50%, 60%, 70%, 80%, or 90%.

Then, in a first aspect, the presence of ANTXR1⁺ fibroblasts, especially CAFs, in a cancer sample of a patient represents the number of ANTXR1⁺ fibroblasts, especially CAFs cells, on the total number of cells in a cancer sample. Preferably, the presence of ANTXR1⁺ fibroblasts, especially CAFs, corresponds to a percentage of at least 0.001%, 0.01%, 0.1%, 1%, 5%, 10%, 20% or 30%.

In a second aspect, the presence of ANTXR1⁺ fibroblasts, especially CAFs, in a cancer sample of a patient represents the number of ANTXR1⁺ fibroblasts, especially CAFs cells, on the total number of stromal cells in a cancer sample. Preferably, the presence of ATXR1+ fibroblasts, especially CAFs, corresponds to a percentage of at least 0.001%, 0.1%, 1%, 5%, 10%, 20% or 30%.

In a third aspect, the presence of ANTXR1⁺ fibroblasts, especially CAFs, in a cancer sample of a patient represents the number of ANTXR1⁺ fibroblasts, especially CAFs cells, on the total number of CAFs cells in a cancer sample. Preferably, the presence of ATXR1+ fibroblasts, especially CAFs, corresponds to a percentage of at least 0.1%, 1%, 5%, 10%, 20%, 30%, 40% or 50%.

In a fourth aspect, the presence of ANTXR1⁺ fibroblasts, especially CAFs, in a cancer sample of a patient represents the number of ANTXR1⁺ fibroblasts, especially CAFs, on the total number of CAF-S1 cells in a cancer sample. Preferably, the presence of ATXR1+ fibroblasts, especially CAFs, corresponds to a percentage of at least 0.1%, 1%, 5%, 10%, 20%, 30%, 40% or 50%.

In an aspect, the presence of ANTXR1⁺ fibroblasts, especially CAFs, in the cancer sample, for instance the percentage of ANTXR1⁺ fibroblasts, especially CAFs, in the cancer sample, is inversely proportional of the responsiveness of said patient to an immunotherapy treatment; and optionally the method further comprising selecting patients without or with a low percentage of ANTXR1⁺ fibroblasts, especially CAFs, in the cancer sample as suitable for an immunotherapy treatment, and/or selecting patients with medium or high percentage of ANTXR1⁺ fibroblasts, especially CAFs, in the cancer sample for an alternative treatment such as anti-cancer treatment excluding immunotherapy or immunotherapy treatment combined with a treatment reducing the immunosuppression induced by ANTXR1⁺ fibroblasts, especially CAFs.

By "inversely proportional" it is meant that the more ANTXR1⁺ fibroblasts, especially CAFs, are in the cancer sample, the less the subject suffering from cancer will respond to immunotherapy. These terms do not necessarily imply a direct and measurable correlation factor.

In one aspect, the invention concerns an *in vitro* method for predicting a response of a subject suffering from cancer to an immunotherapy treatment, wherein the method comprises detecting ANTXR1⁺ fibroblasts, especially CAFs, in a cancer sample from said patient, wherein ANTXR1⁺ fibroblasts, especially CAFs, in the cancer sample are indicative or predictive of the non-responsiveness of said patient to an immunotherapy treatment. In particular, the more ANTXR1⁺ fibroblasts, especially CAFs, are detected in a cancer sample, the less the patient is susceptible to respond to an immunotherapy treatment. Optionally, the ANTXR1⁺ fibroblasts are ANTXR1⁺ CAFs. ANTXR1⁺ fibroblasts are FAP⁺ fibroblast.ANTXR1⁺ CAFs are FAP⁺ ANTXR1⁺ CAFs.

Indeed, the present invention relates to the use of ANTXR1 in fibroblasts, especially CAFs, population as biomarker in a tumor of a subject suffering from cancer, for predicting the response of the subject to an immunotherapy.

Optionally, the invention concerns an *in vitro* method for predicting a response of a subject suffering from cancer to an immunotherapy treatment, wherein the method comprises: (a) detecting ANTXR1⁺ fibroblasts, especially ANTXR1⁺ CAFs, in a cancer sample from said patient, wherein ANTXR1⁺ fibroblasts, especially CAFs, in the cancer sample are indicative or predictive of the non-responsiveness of said patient to an immunotherapy treatment;
(b) optionally, selecting patients without ANTXR1⁺ fibroblasts, especially ANTXR1⁺ CAFs, as suitable for an immunotherapy treatment, and/or selecting patients with ANTXR1⁺ fibroblasts, especially ANTXR1⁺ CAFs, for an alternative treatment such as anti-cancer treatment excluding immunotherapy or immunotherapy treatment combined with a treatment reducing the immunosuppression induced by ANTXR1⁺ fibroblasts, especially ANTXR1⁺ CAFs.

Optionally, the method comprises detecting FAP⁺ ANTXR1⁺ fibroblasts or CAFs in the cancer sample from said patient. Optionally, the method comprises detecting ecm-myCAF (ANTXR1⁺ SDC1⁺ LAMP5⁻), TGFb-myCAF (ANTXR1⁺ LAMP5⁺ SDC1^{+/-}) and/or wound-myCAF (ANTXR1⁺ SDC1⁻ LAMP5⁻ CD9⁺) CAFs in the cancer sample from said patient. Optionally, the method comprises detecting ecm-myCAF (ANTXR1⁺ SDC1⁺ LAMP5⁻) CAFs in the cancer sample from said patient. Optionally, the method comprises detecting TGFb-myCAF (ANTXR1⁺ LAMP5⁺ SDC1^{+/-}) CAFs in the cancer sample from said patient. Optionally; the method comprises detecting wound-myCAF (ANTXR1⁺ SDC1⁻ LAMP5⁻ CD9⁺) CAFs in the cancer sample from said patient. Optionally, the method comprises detecting ecm-myCAF (ANTXR1⁺ SDC1⁺ LAMP5⁻) and TGFb-myCAF (ANTXR1⁺ LAMP5⁺ SDC1^{+/-}) CAFs in the cancer sample from said patient. Optionally, the method comprises detecting ecm-myCAF (ANTXR1⁺ SDC1⁺ LAMP5⁻) and wound-myCAF (ANTXR1⁺ SDC1⁻ LAMP5⁻ CD9⁺) CAFs in the cancer sample from said patient. Optionally, the method comprises detecting TGFb-myCAF (ANTXR1⁺ LAMP5⁺ SDC1^{+/-}) and wound-myCAF (ANTXR1⁺ SDC1⁻ LAMP5⁻ CD9⁺) CAFs in the cancer sample from said patient. Optionally, the method comprises detecting ecm-myCAF (ANTXR1⁺ SDC1⁺ LAMP5⁻), TGFb-myCAF (ANTXR1⁺ LAMP5⁺ SDC1^{+/-}) and wound-myCAF (ANTXR1⁺ SDC1⁻ LAMP5⁻ CD9⁺) CAFs in the cancer sample from said patient.

In one aspect, the CAFs subpopulations are detected by specific gene signatures. A "gene signature" or "gene expression signature" is a single or a group of genes in a cell, particularly a fibroblast, with a uniquely characteristic pattern of gene expression. In particular, the gene signature corresponds to the deregulation of specific genes, in particular, overexpression of genes.

In one aspect, the ecm-myCAF gene signature comprises the following genes:
ASPN, COL3A1, THY1, SFRP2, COL10A1, COL6A3, LRRC17, CILP, GRP, ITGBL1, COL8A1, COL14A1, ADAM12, OLFML2B, ELN, PLPP4, CREB3L1, FBN1, LOXL1, MATN3, LRRC15, COMP, ISLR, P3H1, COL11A1, SEPT11, NBL1, SPON1, SULF1, FNDC1, CNN1, MIAT, MMP23B, CPXM1, FIBIN, P4HA3, GXYLT2, CILP2, P3H4, and CCDC80. In a particular aspect, the ecm-myCAF gene signature further comprises the following genes: FAP and/or ANTXR1.

In one aspect, the TGFβ-myCAF gene signature comprises the following genes:
CST1, LAMP5, LOXL1, EDNRA, TGFB1, TGFB3, TNN, CST2, HES4, COL10A1, ELN, THBS4, NKD2, OLFM2, COL6A3, LRRC17, COL3A1, THY1, HTRA3, TMEM204, SEPT11, COMP, TNFAIP6, ID4, GGT5, INAFM1, CILP and OLFML2B. In a particular aspect, the TGFβ-myCAF gene signature further comprises the following genes: FAP and/or ANTXR1.

In one aspect, the wound-myCAF gene signature comprises the following genes:
SFRP4, CCDC80, OGN, DCN, PTGER3, SFRP2, PDGFRL, SMOC2, MMP23B, CPXM2, COL14A1, ITGBL1, WISP2, CILP2, COL8A1, GAS1, COL3A1, OMD, COL11A1, CILP, NEXN, ASPN, RARRES2, FIBIN, TMEM119, KERA, ID4, GRP, COMP, DPT, ELN, FBLN2, IGF1 and IGF2. In a particular aspect, the wound-myCAF gene signature further comprises the following genes: FAP and/or ANTXR1.

Therefore, in the method of the present disclosure, the ecm-myCAF, TGFβ-myCAF and/or wound-myCAF are detected by determining the expression of genes comprising the gene signature specific of the ecm-myCAF, TGFβ-myCAF and/or wound-myCAF, an overexpression thereof being indicative of the presence of these CAF subpopulations. Optionally, the gene signature may comprise additional genes but no more than 50 genes, especially no more than 40 genes.

The present invention further relates to the use of these gene signatures for detecting the ecm-myCAF, TGFβ-myCAF and/or wound-myCAF subpopulation, in particular for detecting the presence of immunosuppressive CAF and/or predicting the response to a treatment by an immunotherapy. Optionally, before determining the expression of the gene signature, the sample can be treated in order to remove the non-CAF cells.

By "overexpressed" or "overexpression" it is referred to an expression level measured at the nucleic acid level, especially mRNA level. It can be measured by any method known by the person skilled in the art. A gene is overexpressed when its expression is increased, at least by a log2, when compared to a level of reference. The level of reference can be the expression of the gene in a control cell or cells, the control being for instance a population of fibroblasts, particularly CAFs, preferably a population of FAP+ CAFs.. In a particular aspect, the control cells is a cluster of CAF that is not ecm-myCAF (cluster 0), TGFβ-myCAF (cluster 3) and/or wound-myCAF (4), for example detox-iCAF (cluster 1), IL-iCAF (cluster 2), IFN y -iCAF (cluster 5), IFN αβ -myCAF (cluster 6) and acto-myCAF (cluster 7), or any combination thereof.

These gene signatures specific of the ecm-myCAF, TGFβ-myCAF and/or wound-myCAF subpopulations can be used in combination with other gene signatures known by the person skilled in the art, in particular gene signatures associated with a response prediction or a cancer diagnosis.

In another embodiment, the invention concerns an *in vitro* method for predicting a response of a subject suffering from cancer to an immunotherapy treatment, wherein the method comprises:
(a) detecting ANTXR1⁺ fibroblasts, especially ANTXR1⁺ CAFs, in a cancer sample from said patient;
(b) determining the percentage of ANTXR1⁺ fibroblasts, especially ANTXR1⁺ CAFs, in said cancer sample, wherein the responsiveness of said patient to an immunotherapy treatment is inversely proportional to the percentage of ANTXR1⁺ fibroblasts, especially ANTXR1⁺ CAFs, in the cancer sample;
(c) optionally, selecting patients with low percentage of ANTXR1⁺ fibroblasts, especially ANTXR1⁺ CAFs, in particular patients without any ANTXR1⁺ fibroblasts, especially ANTXR1⁺ CAFs, as suitable for an immunotherapy treatment and selecting patients with medium or high percentage(s) of ANTXR1⁺ fibroblasts, especially ANTXR1⁺ CAFs for an alternative treatment such as anti-cancer treatment excluding immunotherapy or immunotherapy treatment combined with a treatment reducing the immunosuppression induced by ANTXR1⁺ fibroblasts, especially ANTXR1⁺ CAFs.

Optionally, the method comprises detecting FAP⁺ ANTXR1⁺ fibroblasts or CAFs in the cancer sample from said patient. Optionally, the method comprises detecting ecm-myCAF (ANTXR1⁺ SDC1⁺ LAMP5⁻), TGFb-myCAF (ANTXR1⁺ LAMP5⁺ SDC1^{+/-}) and/or wound-myCAF (ANTXR1⁺ SDC1⁻ LAMP5⁻ CD9⁺) CAFs in the cancer sample from said patient. Optionally, the method comprises detecting ecm-myCAF (ANTXR1⁺ SDC1⁺ LAMP5⁻) CAFs in the cancer sample from said patient. Optionally, the method comprises detecting TGFb-myCAF (ANTXR1⁺ LAMP5⁺ SDC1^{+/-}) CAFs in the cancer sample from said patient. Optionally; the method comprises detecting wound-myCAF (ANTXR1⁺ SDC1⁻ LAMP5⁻ CD9⁺) CAFs in the cancer sample from said patient. Optionally, the method comprises detecting ecm-myCAF (ANTXR1⁺ SDC1⁺ LAMP5⁻) and TGFb-myCAF (ANTXR1⁺ LAMP5⁺ SDC1^{+/-}) CAFs in the cancer sample from said patient. Optionally, the method comprises detecting ecm-myCAF (ANTXR1⁺ SDC1⁺ LAMP5⁻) and wound-myCAF (ANTXR1⁺ SDC1⁻ LAMP5⁻ CD9⁺) CAFs in the cancer sample from said patient. Optionally, the method comprises detecting TGFb-myCAF (ANTXR1⁺ LAMP5⁺ SDC1^{+/-}) and wound-myCAF (ANTXR1⁺ SDC1⁻ LAMP5⁻ CD9⁺) CAFs in the cancer sample from said patient. Optionally, the method comprises detecting ecm-myCAF (ANTXR1⁺ SDC1⁺ LAMP5⁻), TGFb-myCAF (ANTXR1⁺ LAMP5⁺ SDC1^{+/-}) and wound-myCAF (ANTXR1⁺ SDC1⁻ LAMP5⁻ CD9⁺) CAFs in the cancer sample from said patient. Optionally, the method may further comprise a step of providing a cancer sample from said patient before the step (a).

In another particular aspect, the present invention also concerns a method for selecting a patient affected with a tumor for an immunotherapy treatment or for determining whether a patient affected with a tumor is susceptible to benefit from an immunotherapy treatment, wherein the method comprises determining the presence of ANTXR1⁺ fibroblasts or CAFs in a cancer sample from said patient and optionally selecting patients without ANTXR1⁺ fibroblasts or CAFs or with low percentage of ANTXR1⁺ fibroblasts or CAFs for an immunotherapy treatment.

Optionally, the method further comprises a previous step of providing a cancer sample from said patient. In still another particular aspect, the present invention also concerns a method for selecting a patient affected with a tumor for an alternative treatment such as anti-cancer treatment excluding immunotherapy or an immunotherapy treatment combined with a treatment reducing the immunosuppression induced by ANTXR1⁺ fibroblasts or CAFs or for determining whether a patient affected with a tumor is susceptible to benefit from an alternative treatment such as anti-cancer treatment excluding immunotherapy or an immunotherapy treatment combined with a treatment reducing the immunosuppression induced by ANTXR1⁺ fibroblasts or CAFs, wherein the method comprises determining the presence of ANTXR1⁺ fibroblasts or CAFs in a cancer sample from said patient and optionally selecting patients with ANTXR1⁺ CAFs or with high percentage of ANTXR1⁺ fibroblasts or CAFs for such an alternative treatment.

ANTXR1⁺ fibroblasts or CAFs are FAP⁺ ANTXR1⁺ fibroblasts or CAFs. Preferably, the ANTXR1⁺ CAFs are selected from the group consisting of FAP⁺ ANTXR1⁺ CAFs, FAP⁺ ANTXR1⁺ LAMP5⁻ SDC1⁺ CAFs, FAP⁺ ANTXR1⁺ LAMP5⁺ SDC1^{+/-} CAFs, FAP⁺ ANTXR1⁺ LAMP5⁻ SDC1⁺ CAFs and/or FAP⁺ ANTXR1⁺ LAMP5⁺ SDC1^{+/-} CAFs.

Optionally, the ANTXR1⁺ CAFs are ecm-myCAF (ANTXR1⁺ SDC1⁺ LAMP5⁻) CAFs. Optionally, the ANTXR1⁺ CAFs are TGFb-myCAF (ANTXR1⁺ LAMP5⁺ SDC1^{+/-}) CAFs. Optionally; the ANTXR1⁺ CAFs are wound-myCAF (ANTXR1⁺ SDC1⁻ LAMP5⁻ CD9⁺) CAFs. Optionally, the ANTXR1⁺ CAFs are ecm-myCAF (ANTXR1⁺ SDC1⁺ LAMP5⁻) and TGFb-myCAF (ANTXR1⁺ LAMP5⁺ SDC1^{+/-}) CAFs. Optionally, the ANTXR1⁺ CAFs are ecm-myCAF (ANTXR1⁺ SDC1⁺ LAMP5⁻) and wound-myCAF (ANTXR1⁺ SDC1⁻ LAMP5⁻ CD9⁺) CAFs. Optionally, the ANTXR1⁺ CAFs are TGFb-myCAF (ANTXR1⁺ LAMP5⁺ SDC1^{+/-}) and wound-myCAF (ANTXR1⁺ SDC1⁻ LAMP5⁻ CD9⁺) CAFs. Optionally, the ANTXR1⁺ CAFs are ecm-myCAF (ANTXR1⁺ SDC1⁺ LAMP5⁻), TGFb-myCAF (ANTXR1⁺ LAMP5⁺ SDC1^{+/-}) and wound-myCAF (ANTXR1⁺ SDC1⁻ LAMP5⁻ CD9⁺) CAFs.

In yet another embodiment, the invention concerns a method for selecting a patient for immunotherapy, wherein the method comprises:
(a) detecting ANTXR1⁺ fibroblasts or CAFs in a cancer sample from said patient;
(b) determining the percentage of ANTXR1⁺ fibroblasts or CAFs in said cancer sample, and wherein a percentage of ANTXR1⁺ CAFs lower than 25%, preferably lower than 20%, more preferably lower than 10%, still more preferably lower than 5%, yet more preferably lower than 1%, even more preferably lower than 0,5%; is predictive of the responsiveness of said patient to an immunotherapy treatment.

The method may further comprise a step c) of administration of an immunotherapy, preferably the immunotherapy being an immune checkpoint inhibitor.

Optionally, the method further comprises a previous step of providing a cancer sample from said patient. In yet another embodiment, the invention concerns a method for selecting a patient for an alternative anti-cancer treatment, in particular excluding immunotherapy or comprising wherein the method comprises:
(a) detecting ANTXR1⁺ fibroblasts or CAFs in a cancer sample from said patient;
(b) determining the percentage of ANTXR1⁺ fibroblasts or CAFs in said cancer sample, and wherein a percentage of ANTXR1⁺ CAFs higher than 1%, preferably higher than 5%, more preferably higher than 10%, still more preferably higher than 20%, yet more preferably higher than 30%, even more preferably higher than 40%; is indicative or predictive of the non-responsiveness of said patient to an immunotherapy treatment.

The method may further comprise a step c) of administration of an alternative treatment, preferably selected from the group consisting of surgery, chemotherapy, radiation therapy, hormonal therapy, targeted therapy and palliative care or any combination thereof.

Optionally, the method further comprises a previous step of providing a cancer sample from said patient.

The present invention further relates to an immunotherapy treatment for use in the treatment of a cancer in a patient wherein the patient presents in a cancer sample having (a) low number or percentage of ANTXR1⁺ fibroblasts or CAFs; or (b) no ANTXR1⁺ fibroblasts or CAFs.

ANTXR1⁺ fibroblasts or CAFs are FAP⁺ ANTXR1⁺ fibroblasts or CAFs. Optionally, the ANTXR1⁺ CAFs are ecm-myCAF (ANTXR1⁺ SDC1⁺ LAMP5⁻) CAFs. Optionally, the ANTXR1⁺ CAFs are TGFb-myCAF (ANTXR1⁺ LAMP5⁺ SDC1^{+/-}) CAFs. Optionally; the ANTXR1⁺ CAFs are wound-myCAF (ANTXR1⁺ SDC1⁻ LAMP5⁻ CD9⁺) CAFs. Optionally, the ANTXR1⁺ CAFs are ecm-myCAF (ANTXR1⁺ SDC1⁺ LAMP5⁻) and TGFb-myCAF (ANTXR1⁺ LAMP5⁺ SDC1^{+/-}) CAFs. Optionally, the ANTXR1⁺ CAFs are ecm-myCAF (ANTXR1⁺ SDC1⁺ LAMP5⁻) and wound-myCAF (ANTXR1⁺ SDC1⁻ LAMP5⁻ CD9⁺) CAFs. Optionally, the ANTXR1⁺ CAFs are TGFb-myCAF (ANTXR1⁺ LAMP5⁺ SDC1^{+/-}) and wound-myCAF (ANTXR1⁺ SDC1⁻ LAMP5⁻ CD9⁺) CAFs. Optionally, the ANTXR1⁺ CAFs are ecm-myCAF (ANTXR1⁺ SDC1⁺ LAMP5⁻), TGFb-myCAF (ANTXR1⁺ LAMP5⁺ SDC1^{+/-}) and wound-myCAF (ANTXR1⁺ SDC1⁻ LAMP5⁻ CD9⁺) CAFs.

### Immunotherapy treatment

The immunosuppressive fibroblasts, in particular immunosuppressive CAFs, according to the invention can be used in the prediction or evaluation of the response to immunotherapy.

Preferably, the immunotherapy treatment is selected from the group consisting of therapeutic treatments that stimulate the patient's immune system to attack the malignant tumor cells, immunization of the patient with tumor antigens, e.g. by administering a cancer vaccine, administration of molecules stimulating the immune system such as cytokines, administration of therapeutic antibodies, preferably monoclonal antibodies, as drugs, in particular antibodies directed against antigens specifically presented or overexpressed at the membrane of tumor cells or directed against cell receptors which blockade prevent tumor growth, adoptive T-cell therapy, immune checkpoint inhibitor treatment, and any combination thereof, preferably an immune checkpoint inhibitor treatment.

In a preferred embodiment, the immunotherapy treatment is an immune checkpoint inhibitor treatment, preferably selected from the group consisting of an antibody directed against an anti-CTLA-4 (cytotoxic T lymphocyte associated protein 4) such as ipilimumab, an antibody directed against PD-1 (programmed cell death protein 1) such as nivolumab, pembrolizumab, or BGB-A317, an antibody directed against PDL1 (programmed cell death ligand) such as atezolizumab, avelumab, or durvalumab, an antibody directed against LAG-3 (Lymphocyte-activation gene 3) such as BMS-986016, an antibody directed against TIM-3 (T-cell immunoglobulin and mucin-domain containing-3), an antibody directed against TIGIT (T cell immunoreceptor with Ig and ITIM domains), an antibody directed against BLTA (B- and T-lymphocyte attenuator), IDO1 inhibitors such as epacadostat, or a combination thereof.

In a most preferred aspect, the immunotherapy is an immune checkpoint inhibitor therapy, wherein the immune checkpoint is selected from the group consisting of c an antibody directed against cytotoxic T lymphocyte associated protein (CTLA-4), programmed cell death protein 1 (PD-1), programmed cell death ligand (PD-L1), T cell immunoreceptor with Ig and ITIM domains (TIGIT), Lymphocyte-activation gene 3 (LAG-3), T-cell immunoglobulin and mucin-domain containing-3 (TIM-3), B- and T-lymphocyte attenuator (BLTA), IDO1 inhibitor, or any combination thereof, preferably selected from the group consisting of an antibody directed against CTLA-4, PD-1 and TIGIT or any combination thereof, more preferably an antibody directed against PD-1 or CTLA-4 and the combination thereof.

Several anti-PD-1 antibodies are already clinically approved and others are still in clinical developments. For instance, the anti-PD1 antibody can be selected from the group consisting of Pembrolizumab (also known as Keytruda lambrolizumab, MK-3475), Nivolumab (Opdivo, MDX-1106, BMS-936558, ONO-4538), Pidilizumab (CT-011), Cemiplimab (Libtayo), Camrelizumab, AUNP12, AMP-224, AGEN-2034, BGB-A317 (Tisleizumab), PDR001 (spartalizumab), MK-3477, SCH-900475, PF-06801591, JNJ-63723283, genolimzumab (CBT-501), LZM-009, BCD-100, SHR-1201, BAT-1306, AK-103 (HX-008), MEDI-0680 (also known as AMP-514) MEDI0608, JS001 (see Si-Yang Liu et al., J. Hematol. Oncol.10:136 (2017)), BI-754091, CBT-501, INCSHR1210 (also known as SHR-1210), TSR-042 (also known as ANB011), GLS-010 (also known as WBP3055), AM-0001 (Armo), STI-1110 (see WO 2014/194302), AGEN2034 (see WO 2017/040790), MGA012 (see WO 2017/19846), or IBI308 (see WO 2017/024465, WO 2017/025016, WO 2017/132825, and WO 2017/133540), monoclonal antibodies 5C4, 17D8, 2D3, 4H1, 4A11, 7D3, and 5F4, described in WO 2006/121168. Bifunctional or bispecific molecules targeting PD-1 are also known such as RG7769 (Roche), XmAb20717 (Xencor), MEDI5752 (AstraZeneca), FS118 (F-star), SL-279252 (Takeda) and XmAb23104 (Xencor).

Antibodies directed against CTLA-4 and bifunctional or bispecific molecules targeting CTLA-4 are also known such as ipilimumab, tremelimumab, MK-1308, AGEN-1884, XmAb20717 (Xencor), MEDI5752 (AstraZeneca).

Antibodies directed against TIGIT are also known in the art, such as BMS-986207 or AB154, BMS-986207 CPA.9.086, CHA.9.547.18, CPA.9.018, CPA.9.027, CPA.9.049, CPA.9.057, CPA.9.059, CPA.9.083, CPA.9.089, CPA.9.093, CPA.9.101, CPA.9.103, CHA.9.536.1, CHA.9.536.3, CHA.9.536.4, CHA.9.536.5, CHA.9.536.6, CHA.9.536.7, CHA.9.536.8, CHA.9.560.1, CHA.9.560.3, CHA.9.560.4, CHA.9.560.5, CHA.9.560.6, CHA.9.560.7, CHA.9.560.8, CHA.9.546.1, CHA.9.547.1, CHA.9.547.2, CHA.9.547.3, CHA.9.547.4, CHA.9.547.6, CHA.9.547.7, CHA.9.547.8, CHA.9.547.9, CHA.9.547.13, CHA.9.541.1, CHA.9.541.3, CHA.9.541.4, CHA.9.541.5, CHA.9.541.6, CHA.9.541.7, and CHA.9.541.8 as disclosed in WO19232484. Anti-TIGIT antibodies are also disclosed in WO16028656, WO16106302, WO16191643, WO17030823, WO17037707, WO17053748, WO17152088, WO18033798, WO18102536, WO18102746, WO18160704, WO18200430, WO18204363, WO19023504, WO19062832, WO19129221, WO19129261, WO19137548, WO19152574, WO19154415, WO19168382 and WO19215728.

Preferably, the immunotherapy is selected from the group consisting of ipilimumab, nivolumab, BGB-A317, pembrolizumab, atezolizumab, avelumab, or durvalumab, BMS-986016, and epacadostat, or any combination thereof.

### ANTXR1+ fibroblasts or CAFs targeting agents

In another aspect, the invention also concerns an agent targeting ANTXR1⁺ fibroblasts or an agent targeting FAP⁺ fibroblasts or a pharmaceutical composition comprising it for its use in combination with an immunotherapy treatment for the treatment of a subject having a cancer or for its use in a subject having a cancer, the subject having ANTXR1⁺ fibroblasts, especially in a tumor sample from the subject. Indeed, a treatment reducing the immunosuppression induced by ANTXR1⁺ fibroblasts, especially ANTXR1⁺ CAFs, can be for instance an agent targeting ANTXR1⁺ fibroblasts or CAFs or a FAP inhibitor. The invention also concerns an agent targeting ANTXR1⁺ CAFs or FAP⁺ CAFs or a pharmaceutical composition comprising it for its use in combination with an immunotherapy treatment for the treatment of a subject having a cancer or for its use in a subject having a cancer, the subject having ANTXR1⁺ CAFs, especially in a tumor sample from the subject. More particularly, when using a FAP inhibitor, the subject has ANTXR1⁺ FAP⁺ fibroblasts, especially ANTXR1⁺ FAP⁺ CAFs.

In an aspect that is not claimed, the disclosure refers to ANTXR1 inhibitors. The present invention also relates to a product or kit comprising a) an agent targeting ANTXR1⁺ CAFs or FAP⁺ CAFs and b) an immunotherapy treatment, as a combined preparation for simultaneous, separate or sequential use in the treatment of a cancer in a patient, especially a patient having ANTXR1⁺ fibroblasts or CAFs. In particular, the subject has a tumor sample with medium or high percentage of ANTXR1⁺ fibroblasts or CAFs in the cancer sample.

The present invention also relates to a method for selecting a patient having a cancer for a treatment with an agent targeting ANTXR1⁺ CAFs or FAP⁺ CAFs, the method comprising detecting the immunosuppressive fibroblasts or CAFs as disclosed above and selecting the patient if the patient has ANTXR1⁺ fibroblasts or CAFs in a cancer sample from the patient.

The agent suppresses or reduces the immune-suppressive action of ANTXR1⁺ fibroblasts or CAFs.

In an aspect that is not claimed, the agent is selected from the group consisting of an ANTXR1 inhibitor, an anti-ANTXR1 antibody optionally conjugated to a cytotoxic drug, a multispecific molecule comprising an anti-ANTXR1 moiety, an anti-ANTXR1 T cell receptor (TCR), an anti-ANTXR1 chimeric antigen receptor (CAR), and an immune cell, preferably a T cell, expressing an anti-ANTXR1 TCR or an anti-ANTXR1 CAR, or any combination thereof.

Additionally or alternatively, the agent is selected from the group consisting of an FAP inhibitor, an anti-FAP antibody optionally conjugated to a cytotoxic drug, a multispecific molecule comprising an anti- FAP moiety, an anti- FAP T cell receptor (TCR), an anti- FAP chimeric antigen receptor (CAR), and an immune cell, preferably a T cell, expressing an anti- FAP TCR or an anti- FAP CAR, or any combination thereof.

In particular, ANTXR1 inhibitors can be for example ebselen or phenylmercuricacetate.

The FAP inhibitor (FAPI) is selected from the group consisting of talabostat, PT-100, linagliptin (Tradjenta), FAP inhibitors with a N-(4-quinolinoyl)-Gly-(2-cyanopyrrolidine) scaffold as disclosed in Jansen et al., ACS Med Chem Lett. 2013 May 9; 4(5): 491-496, MIP-1232 such as described in Mueller et al. J Biol Chem 1999;274:24947-52, and FAP inhibitors such as FAPI-02 and FAPI-04 for example such as described in Lindner et al. EJNMMI Radiopharmacy and Chemistry (2019) 4:16 and Lidner et al., Journal of Nuclear Medicine, published on April 6, 2018. For instance, FAP inhibitors are described in WO20081522, WO20132661, WO20245173, WO21005125, WO21005131, US2020246383, WO19154859, WO19083990, WO19118932, WO18111989, WO17189569, WO13107820, WO08116054 or WO07085895. FAP inhibitor can also be a FAP binding peptide linked to a radionuclide (e.g., Lutetium-177), such as the FAP inhibitor FAP-2286 developed by Clovis Oncology.

In a particular embodiment, the invention concerns the use of a FAP inhibitor for the treatment of a subject having a cancer or for use in the treatment of a subject having a cancer, the subject having ANTXR1⁺ fibroblasts or CAFs, especially in a tumor sample from the subject or in the microenvironment of the tumor.

In an aspect that is not claimed, the agent is an anti-ANTXR1 or anti-FAP antibody. The antibody according to the disclosure can be any kind of antibody. In particular, the antibody can comprise, consist or consist essentially in a classical Y-shaped antibody with two heavy chains and light chains or a fragment thereof. Preferably said fragment comprises the antigen binding or variable region of the antibody. Said fragment may be selected, without limitation, from the group consisting in Fv, Fab, Fab', F(ab)2, F(ab')2, F(ab)3, Fv, single-chain Fv (scFv), di-scFvs or sc(Fv)2, dsFv, Fd, dAb, CDRs, VH, VL, VHH, V-NAR, nanobodies, minibodies, diabodies, and multi-specific antibodies formed from antibodies fragments.

The antibody according to the disclosure may be a monomeric antibody or a multimeric antibody. In particular, the antibody according to the invention is a monomeric antibody.

The antibody according to the disclosure can be monoclonal or polyclonal. Preferably, the antibody according to the disclosure is monoclonal.

In another aspect that is not claimed, the targeting agent is an anti-ANTXR1 or anti-FAP antibody or a peptide binding ANTXR1 or FAP, conjugated to a drug, preferably a cytotoxic drug. Optionally, the anti-ANTXR1 or anti-FAP antibody may have an inhibitory or antagonistic activity.

The drug according to the disclosure is preferably a cytotoxic drug. As used herein, the term "cytotoxic drug" refers to a molecule that when entering in contact with a cell, eventually upon internalization into the cell, alters a cell function (e.g. cell growth and/or proliferation and/or differentiation and/or metabolism such as protein and/or DNA synthesis) in a detrimental way or leads to cell death. As used herein, the term "cytotoxic drug" encompasses toxins, in particular cytotoxins.

The cytotoxic drug according to the disclosure may be selected from the group consisting of dolastatins such as dolastin 10, dolastin 15, auristatin E, auristatin EB (AEB), auristatin EFP (AEFP), monomethyl auristatin F (MMAF), monomethylauristatin-D (MMAD), monomethyl auristatin E (MMAE), and 5-benzoylvaleric acid-AE ester (AEVB), maytansines such ansamitocin, mertansine (also called emtansine or DM1) and ravtansine (also called soravtansine or DM4), antracyclins such as daunorubicin, epirubicin, pirarubicin, idarubicin, zorubicin, cerubidin, aclarubicin, adriblastin, doxorubicin, mitoxantrone,daunoxome, nemorubicin and PNU-159682, calicheamicins such as calicheamicin beta 1Br, calicheamicin gamma 1Br, calicheamicin alpha 2I, calicheamicin alpha 3I, calicheamicin beta 1I, calicheamicin gamma 1L, calicheamicin delta 1I and ozogamicin, esperamicins such as esperamicin A1, neocarzinostatins, bleomycin, duocarymycins such as CC-1065 and duocarmycin A, pyrrolobenzodiazepines such as anthramycin, abbeymycin, chicamycin, DC-81, mazethramycin, neothramycins A and B, porothramycin prothracarcin, sibanomicin (DC-102), sibiromycin and tomamycin, pyrrolobenzodiazepine dimers (or PBD), indolino-benzodiazepines, indolino-benzodiazepine dimers, α-amanitins, abraxane, actinomycin, aldesleukin, altretamine, alitretinoin, amsacrine, anastrozole, arsenic, asparaginase, azacitidine, azathioprine, bexarotene, bendamustine, bicalutamide, bortezomib, busulfan, capecitabine, carboplatin, carmustine, chlorambucil, cisplatin, cladribine, clofarabine, cyclophosphamide, cytarabine, chloramphenicol, ciclosporin, cidofovir, coal tar containing products, colchicine, dacarbazine, dactinomycin, danazol, dasatinib, diethylstilbestrol, dinoprostone, dithranol, dutasteride, dexrazoxane, docetaxel, doxifluridine, erlotinib, estramustine, etoposide, exemestane, finasteride, flutamide, floxuridine, flucytosine, fludarabine, fluorouracil, ganciclovir, gefitinib, gemcitabine, goserelin, hydroxyurea, hydroxycarbamide, ifosfamide, irinotecan, imatinib, lenalidomide, leflunomide, letrozole, leuprorelin acetate, lomustine, mechlorethamine, melphalan, mercaptopurine, methotrexate, mitomycin, mitotane, menotropins, mifepristone, nafarelin, nelarabin, nitrogen mustard, nitrosoureas, oxaliplatin, ozogamicin, paclitaxel, podophyllyn, pegasparaginase, pemetrexed, pentamidine, pentostatin, procarbazin, raloxifene, ribavarin, raltitrexed, rituximab, romidepsin, sorafenib, streptozocin, sunitinib, sirolimus, streptozocin, temozolomide, temsirolimus, teniposide, thalidomide, thioguanine, thiotepa, topotecan, tacrolimus, taxotere, tafluposide, toremifene, tretinoin, trifluridine, triptorelin, valganciclovir, valrubicin, vinblastine, vidaradine, vincristine, vindesine, vinorelbine, vemurafenib, vismodegib, vorinostat, zidovudine, vedotine, derivatives and combinations thereof. The cytotoxic drug can also be a radionuclide such as Lutetium-177, lodine-131, samarium-153, and yttrium-90 or astatine-211, bismuth-212, lead-212, bismuth-213, actinium-225, radium-223 and thorium-227.

In an aspect that is not claimed, the antibody-drug conjugate of the disclosure comprises a linker between the antibody and the drug. The linker according to the disclosure may be cleavable or non-cleavable, preferably, the linker is cleavable. Examples of cleavable linkers according to the disclosure include, without limitations, disulfides, hydrazones and peptides. Examples of non-cleavable linkers according to the disclosure include, without limitations, thioethers.

In a particular aspect that is not claimed, the drug is linked to a cysteine or a lysine residue of the antibody. Preferably, the drug or antigen is linked to unnatural amino acids that have been incorporated into the antibody.

Methods to make antibody-drug conjugates are well known from the man skilled in the art.

In an aspect that is not claimed, the agent is an anti-ANTXR1 or anti-FAP CAR cell. The terms "Chimeric antigen receptor" (CAR), "engineered cell receptor", "chimeric cell receptor", or "chimeric immune receptor" (ICR) as used herein refer to engineered receptors, which graft an antigen binding specificity (e.g. antibody) onto immune cells (e.g. T cells or NK cells), thus combining the antigen binding properties of the antigen binding domain with the immunogenic activity of the immune cell, such as the lytic capacity and self-renewal of immune cells. CAR cells directed against ANTXR1 are available for the person skilled in the art. For instance, anti-ANTXR1 CAR T cells are described in patent application KR1020190013612, US2016264662A, US2017114133A and CN108707199A, and in publications such as in Byrd et al. (Cancer Res. 2018;78(2):489-500. 10.1158/0008-5472.CAN-16-1911).

The invention also concerns a pharmaceutical composition comprising a FAP inhibitor. In particular, such pharmaceutical composition comprises at least one pharmaceutically acceptable excipient. For this formulation, conventional excipient can be used according to techniques well known by those skilled in the art.

The formulations can be sterilized and, if desired, mixed with auxiliary agents such as pharmaceutically acceptable carriers, excipients, salts, anti-oxidant and/or stabilizers which do not deleteriously interact with the FAP CAFs targeting agent.

Optionally, the pharmaceutical composition may further comprise an additional therapeutic agent, in particular immunotherapeutic agent such as described above.

It will be understood by one skilled in the art that the formulations of the invention may be isotonic with human blood that is the formulations of the invention have essentially the same osmotic pressure as human blood. Such isotonic formulations generally have an osmotic pressure from about 250 mOSm to about 350 mOSm. Isotonicity can be measured by, for example, a vapor pressure or ice-freezing type osmometer.

The amount of ANTXR1⁺ CAFs and/or FAP⁺ CAFs targeting agent according to the invention or of the pharmaceutical composition according to the invention to be administered can be determined by standard procedure well known by those of ordinary skills in the art. Physiological data of the patient (e.g. age, size, and weight) and the routes of administration have to be taken into account to determine the appropriate dosage, so as a therapeutically effective amount will be administered to the patient.

When a combined preparation, a kit or a product for use according to the invention are administered separately or sequentially, especially when administered separately, the treatment with an ANTXR1⁺ CAFs and/or FAP⁺ CAFs targeting agent according to the invention is preferably carried out before the immunotherapy treatment. The method of treatment may further comprise a step of determining the percentage of ANTXR1⁺ CAFs and/or a FAP⁺ CAFs after the administration of the treatment with a ANTXR1⁺ CAFs and/or a FAP⁺ CAFs targeting agent according to the invention and preferably prior the administration of the immunotherapy treatment, the immunotherapy treatment being administered only if the percentage of ANTXR1⁺ CAFs is low or if ANTXR1⁺ CAFs are absent.

### Use of an immunotherapy treatment and treatment methods

In a particular aspect, the invention also concerns an immune checkpoint inhibitor treatment, for use in the treatment of a cancer in a patient wherein the patient presents in a cancer sample:
(a) low level or low percentage of ANTXR1⁺ fibroblasts, in particular ANTXR1⁺ CAFs; or
(b) no ANTXR1⁺ fibroblasts, in particular no ANTXR1⁺ CAFs.

The percentage of ANTXR1+ fibroblasts or CAFs are as described above. The ANTXR1+ fibroblasts or CAFs are as described above, the cancer is as defined below and the immunotherapy treatment are as defined above.

Preferably, the ANTXR1⁺ CAFs are selected from the group consisting of FAP⁺ ANTXR1⁺ LAMP5⁻ SDC1⁺ CAFs, FAP⁺ ANTXR1⁺ LAMP5⁺ SDC1^{+/-} CAFs, FAP⁺ ANTXR1⁺ LAMP5⁻ SDC1⁺ CAFs and/or FAP⁺ ANTXR1⁺ LAMP5⁺ SDC1^{+/-} CAFs.

The ANTXR1⁺ CAFs are FAP⁺ ANTXR1⁺ CAFs. Optionally, the ANTXR1⁺ CAFs are ecm-myCAF (ANTXR1⁺ SDC1⁺ LAMP5⁻) CAFs. Optionally, the ANTXR1⁺ CAFs are TGFb-myCAF (ANTXR1⁺ LAMP5⁺ SDC1^{+/-}) CAFs. Optionally; the ANTXR1⁺ CAFs are wound-myCAF (ANTXR1⁺ SDC1⁻ LAMP5⁻ CD9⁺) CAFs. Optionally, the ANTXR1⁺ CAFs are ecm-myCAF (ANTXR1⁺ SDC1⁺ LAMP5⁻) and TGFb-myCAF (ANTXR1⁺ LAMP5⁺ SDC1^{+/-}) CAFs. Optionally, the ANTXR1⁺ CAFs are ecm-myCAF (ANTXR1⁺ SDC1⁺ LAMP5⁻) and wound-myCAF (ANTXR1⁺ SDC1⁻ LAMP5⁻ CD9⁺) CAFs. Optionally, the ANTXR1⁺ CAFs are TGFb-myCAF (ANTXR1⁺ LAMP5⁺ SDC1^{+/-}) and wound-myCAF (ANTXR1⁺ SDC1⁻ LAMP5⁻ CD9⁺) CAFs. Optionally, the ANTXR1⁺ CAFs are ecm-myCAF (ANTXR1⁺ SDC1⁺ LAMP5⁻), TGFb-myCAF (ANTXR1⁺ LAMP5⁺ SDC1^{+/-}) and wound-myCAF (ANTXR1⁺ SDC1⁻ LAMP5⁻ CD9⁺) CAFs. Preferably, the immune checkpoint inhibitor immunotherapy is selected from the group consisting of an anti-CTLA-4 antibody, anti-PD-1 antibody and anti-TIGIT antibody or any combination thereof, more preferably an anti-CTLA-4 antibody and/or anti-PD-1 antibody.

Particularly, the amount of ANTXR1⁺ CAFs and/or FAP⁺ CAFs targeting agent according to the invention or of the pharmaceutical composition according to the invention to be administered can be determined by standard procedure well known by those of ordinary skills in the art. Physiological data of the patient (e.g. age, size, and weight) and the routes of administration have to be taken into account to determine the appropriate dosage, so as a therapeutically effective amount will be administered to the patient.

In some embodiments, the immunotherapy treatment, the agent targeting ANTXR1⁺ fibroblasts, the agent targeting FAP⁺ fibroblasts and/or the pharmaceutical composition according to the invention is to be administered in combination with additional cancer therapies. In particular, the immunotherapy treatment, the agent targeting ANTXR1⁺ fibroblasts, the agent targeting FAP⁺ fibroblasts and/or the pharmaceutical composition of the invention may be administered in combination with other targeted therapy, other immunotherapy, chemotherapy and/or radiotherapy.

In some embodiments, the immunotherapy treatment, the agent targeting ANTXR1⁺ fibroblasts, the agent targeting FAP⁺ fibroblasts and/or the pharmaceutical composition of the invention is to be administered to the patient in combination with chemotherapy. As used herein, the term "chemotherapy" has its general meaning in the art and refers to the treatment that consists in administering to the patient a chemotherapeutic agent. Chemotherapeutic agents include, but are not limited to alkylating agents such as thiotepa and cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e.g. , calicheamicin, especially calicheamicin gammall and calicheamicin omegall ; dynemicin, including dynemicin A; bisphosphonates, such as clodronate; an esperamicin; as well as neocarzinostatin chromophore and related chromoprotein enediyne antiobiotic chromophores, aclacinomysins, actinomycin, authrarnycin, azaserine, bleomycins, cactinomycin, carabicin, caminomycin, carzinophilin, chromomycinis, dactinomycin, daunorubicin, detorubicin, 6-diazo-5-oxo-L-norleucine, doxorubicin (including morpholino-doxorubicin, cyanomorpholino-doxorubicin, 2-pyrrolino-doxorubicin and deoxy doxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elformithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2-ethylhydrazide; methylhydrazine derivatives including N-methylhydrazine (MIH) and procarbazine; PSK polysaccharide complex); razoxane; rhizoxin; sizofuran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g., paclitaxel and doxetaxel; chlorambucil; gemcitabine; 6-thioguanine; mercaptopurine; methotrexate; platinum coordination complexes such as cisplatin, oxaliplatin and carboplatin; vinblastine; platinum; etoposide (VP- 16); ifosfamide; mitoxantrone; vincristine; vinorelbine; novantrone; teniposide; edatrexate; daunomycin; aminopterin; xeloda; ibandronate; irinotecan (e.g., CPT-1 1); topoisomerase inhibitor RFS 2000; difluoromethylomithine (DMFO); retinoids such as retinoic acid; capecitabine; anthracyclines, nitrosoureas, antimetabolites, epipodophylotoxins, enzymes such as L-asparaginase; anthracenediones; hormones and antagonists including adrenocorticosteroid antagonists such as prednisone and equivalents, dexamethasone and aminoglutethimide; progestin such as hydroxyprogesterone caproate, medroxyprogesterone acetate and megestrol acetate; estrogen such as diethylstilbestrol and ethinyl estradiol equivalents; antiestrogen such as tamoxifen; androgens including testosterone propionate and fluoxymesterone/equivalents; antiandrogens such as flutamide, gonadotropin-releasing hormone analogs and leuprolide; and non-steroidal antiandrogens such as flutamide; and pharmaceutically acceptable salts, acids or derivatives of any of the above.

In some embodiments, the immunotherapy treatment, the agent targeting ANTXR1⁺ fibroblasts, the agent targeting FAP⁺ fibroblasts and/or the pharmaceutical composition of the invention is to be administered to the patient in combination with radiotherapy. Suitable examples of radiation therapies include, but are not limited to external beam radiotherapy (such as superficial X-rays therapy, orthovoltage X-rays therapy, megavoltage X-rays therapy, radiosurgery, stereotactic radiation therapy, Fractionated stereotactic radiation therapy, cobalt therapy, electron therapy, fast neutron therapy, neutron-capture therapy, proton therapy, intensity modulated radiation therapy (IMRT), 3-dimensional conformal radiation therapy (3D-CRT) and the like); brachytherapy; unsealed source radiotherapy; tomotherapy; and the like. Gamma rays are another form of photons used in radiotherapy. Gamma rays are produced spontaneously as certain elements (such as radium, uranium, and cobalt 60) release radiation as they decompose, or decay.

In some embodiments, radiotherapy may be proton radiotherapy or proton minibeam radiation therapy. Proton radiotherapy is an ultra-precise form of radiotherapy that uses proton beams (Prezado Y, Jouvion G, Guardiola C, Gonzalez W, Juchaux M, Bergs J, Nauraye C, Labiod D, De Marzi L, Pouzoulet F, Patriarca A, Dendale R. Tumor Control in RG2 Glioma-Bearing Rats: A Comparison Between Proton Minibeam Therapy and Standard Proton Therapy. Int J Radiat Oncol Biol Phys. 2019 Jun 1;104(2):266-271. doi: 10.1016/j.ijrobp.2019.01.080; Prezado Y, Jouvion G, Patriarca A, Nauraye C, Guardiola C, Juchaux M, Lamirault C, Labiod D, Jourdain L, Sebrie C, Dendale R, Gonzalez W, Pouzoulet F. Proton minibeam radiation therapy widens the therapeutic index for high-grade gliomas. Sci Rep. 2018 Nov 7;8(1):16479. doi: 10.1038/s41598-018-34796-8). Radiotherapy may also be FLASH radiotherapy (FLASH-RT) or FLASH proton irradiation. FLASH radiotherapy involves the ultra-fast delivery of radiation treatment at dose rates several orders of magnitude greater than those currently in routine clinical practice (ultra-high dose rate) (Favaudon V, Fouillade C, Vozenin MC. The radiotherapy FLASH to save healthy tissues. Med Sci (Paris) 2015 ; 31 : 121-123. DOI: 10.1051/medsci/20153102002); Patriarca A., Fouillade C. M., Martin F., Pouzoulet F., Nauraye C., et al. Experimental set-up for FLASH proton irradiation of small animals using a clinical system. Int J Radiat Oncol Biol Phys, 102 (2018), pp. 619-626. doi: 10.1016/j.ijrobp.2018.06.403. Epub 2018 Jul 11).

### Patient, regimen and administration

The patient is an animal, preferably a mammal, even more preferably a human. However, the patient can also be a non-human animal, in particular mammals such as dogs, cats, horses, cows, pigs, sheep, donkeys, rabbits, ferrets, gerbils, hamsters, chinchillas, rats, mice, guinea pigs and non-human primates, among others, that are in need of treatment.

The human patient according to the invention may be a human at the prenatal stage, a new-born, a child, an infant, an adolescent or an adult, in particular an adult of at least 30 years old or at least 40 years old, preferably an adult of at least 50 years old, still more preferably an adult of at least 60 years old, even more preferably an adult of at least 70 years old.

In one embodiment, the patient is an active smoker or a former smoker.

Preferably, the patient has been diagnosed with a cancer. In another particular embodiment, the patient suffers from a metastatic cancer or a cancer at an advanced stage. In one embodiment, the patient has been diagnosed with a cancer of stage III or IV.

In one embodiment, the patient suffering from cancer has metastasis, in particular brain, liver, bone and/or suprarenal gland metastasis.

In a particular embodiment, the patient has already received at least one line of treatment, in particular one line of treatment, two lines of treatment or three lines of treatment or more, preferably several lines of treatment. Alternatively, the patient has not received any treatment. Particularly, the patient already received nivolumab, pembrolizumab, ipilumumab or any combination thereof.

The cancer treatment, in particular the immunotherapy treatment or the treatment comprising an anti-FAP agent, can be administered by any conventional route of administration, such as topical, enteral, oral, parenteral, intranasal, intravenous, intramuscular, subcutaneous or intraocular route of administration and the like.

In particular, the cancer treatment can be immunotherapy, surgery, chemotherapy, radiation therapy, hormonal therapy, targeted therapy, palliative care, treatment comprising an anti-FAP agent and any combination thereof.

Preferably, the cancer treatment starts no longer than a month, preferably no longer than a week, after the determination of the presence of ANTXR1+ fibroblasts, in particular ANTXR1⁺ CAFs.

The cancer treatment may be administered as a single dose or in multiple doses.

Preferably, the cancer treatment is administered regularly, preferably between every day and every month, more preferably between every day and every two weeks, even more preferably between every day and every week.

The duration of treatment is preferably comprised between 1 day and 24 weeks, more preferably between 1 day and 10 weeks, even more preferably between 1 day and 4 weeks. In a particular embodiment, the treatment last as long as the cancer persists.

The amount of cancer treatment, in particular the immunotherapy treatment or the treatment comprising an anti-FAP agent, to be administered is determined by standard procedure well known by those of ordinary skills in the art. Physiological data of the patient (e.g. age, size, weight, and physical general condition) and the routes of administration are taken into account to determine the appropriate dosage, so as a therapeutically effective amount will be administered to the patient.

In the context of a combination of active ingredients, the active ingredients can be administered to the subject by the same or different routes of administration. Administration routes usually depend on the pharmaceutical compositions used.

### Cancer

The method of the invention is aimed to select and/or treat a patient affected with a tumor.

In one embodiment, the tumor is from a cancer selected from the group consisting of leukemias, seminomas, melanomas, teratomas, lymphomas, non-Hodgkin lymphoma, neuroblastomas, gliomas, adenocarninoma, mesothelioma (including pleural mesothelioma, peritoneal mesothelioma, pericardial mesothelioma and end stage mesothelioma), rectal cancer, endometrial cancer, thyroid cancer (including papillary thyroid carcinoma, follicular thyroid carcinoma, medullary thyroid carcinoma, undifferentiated thyroid cancer, multiple endocrine neoplasia type 2A, multiple endocrine neoplasia type 2B, familial medullary thyroid cancer, pheochromocytoma and paraganglioma), skin cancer (including malignant melanoma, basal cell carcinoma, squamous cell carcinoma, Karposi's sarcoma, keratoacanthoma, moles, dysplastic nevi, lipoma, angioma and dermatofibroma), nervous system cancer, brain cancer (including astrocytoma, medulloblastoma, glioma, lower grade glioma, ependymoma, germinoma (pinealoma), glioblastoma multiform, oligodendroglioma, schwannoma, retinoblastoma, congenital tumors, spinal cord neurofibroma, glioma or sarcoma), skull cancer (including osteoma, hemangioma, granuloma, xanthoma or osteitis deformans), meninges cancer (including meningioma, meningiosarcoma or gliomatosis), head and neck cancer (including head and neck squamous cell carcinoma and oral cancer (such as, e.g., buccal cavity cancer, lip cancer, tongue cancer, mouth cancer or pharynx cancer)), lymph node cancer, gastrointestinal cancer, liver cancer (including hepatoma, hepatocellular carcinoma, cholangiocarcinoma, hepatoblastoma, angiosarcoma, hepatocellular adenoma and hemangioma), colon cancer, stomach or gastric cancer, esophageal cancer (including squamous cell carcinoma, larynx, adenocarcinoma, leiomyosarcoma or lymphoma), colorectal cancer, intestinal cancer, small bowel or small intestines cancer (such as, e.g., adenocarcinoma lymphoma, carcinoid tumors, Karposi's sarcoma, leiomyoma, hemangioma, lipoma, neurofibroma or fibroma), large bowel or large intestines cancer (such as, e.g., adenocarcinoma, tubular adenoma, villous adenoma, hamartoma or leiomyoma), pancreatic cancer (including ductal adenocarcinoma, insulinoma, glucagonoma, gastrinoma, carcinoid tumors or vipoma), ear, nose and throat (ENT) cancer, breast cancer (including HER2-enriched breast cancer, luminal A breast cancer, luminal B breast cancer and triple negative breast cancer), cancer of the uterus (including endometrial cancer such as endometrial carcinomas, endometrial stromal sarcomas and malignant mixed Müllerian tumors, uterine sarcomas, leiomyosarcomas and gestational trophoblastic disease), ovarian cancer (including dysgerminoma, granulosa-theca cell tumors and Sertoli-Leydig cell tumors), cervical cancer, vaginal cancer (including squamous-cell vaginal carcinoma, vaginal adenocarcinoma, clear cell vaginal adenocarcinoma, vaginal germ cell tumors, vaginal sarcoma botryoides and vaginal melanoma), vulvar cancer (including squamous cell vulvar carcinoma, verrucous vulvar carcinoma, vulvar melanoma, basal cell vulvar carcinoma, Bartholin gland carcinoma, vulvar adenocarcinoma and erythroplasia of Queyrat), genitourinary tract cancer, kidney cancer (including clear renal cell carcinoma, chromophobe renal cell carcinoma, papillary renal cell carcinoma, adenocarcinoma, Wilm's tumor, nephroblastoma, lymphoma or leukemia), adrenal cancer, bladder cancer, urethra cancer (such as, e.g., squamous cell carcinoma, transitional cell carcinoma or adenocarcinoma), prostate cancer (such as, e.g., adenocarcinoma or sarcoma) and testis cancer (such as, e.g., seminoma, teratoma, embryonal carcinoma, teratocarcinoma, choriocarcinoma, sarcoma, interstitial cell carcinoma, fibroma, fibroadenoma, adenomatoid tumors or lipoma), lung cancer (including small cell lung carcinoma (SCLC), non-small cell lung carcinoma (NSCLC) including squamous cell lung carcinoma, lung adenocarcinoma (LUAD), and large cell lung carcinoma, bronchogenic carcinoma, alveolar carcinoma, bronchiolar carcinoma, bronchial adenoma, lung sarcoma, chondromatous hamartoma and pleural mesothelioma), sarcomas (including Askin's tumor, sarcoma botryoides, chondrosarcoma, Ewing's sarcoma, malignant hemangioendothelioma, malignant schwannoma, osteosarcoma and soft tissue sarcomas), soft tissue sarcomas (including alveolar soft part sarcoma, angiosarcoma, cystosarcoma phyllodes, dermatofibrosarcoma protuberans, desmoid tumor, desmoplastic small round cell tumor, epithelioid sarcoma, extraskeletal chondrosarcoma, extraskeletal osteosarcoma, fibrosarcoma, gastrointestinal stromal tumor (GIST), hemangiopericytoma, hemangiosarcoma, Kaposi's sarcoma, leiomyosarcoma, liposarcoma, lymphangiosarcoma, lymphosarcoma, malignant peripheral nerve sheath tumor (MPNST), neurofibrosarcoma, plexiform fibrohistiocytic tumor, rhabdomyosarcoma, synovial sarcoma and undifferentiated pleomorphic sarcoma, cardiac cancer (including sarcoma such as, e.g., angiosarcoma, fibrosarcoma, rhabdomyosarcoma or liposarcoma, myxoma, rhabdomyoma, fibroma, lipoma and teratoma), bone cancer (including osteogenic sarcoma, osteosarcoma, fibrosarcoma, malignant fibrous histiocytoma, chondrosarcoma, Ewing's sarcoma, malignant lymphoma and reticulum cell sarcoma, multiple myeloma, malignant giant cell tumor chordoma, osteochronfroma, osteocartilaginous exostoses, benign chondroma, chondroblastoma, chondromyxoid fibroma, osteoid osteoma and giant cell tumors), hematologic and lymphoid cancer, blood cancer (including acute myeloid leukemia, chronic myeloid leukemia, acute lymphoblastic leukemia, chronic lymphocytic leukemia, myeloproliferative diseases, multiple myeloma and myelodysplasia syndrome), Hodgkin's disease, non-Hodgkin's lymphoma and hairy cell and lymphoid disorders, and the metastases thereof.

Preferably, the tumor is from a cancer selected from the group consisting of prostate cancer, lung cancer, Non-Small Cell Lung Carcinoma (NSCLC), breast cancer, gastric cancer, kidney cancer, ovarian cancer, hepatocellular cancer, osteosarcoma, melanoma, hypopharynx cancer, esophageal cancer, endometrial cancer, cervical cancer, pancreatic cancer, liver cancer, colon or colorectal cancer, neuroendocrine tumors, muscle cancer, adrenal cancer, adenocarcinoma, sarcoma, non-Squamous cell carcinoma, Squamous cell carcinoma, thyroid cancer, uterine cancer, skin cancer, melanoma, metastatic melanoma, bladder cancer, head and neck cancer. More preferably, the cancer is selected from the group consisting of ovarian cancer, breast cancer, adenocarcinoma, sarcoma, non-Squamous cell carcinoma, Squamous cell carcinoma, lung cancers, NSCLC, colorectal cancers, pancreatic cancers, head and neck cancer, melanoma and metastatic melanoma. In a very particular aspect, the cancer is selected from the group consisting of head and neck cancer, NSCLC, melanoma and metastatic melanoma.

In one embodiment, the cancer is an ovarian cancer, preferably a mesenchymal ovarian cancer, in particular a high-grade ovarian cancer of the serous type, or a breast cancer, preferably an invasive breast cancer and/or its metastasis, in particular axillary metastasis.

In another embodiment, the cancer is NSCLC or head and neck cancer.

In one embodiment, the cancer is sarcoma, in particular a fibroblastic sarcoma.

In another embodiment, the is cancer is selected from the group consisting of adenocarcinoma, non-Squamous cell carcinoma and Squamous cell carcinoma.

In particular, the cancer of the patient or a cancer sample from said patient or the microenvironment of the tumor presents immunosuppressive fibroblasts, in particular immunosuppressive CAFs, in particular ANTXR1⁺ FAP⁺ CAFs.

Preferably, the cancer of the patient or a cancer sample from said patient presents ANTXR1⁺ CAFs, preferably FAP⁺ ANTXR1⁺ LAMP5⁻ SDC1⁺ CAFs, FAP⁺ ANTXR1⁺ LAMP5⁺ SDC1^{+/-} CAFs, and/or ANTXR1⁺ SDC1⁻ LAMP5⁻ CD9⁺, preferably FAP⁺ ANTXR1⁺ LAMP5⁻ SDC1⁺ CAFs and/or FAP⁺ ANTXR1⁺ LAMP5⁺ SDC1^{+/-} CAFs. Optionally, the ANTXR1⁺ CAFs are FAP⁺ ANTXR1⁺ CAFs. Optionally, the ANTXR1⁺ CAFs are ecm-myCAF (ANTXR1⁺ SDC1⁺ LAMP5⁻) CAFs. Optionally, the ANTXR1⁺ CAFs are TGFb-myCAF (ANTXR1⁺ LAMP5⁺ SDC1^{+/-}) CAFs. Optionally; the ANTXR1⁺ CAFs are wound-myCAF (ANTXR1⁺ SDC1⁻ LAMP5⁻ CD9⁺) CAFs. Optionally, the ANTXR1⁺ CAFs are ecm-myCAF (ANTXR1⁺ SDC1⁺ LAMP5⁻) and TGFb-myCAF (ANTXR1⁺ LAMP5⁺ SDC1^{+/-}) CAFs. Optionally, the ANTXR1⁺ CAFs are ecm-myCAF (ANTXR1⁺ SDC1⁺ LAMP5⁻) and wound-myCAF (ANTXR1⁺ SDC1⁻ LAMP5⁻ CD9⁺) CAFs. Optionally, the ANTXR1⁺ CAFs are TGFb-myCAF (ANTXR1⁺ LAMP5⁺ SDC1^{+/-}) and wound-myCAF (ANTXR1⁺ SDC1⁻ LAMP5⁻ CD9⁺) CAFs. Optionally, the ANTXR1⁺ CAFs are ecm-myCAF (ANTXR1⁺ SDC1⁺ LAMP5⁻), TGFb-myCAF (ANTXR1⁺ LAMP5⁺ SDC1^{+/-}) and wound-myCAF (ANTXR1⁺ SDC1⁻ LAMP5⁻ CD9⁺) CAFs.

### Kit

Furthermore, the present invention provides a kit useful for carrying out the method disclosed herein, the kit comprising a mean capable of detecting FAP⁺ ANTXR1⁺ CAFs, even more preferably FAP⁺ ANTXR1⁺ LAMP5⁻ SDC1⁺ CAFs, FAP⁺ ANTXR1⁺ LAMP5⁺ SDC1^{+/-} CAFs, and/or ANTXR1⁺ SDC1⁻ LAMP5⁻ CD9⁺ CAFs.

For example, said kit may comprise mean(s) required for the detection of FAP⁺ ANTXR1⁺ CAFs, even more preferably FAP⁺ ANTXR1⁺ LAMP5⁻ SDC1⁺ CAFs, FAP⁺ ANTXR1⁺ LAMP5⁺ SDC1^{+/-} CAFs, and/or ANTXR1⁺ SDC1⁻ LAMP5⁻ CD9⁺ CAFs. The skilled person knows which mean(s) is (are) required for specifically determining the presence of ANTXR1, preferably the presence of FAP and ANTXR1, even more preferably the presence of FAP, ANTXR1, LAMP5 and SDC1; FAP, ANTXR1, LAMP5 and SDC1; and/or ANTXR1, SDC1, LAMP5 and CD9. The presence can be detected at the nucleic acid level, in particular mRNA, or at the protein level. For example, such mean(s) may be probe(s), primer(s), antibody(ies) and/or aptamer(s) specific for the detection of FAP⁺ ANTXR1⁺ CAFs, even more preferably FAP⁺ ANTXR1⁺ LAMP5⁻ SDC1⁺ CAFs, FAP⁺ ANTXR1⁺ LAMP5⁺ SDC1^{+/-} CAFs, and/or ANTXR1⁺ SDC1⁻ LAMP5⁻ CD9⁺ CAFs. In particular, such means are antibodies directed against ANTXR1, FAP, LAMP5 and SDC1; and/or CD9 such as disclosed herein. Alternatively, such means can be probes and/or primers specific for ANTXR1, FAP, LAMP5 and SDC1; and/or CD9. Additionally or alternatively, the kit comprises means, in particular nucleic acid(s), probe(s) or primer(s) targeting at least one gene of the gene signature of ecm-myCAF, TGFβ-myCAF and/or wound-myCAF such as described above. More particular, it comprises means for detecting all the gene of the gene signature ecm-myCAF, TGFβ-myCAF and/or wound-myCAF. In particular, the means are primers and/or probes.

The kit of may be a diagnostic kit. The components of the kits may be packaged either in aqueous media or in lyophilized form. The container means of the kits will generally include at least one vial, test tube, flask, bottle, syringe or other container means, into which a component may be placed, and preferably, suitably aliquoted. Where there is more than one component in the kit, the kit also will generally contain a second, third or other additional container into which the additional components may be separately placed. However, various combinations of components may be comprised in a vial.

In some embodiments, means of taking a sample from an individual and/or of assaying the sample may be provided. The kit may also comprise means for containing a sterile, pharmaceutically acceptable buffer(s) and/or other diluent(s). Optionally, a leaflet is provided for guidelines to use such a kit.

In another aspect, the invention also concerns the use of a kit as disclosed above for:
- detecting immunosuppressive Cancer Associated Fibroblast (CAFs) in a cancer sample from a subject suffering from cancer;
- predicting a response of a subject suffering from cancer to an immunotherapy treatment;
- selecting or not a patient having a cancer for an immunotherapy treatment;
- selecting or not a patient having a cancer for a treatment with an agent targeting ANTXR1⁺ CAFs;
- selecting or not a patient having a cancer for a treatment with an agent targeting ANTXR1⁺ CAFs and an immunotherapy treatment.

Further aspects and advantages of the present invention will be described in the following examples, which should be regarded as illustrative and not limiting.

### EXEMPLES

Distinct cellular clusters are identified within the immunosuppressive CAF-S1 subset by single-cell approach. The inventor used single-cell RNA sequencing (scRNA-seq) to investigate cellular heterogeneity within the CAF-S1 immunosuppressive subset. CAF-S1 fibroblasts were isolated from human BC (see description of prospective cohort 1 in **Table 1)** by FACS as previously described (Costa A, et al. Cancer Cell 2018;33(3):463-79 e10). In brief, from freshly resected tumors, the inventor first excluded debris, dead cells, doublets, epithelial (EPCAM⁺), hematopoietic (CD45⁺), endothelial (CD31⁺) and red blood (CD235a⁺) cells (Fig. 7A). The inventor considered EPCAM⁻ CD45⁻ CD31⁻ CD235a⁻ as the fraction of cells enriched in fibroblasts and next performed FAP and CD29 staining (Fig. 7A), which enabled to distinguish CAF-S1 (FAP^{high} CD29^{Med-High}) from the other CAF subpopulations (CAF-S2: FAP^{Neg} CD29^{Low}; CAF-S3: FAP^{Neg} CD29^{Med}; CAF-S4: FAP^{Neg} CD29^{High}), as previously established in Costa A, et al. Cancer Cell 2018;33(3):463-79 e10). The inventor then performed scRNA-seq of 18 805 CAF-S1 fibroblasts from 7 BC patients prior any treatment. After quality control, 18 296 CAF-S1 fibroblasts with a median of 2428 genes detected per cell were conserved for further analyses. Unsupervised graph-based clustering identified 8 CAF-S1 clusters, visualized with the Uniform Manifold Approximation and Projection (UMAP) algorithm (Fig. 1). All clusters were found in most patients, albeit it at varying levels. No individual cluster was associated with a particular phase of cell cycle or with high proliferation, as shown using G1/S and G2/M gene signatures (Tirosh I, et al. Science 2016;352(6282). The inventor confirmed the detection of these different CAF-S1 cellular clusters using the Label Transfer algorithm described in Stuart T, et al. Cell 2019;177(7):1888-902 e21. Indeed, this algorithm successfully transferred with high prediction scores all the 8 cluster labels in an independent CAF-S1 scRNA-seq dataset, newly generated from an 8^{th} BC patient.

Differential gene expression analyses revealed that each cluster was characterized by a specific transcriptional profile. Importantly, the CAF-S1 signature published in Costa A, et al. Cancer Cell 2018;33(3):463-79e10, in Givel et al. Nat Commun 2018;9(1):1056 and in Kieffer et al. Cancer Discov. 2020;10(9):1330-1351 comprises genes that are not restricted to specific CAF-S1 clusters (Fig. 11). Cluster 0 was associated with ECM remodeling, cell-substrate adhesion and collagen formation; cluster 1 with detoxification and inflammatory response; cluster 2 with response to growth factor, TNF signaling and Interleukin pathway; cluster 3 with TGFβ signaling pathway and matrisome; cluster 4 with assembly of collagen fibrils and wound healing; cluster 5 with response to Interferon y (IFNy and cytokine-mediated signaling pathway; cluster 6 with IFNβα signaling; and cluster 7 with acto-myosin complex. As examples, the inventor found high expression of *LRRC15 (Leucine Rich Repeat Containing 15*)*,* a marker recently identified in CAF from pancreatic cancer and *GBJ2* (*Gap junction protein beta 2*) in cluster 0, *ADH1B* (*Alcohol dehydrogenase 1)* and *GPX3* (*Glutathione peroxidase 3*) in cluster 1, RGMA (Repulsive guidance molecule BMP co-receptor) and *SCARA5 (Scavenger receptor class A member 5)* in cluster 2, *CST1 (Cystatin)* and *TGFβ1* in cluster 3, *SEMA3C* (*Semaphorin 3C*) and *SFRP4 (Secreted Frizzled Related Protein 4*) in cluster 4, *CCL19* and *CCL5* (*CC motif chemokine ligand 19 and 5)* in cluster 5, *IFIT3* (*Interferon induced protein with tetratricopeptide repeats 3)* and *IRF7* (*Interferon regulatory factor 7*) in cluster 6, *GGH* (*y-glutamyl hydrolase*) and *PLP2* (*Proteolipid protein 2*) in cluster 7. Interestingly, in human BC, the inventor were able to distinguish the myofibroblastic ("myCAF") and inflammatory ("iCAF") fibroblast subgroups, previously identified in FAP⁺ fibroblasts from pancreatic cancer (Elyada et al., Cancer Discov 2019;9(8):1102-23, Ohlund D, et al. J Exp Med 2017;214(3):579-96). CAF-S1 clusters 1, 2 and 5 were identified as iCAF and clusters 0, 3, 4, 6, and 7 as myCAF. Consistent with data from pancreas cancer, iCAF showed high expression of chemokines and pro-inflammatory molecules such as *CXCL12 (CXC motif chemokine ligand 12)* and *SOD2 (Superoxide dismutase 2),* while myCAF expressed myofibroblast markers, including *COL1A2 (Collagen type 1 alpha 2 chain)* and *TAGLN* (*Transgelin*)*.* In addition, the inventor observed that the iCAF cluster 5 expressed high levels of *CD74,* encoding *Major Histocompatibility Class (MHC) II invariant chain. CD74* was recently shown to be specifically expressed in the antigen-presenting CAF ("apCAF") in pancreatic cancer, suggesting that the CAF-S1 cluster 5 might be reminiscent of such apCAF. To summarize, the inventor identified 8 different CAF-S1 clusters in BC. Clusters 1, 2 and 5 belong to the iCAF subgroup, with cluster 5 that might correspond to the apCAF cluster, whereas clusters 0, 3, 4, 6 and 7 belong to the myCAF subgroup. Moreover, iCAF clusters are characterized by detoxification (cluster 1), response to stimuli (cluster 2), IFNy and cytokines (cluster 5); and myCAF clusters by ECM (cluster 0), TGFβ (cluster 3), wound-healing (cluster 4), IFNαβ (cluster 6) and acto-myosin (cluster 7). Accordingly, the inventor proposed the following nomenclature for these different FAP^{High} CAF-S1 clusters: cluster 0 = ecm-myCAF, cluster 1 = detox-iCAF, cluster 2 = IL-iCAF, cluster 3 = TGFβ-myCAF, cluster 4 = wound-myCAF, cluster 5 = IFNy-iCAF, cluster 6 = IFNαβ-myCAF and cluster 7 = acto-myCAF.

Finally, the inventor wondered whether these CAF-S1 clusters accumulate differentially in the different BC subtypes. Since the inventor performed analysis on patients prior any treatment, the fresh samples collected for scRNA-seq were mostly collected from Luminal (Lum) patients, the HER2 and TN BC patients being preferentially treated in neoadjuvant settings. Consequently, there was no HER2 patient and only 2 TN BC patients in the prospective cohort 1 **(Table 1).** Still, in this dataset, it could detect that TN BC patients exhibited higher proportions of iCAF clusters than LumA BC patients, which accumulated more myCAF clusters (in LumA: iCAF = 43.4%, myCAF= 56.6%; in TN: iCAF = 57.1%, myCAF= 42.9%; P value = 1.29e-64 from Fisher's exact test). Due to the low number of TN BC in the dataset, this question was addressed by taking advantage of the TCGA database, which contains RNA-Seq data from a high number of LumA and TN BC patients. To this end, specific signatures of the 5 most abundant CAF-S1 clusters (that represented up to 91% of sequenced CAF-S1 cells) were defined by identifying differentially expressed genes in each cluster compared to the others **(****Fig. 7B****).** As these signatures were also used for detecting these clusters in melanoma, NSCLC and HNSCC data (see below **Fig. 3** and **Fig. 6****),** the inventors next discarded any gene of these signatures that was also expressed by melanoma, NSCLC and HNSCC cancer cells to avoid any signal from cancer cells and to guarantee a signal strictly specific of CAF-S1 clusters (Fig. 7B for specificity of CAF-S1 cluster signatures). The differential of expression of CAF-S1 cluster-specific signatures between LumA and TN BC subtypes from the TCGA RNA-seq database (https://portal.gdc.cancer.gov/) was assessed. This confirmed the accumulation of iCAF clusters in TN and myCAF clusters in LumA BC. Specifically, detox-iCAF and IL-iCAF showed higher expression in TN compared to Lum BC, while ecm-myCAF, TGFβ-myCAF and wound-myCAF expression was higher in LumA BC, this increase in iCAF content in TN BC being consistent with the reported presence of numerous TILs in some TN BC. In summary, using scRNA-seq from a large number of FAP⁺ CAF-S1 fibroblasts isolated from BC, 8 clusters were detected that exhibit distinct signatures and accumulated differentially in BC subtypes.

### CAF-S1 cellular clusters are validated by multicolor flow cytometry in BC

The inventors next aimed at validating CAF-S1 clusters by using multi-color flow cytometry (FACS) on fresh BC samples. By analyzing the percentage of each cluster among CAF-S1 defined by scRNA-seq, it was first observed that the 5 first clusters represented up to 91% of total sequenced cells. The inventors thus decided to focus the FACS analysis on these 5 most abundant clusters and sought to identify surface markers for each cluster. Using pairwise comparisons of CAF-S1 cluster expression profiles, 6 surface markers were identified with commercially available antibodies and designed a gating strategy to identify the 5 most abundant clusters (Fig. 8). The inventors sought to validate the specificity of these 6 markers in an independent CAF-S1 dataset. To do so, the inventors studied the CAF-S1 scRNA-seq data corresponding to the 8^{th} patient, in which the clusters labels were transferred successfully by the Label Transfer algorithm. Indeed, the gating strategy relying on these 6 markers and based on 7 BC patients efficiently delineated the 5 most abundant CAF-S1 clusters in the independent dataset. By this way, it was confirmed that these markers were specific of each CAF-S1 cluster. Thus, fresh BC samples were analyzed by FACS by applying the following gating strategy: CAF-S1 fibroblasts (isolated as **CD45⁻, EPCAM⁻, CD31⁻, CD235a⁻, FAP^{High} CD29^{Med}**) were first separated based on ANTXR1 protein level that distinguished myCAF (ANTXR1⁺) from iCAF (ANTXR1⁻) fibroblasts in BC. ANTXR1⁺ (myCAF) CAF-S1 clusters 0 (ecm-myCAF), 3 (TGFβ-myCAF) and 4 (wound-myCAF) were next distinguished according to SDC1, LAMP5 and CD9 protein levels. ANTXR1⁺ SDC1⁺ LAMP5⁻were defined as cluster 0 (ecm-myCAF), ANTXR1⁺ LAMP5⁺ SDC1^{+/-} as cluster 3 (TGFβ-myCAF), and ANTXR1⁺ SDC1⁻ LAMP5⁻ CD9⁺ as cluster 4 (wound-myCAF). ANTXR1⁻ (iCAF) CAF-S1 clusters 1 (detox-iCAF) and 2 (IL-iCAF) were separated using GPC3 and DLK1 markers. ANTXR1⁻ GPC3⁺ DLK1^{+/-} were defined as cluster 1 (detox-iCAF); ANTXR1⁻ GPC3⁻ DLK1⁺ as cluster 2 (IL-iCAF). ANTXR1⁺ CAF-S1 cells that were negative for LAMP5, SDC1 and CD9 and ANTXR1⁻ GPC3⁻ DLK1⁻ cells were pooled and referred to as "other cluster". By applying this gating strategy on 44 fresh samples (prospective cohort 2, Table S1), the existence of these 5 most abundant clusters was validated in BC (Fig. 2). The percentage of each cluster among CAF-S1 cells defined by FACS confirmed the single-cell results, including clear heterogeneity among CAF-S1 fibroblasts and ecm-myCAF as the most abundant population in the majority of patients (Fig. 2). The inventors next analyzed if there was any correlation between the respective proportions of these 5 CAF-S1 clusters across patients. The relative abundances of ecm-myCAF and TGFβ-myCAF (both myCAF) were correlated together, as well as those of detox-iCAF and IL-iCAF (both iCAF). Conversely, the proportions of ecm-myCAF and TGFβ-myCAF were anti-correlated with the ones of detox-iCAF and IL-iCAF. In addition, the wound-myCAF was negatively correlated with detox-iCAF, IL-iCAF but also with ecm-myCAF, suggesting that these different CAF-S1 clusters could accumulate differentially in BC but in a coordinated manner.

### CAF-S1 cellular clusters are identified across cancer types

The inventors next sought to test the existence of CAF-S1 cellular clusters in other cancer types. To do so, publicly available scRNA-seq data from head and neck squamous cell carcinoma (HNSCC) (Puram SV, et al. Cell 2017;171(7):1611-24 e24) and non-small cell lung cancer (NSCLC) (Lambrechts D, et al. Nat Med 2018;24(8):1277-89) were analyzed, since these two studies had isolated enough CAF to investigate clusters. These published studies, included 18 HNSCC patients, of which 5 matched pairs of primary tumors and lymph nodes metastases, for a total of 5902 total cells analyzed (Puram SV, et al. Cell 2017;171(7):1611-24 e24). Moreover, in the NSCLC cohort, more than 52 000 total cells were collected from 5 different patients (Lambrechts D, et al. Nat Med 2018;24(8):1277-89). In these two studies, 1422 cells and 1465 cells were annotated as CAF in HNSCC and NSCLC cohorts, respectively. To pursue the analysis strictly on CAF-S1 fibroblasts, CAF-S1 were distinguished from CAF-S4 based on the expression of FAP and MCAM, two markers regulated at RNA levels in CAF-S1 (FAP^{High} MCAM^{Low}) and CAF-S4 (FAP^{Low} MCAM^{High}) respectively. As a result, 603 CAF-S1 cells in HNSCC and 959 in NSCLC were further analyzed. The similarity between CAF-S1 cells from distinct cancer types was compared by mixing the referent (BC) and target (HNSCC or NSCLC) datasets. Data integration was done using "anchor" correspondences across single-cells from different datasets on the basis of the similarity of their expression profiles, as described in Stuart T, et al. Cell 2019;177(7):1888-902 e21 (Fig. 3). The inventors used CAF-S1 cluster-specific signatures defined by differentially expressed genes in each cluster compared to the others (Fig. 7B). Remarkably, it was found systematic correspondences between CAF-S1 clusters from BC with CAF-S1 clusters from either HNSCC (Fig. 3A) or NSCLC (Fig. 3B). Visualization of the clusters using specific signatures confirmed that the 5 most abundant clusters in HNSCC and NSCLC could be detected (Fig. 3A, B). Hence, the inventors confirm the existence of the 5 most abundant CAF-S1 clusters in HNSCC and NSCLC, highlighting their relevance in other cancers.

### Immunosuppressive environment correlates with specific CAF-S1 clusters

As the inventors showed that CAF-S1 fibroblasts exert immunosuppression in breast and ovarian cancer, they next investigated whether this function could be exerted by all CAF-S1 clusters or restricted to specific ones (Fig. 4). The inventors first tested if they could detect correlations between the content of CAF-S1 clusters and immune cells. To this end, fresh BC samples (prospective cohort 2, Table 1) were characterized both in terms of CAF-S1 cluster content and immune cell infiltration, including CD4⁺, CD8⁺ T lymphocytes and natural killer (NK) cells. The inventors tested associations between stromal and immune cells and analyzed variables exhibiting at least one significant correlation with another variable. The correlation matrix obtained by unsupervised hierarchical clustering highlighted that ecm-myCAF and TGFβ-myCAF are clustered together on the one side, detox-iCAF and IL-iCAF clusters on the other, while the wound-myCAF cluster was quite isolated suggesting that these different clusters interact differentially with T cells. Interestingly, it was found that ecm-myCAF, TGFβ-myCAF and wound-myCAF showed specific associations with T lymphocytes. Indeed, it was first observed that the proportion of ecm-myCAF among CAF-S1 fibroblasts was significantly correlated with CD45⁺ hematopoietic cells infiltration (Fig. 9). More specifically, the abundance of ecm-myCAF was correlated with infiltration of PD-1⁺, CTLA-4⁺ and TIGIT⁺ CD4⁺ T lymphocytes, but anti-correlated with CD8⁺ T lymphocytes. Likewise, although the content in TGFβ-myCAF did not show any global association with CD45⁺ hematopoietic cells, its abundance was positively correlated with infiltration by CTLA-4⁺ CD4⁺ T lymphocytes and negatively correlated with CD8⁺ T lymphocytes. Thus, these data indicate that the abundance of ecm-myCAF and TGFβ-myCAF is associated with an immunosuppressive environment enriched in Tregs. In contrast to ecm-myCAF and TGFβ-myCAF clusters, the abundance of detox-iCAF and IL-iCAF was correlated with CD8⁺ T cell infiltration. The wound-myCAF cluster was globally correlated with T lymphocytes among CD45⁺ cells (Fig. 9) and anti-correlated with CTLA-4⁺, TIGIT⁺, PD-1⁺ and NKG2A⁺ CD4⁺ T lymphocytes. The enrichment in wound-myCAF was also anti-correlated with CTLA-4⁺ CD8⁺, TIGIT CD8⁺, CD244⁺ CD8⁺, CD244⁺ NK, markers of exhaustion, suggesting a global association of this cluster with high T lymphocyte infiltration and immuno-protective environment. Importantly, the CAF-S1 signature published in Costa A, et al. Cancer Cell 2018;33(3):463-79e10, in Givel et al. Nat Commun 2018;9(1):1056 and in Kieffer et al. Cancer Discov. 2020;10(9):1330-1351 does not allow to specifically identify the CAF-S1 immunosuppressive clusters linked to immunotherapy response (i.e ECM-myCAF, TGFβ-myCAF and wound-myCAF). These clusters are ANTXR1+. In contrast, we show that the most differentially CAF-S1 genes are not specifically expressed in these clusters (Fig. 11), suggesting that identifying ANTXR1 as a marker of these clusters among the Top CAF-S1 genes was not possible by chance.

To validate these data in an independent and large cohort of BC patients, the association between CAF-S1 clusters and T cell signatures in the publicly-available TCGA database was next tested. RNA-seq data from TCGA database enabled to confirm that the expression of ecm-myCAF and TGFβ-myCAF clusters was positively correlated with the one of FOXP3 (Fig. 4B), one of the main Treg markers. In addition, while wound-myCAF showed no real association with FOXP3, detox-iCAF and IL-iCAF clusters were negatively correlated with FOXP3 (Fig. 4B). In agreement with these data, a positive correlation between T cell cytolytic index was observed, and detox-iCAF and IL-iCAF clusters, but not with ecm-myCAF, TGFβ-myCAF or wound-myCAF (Fig. 4C). To summarize, while detox-iCAF and IL-iCAF are correlated with immunocompetent environment, both ecm-myCAF and TGFβ-myCAF are associated with an immunosuppressive environment, poor in CD8⁺ T lymphocytes and enriched in CD4⁺ T lymphocytes expressing high levels of immune checkpoints, including PD-1, CTLA-4.

### Positive feedback loop between ecm-myCAF and TGFβ-myCAF with PD-1⁺ and CTLA-4⁺ Tregs

As described above, the inventors observed that the abundance of ecm-myCAF and TGFβ-myCAF, but not detox-iCAF and IL-iCAF, is correlated with that of PD-1⁺ and/or CTLA-4⁺ CD4⁺ T lymphocytes in BC. The inventors investigated the role of CAF-S1 clusters in generating a CD4⁺ CD25⁺-enriched immunosuppressive environment. They therefore established primary cultures of CAF-S1 clusters in order to perform *in vitro* functional assays. Although they did not achieve to establish every CAF-S1 clusters in culture, they succeeded at isolating ecm-myCAF and iCAF clusters by applying two distinct methods, i.e. (1) by leaving CAF-S1 fibroblasts directly escaping and spreading from BC samples seeded in plastic dishes, and (2) by sorting FAP^{High} CD29^{Med} cells by FACS and expanding them in culture in plastic dishes. After expansion during few weeks, the identity of these different cells in the same culture conditions, i. e. those compatible with co-culture with CD4⁺ CD25⁺ T lymphocytes for performing functional assays (see below), were compared. It was observed that CAF-S1 obtained by spreading expressed high levels of myCAF genes, while CAF-S1 isolated by sorting exhibited high expression of iCAF genes. The inventors thus applied the cluster-specific signatures established from scRNA-seq data (Fig. 7B) and found that spread CAF-S1 fibroblasts were enriched in ecm-myCAF, while sorted CAF-S1 were enriched in detox-iCAF, IL-iCAF and IFN-iCAF clusters (see Methods' section *#RNA sequencing of CAF-S1 primary cell lines isolated from BC*)*.* The inventors have previously demonstrated that the global CAF-S1 subpopulation has no direct effect on CD4⁺ CD25⁻ T cells but increases the proportion of FOXP3⁺ Tregs among CD4⁺ CD25⁺ T lymphocytes. As the content in ecm-myCAF and TGFβ-myCAF in BC was associated with a CD4⁺-enriched immunosuppressive micro-environment while iCAF clusters were not, the inventors compared the function of these myCAF and iCAF clusters on CD4⁺ CD25⁺ T cells using functional assays, as previously performed in Costa A, et al. Cancer Cell 2018;33(3):463-79 e10 and Givel et al. Nat Commun 2018;9(1):1056 doi 10.1038/s41467-018-03348-z.)

At first, the impact of myCAF and iCAF clusters on the content of CD4⁺ CD25⁺ FOXP3⁺ T lymphocytes was tested *in vitro* **(****Fig. 5A****).** ecm-myCAF increased the percentage of FOXP3⁺ T cells among the CD4⁺ CD25⁺ population and enhanced the FOXP3 protein level in these T cells **(****Fig. 5A****).** In contrast, iCAF clusters had no impact on either the percentage of CD4⁺ CD25⁺ FOXP3⁺ T lymphocytes or on FOXP3 protein levels **(****Fig. 5A****).** The inventors also tested the impact of culture on the identity and immunosuppressive activity of normal fibroblasts. Fibroblasts isolated upon spreading from healthy tissues were FAP^{Neg-Low} and devoid of immunosuppressive activity at early time point, but became FAP^{Pos-High} and immunosuppressive at later passages, suggesting that the maintenance at long term of CAF on plastic dishes may activate them. Based on the ability of ecm-myCAF to increase CD4⁺ CD25⁺ FOXP3⁺ T lymphocytes, the inventors next compared the capacity of CAF-S1 clusters to modulate the proportion of PD-1⁺, CTLA-4⁺, TIGIT⁺, TIM3⁺ and LAG3⁺ on CD4⁺ CD25⁺ FOXP3⁺ T lymphocytes, considering both percentage of positive cells and surface protein levels of these immune checkpoints **(****Fig. 5B-F****).** ecm-myCAF significantly increased both percentage of PD-1⁺ and CTLA-4⁺ CD4⁺ CD25⁺ FOXP3⁺ T lymphocytes and immune checkpoint levels at their surface **(****Fig. 5B, C****,).** In contrast to ecm-myCAF, iCAF clusters neither affected the percentage of PD-1⁺ and CTLA-4⁺ T cells nor CTLA-4 protein levels **(****Fig. 5B, C****).** Furthermore, although iCAF clusters increased PD-1 protein levels, this effect was at lower efficiency than ecm-myCAF. Both myCAF and iCAF clusters increased the proportion of CD4⁺ CD25⁺ FOXP3⁺ TIGIT⁺ cells **(****Fig. 5D****),** while they had no effect on TIM3⁺ and LAG3⁺ T cells **(****Fig. 5E,F****, ).** Hence, in agreement with the correlations observed in BC between ecm-myCAF and PD-1⁺ and CTLA-4⁺ CD4⁺ T lymphocytes, it is shown here that CAF-S1 from ecm-myCAF have a direct function on Tregs by enhancing PD-1 and CTLA-4 immune checkpoint levels at the surface of CD4⁺ CD25⁺ FOXP3⁺ T lymphocytes, while iCAF clusters have no or minimal effect on these cells. Finally, it was observed that the up-regulation of immune checkpoints at the surface of CD4⁺ CD25⁺ T cells upon co-culture with ecm-myCAF was also detected intracellularly **(****Fig. 10A****),** suggesting that ecm-myCAF increased the total protein levels in T cells. Moreover, the inventors found that expression of FOXP3, CTLA-4, and TIGIT in CD4⁺ CD25⁺ T cells were also up-regulated at mRNA levels following co-culture with ecm-myCAF **(****Fig. 10B****),** PD-1 RNA being almost not detected in T cells *in vitro.* Furthermore, the inventors observed that mRNA levels of NFAT and STAT family members were also elevated in CD4⁺ CD25⁺ T lymphocytes upon co-culture with ecm-myCAF **(****Fig. 10C****).** As NFAT and STAT are well-known transcriptional regulators of immune checkpoints in T cells, these data indicate that ecm-myCAF promotes up-regulation of immune checkpoints at RNA levels in CD4⁺ CD25⁺ T lymphocytes, potentially through the activation of NFAT/STAT-signaling pathways.

Considering the impact of CAF-S1 clusters on Tregs, the inventors next wondered whether T lymphocytes could in turn modulate CAF-S1 cluster identity. The inventors thus evaluated if co-culturing CD4⁺ CD25⁺ T lymphocytes had any impact on the marker cluster levels at the surface of CAF-S1 fibroblasts. Upon co-culture, to avoid any contamination by T cells, CAFs-S1 were isolated by FACS and analyzed cluster markers expressed at their surface. It was observed that the co-culture of CD4⁺ CD25⁺ T cells significantly increased the expression of the TGFβ-myCAF specific marker LAMP5, at the surface of ecm-myCAF fibroblasts **(****Fig. 5G****),** thereby suggesting that the content in TGFβ-myCAF increases upon co-culture with CD4⁺ CD25⁺ T lymphocytes. This effect was only detected in myCAF and not in iCAF fibroblasts **(****Fig. 5G****),** as expected considering that TGFβ-myCAF and ecm-myCAF CAF-S1 fibroblasts belong to the myCAF subgroup. Consistent with this observation, ANTXR1 protein level also showed a trend to increase (although without reaching significance) in ecm-myCAF fibroblasts upon co-culture with CD4⁺ CD25⁺ T cells, while it remained strictly unchanged and low in iCAF cells **(****Fig. 5G****).** Quite surprisingly, DLK1, marker of IL-iCAF, also increased upon co-culture, suggesting a potential plasticity between ecm-myCAF and IL-iCAF. In contrast, the other markers did not show any significant variation upon co-culture and remained either high (SDC1) or low (GPC3 and CD9), as expected based on respective cluster identity **(****Fig. 5G****).** These observations suggest that CD4⁺ CD25⁺ T lymphocytes might promote conversion of ecm-myCAF (ANTXR1⁺ SDC1⁺ LAMP5⁻ CD9^{+/-}) into TGFβ-myCAF (ANTXR1⁺ LAMP5⁺ SDC1^{+/-} CD9^{+/-}), both clusters being myCAF. Taken as a whole, the inventors found that ecm-myCAF can directly impact on PD-1 and CTLA-4 protein levels at the surface of FOXP3⁺ T lymphocytes. Reciprocally, Tregs can promote the conversion of ecm-myCAF into TGFβ-myCAF, thereby underlying a positive feedback loop between these two clusters and CD4⁺ CD25⁺ PD-1⁺ or CTLA-4⁺ T cells that could account for the positive correlations observed in BC.

### ecm-myCAF and TGFβ-myCAF are associated with primary resistance to immunotherapy

Given the direct effect of specific CAF-S1 clusters on PD-1 and CTLA-4 protein levels on Tregs, the inventors next wondered whether some CAF-S1 clusters could be associated with immunotherapy resistance. As they did not have access to data from BC treated by immunotherapy, they took advantage of publicly available data from metastatic melanoma patients treated with anti-PD-1 (Pembrolizumab) therapy (Hugo W et al. Cell 2016; 165(1):35-44), which has revolutionized melanoma treatment. As defined in the aforementioned study, the inventors considered patients as "non-responders" to anti-PD-1 if they showed progressive disease, and as "responders" in case of complete or partial response. By performing gene set enrichment analysis, it first observed that, at time of diagnosis, expression of CAF-S1-specific genes, but not of normal fibroblast content, was significantly enriched in tumors from non-responder patients. Using CAF-S1 cluster-specific signatures, the inventors observed that ecm-myCAF, TGFβ-myCAF and wound-myCAF gene expression was enriched in non-responders compared to responders, while detox-iCAF, IL-iCAF and IFN-iCAF clusters were not (Fig. 6A). They next compared the content in each CAF-S1 cluster in responders *versus* non-responders. They confirmed that ecm-myCAF, TGFβ-myCAF and wound-myCAF expression was significantly higher in non-responders than in responders, while detox-iCAF and IFN-iCAF expression was similar between the two subgroups of patients (Fig. 6B). Moreover, neither the normal fibroblast content, nor the cytolytic index, defined in Rooney et al. Cell 2015; 160(1-2):48-61 was different between responders and non-responders (Fig. 6C, D). In agreement with these observations, the reciprocal analysis (i.e. determining the number of responders and non-responders according to low- or high-CAF-S1 cluster expression) confirmed that the number of non-responder patients was significantly associated with tumors showing high expression of ecm-myCAF, TGFβ-myCAF or wound-myCAF at diagnostic, while the other CAF-S1 clusters, the general CAF content or the cytolytic index were not informative on patient response to immunotherapy (Fig. 6E-G). Together, these data show that 3 specific CAF-S1 clusters (ecm-myCAF, TGFβ-myCAF and wound-myCAF) are indicative at diagnosis of anti-PD-1 response in metastatic melanoma patients, while the other CAF-S1 clusters (detox-iCAF, IL-iCAF and IFN-iCAF), the total CAF content or the cytolytic index are not. Finally, the inventors sought to validate the impact of CAF-S1 clusters, in particular ecm-myCAF, TGFβ-myCAF and wound-myCAF, on primary immunotherapy resistance in a series of metastatic NSCLC patients, another recently established clinical indication for immunotherapy (here treated in second- or third-line setting with Nivolumab, see Table 2 for detailed description of the NSCLC cohort 4). Similar to melanoma, the inventors validated that CAF-S1 signature, evaluated in tumor specimens sampled at diagnosis, was significantly enriched in non-responder patients. In contrast, the normal fibroblast content was higher in responders than in non-responders, suggesting that CAF-S1 was significantly enriched in non-responders. Importantly, the inventors confirmed that ecm-myCAF, TGFβ-myCAF and wound-myCAF were associated with non-responder NSCLC patients, as opposed to detox-iCAF, IL-iCAF and IFNγ-iCAF clusters (Fig. 6I). In conclusion, in contrast to detox-iCAF, IL-iCAF and IFNy-iCAF clusters, the abundance of ecm-myCAF and TGFβ-myCAF at diagnosis is associated with resistance to immunotherapy in both melanoma and NSCLC, consistent with their capacity to increase PD-1⁺ and CTLA-4⁺ protein levels in Tregs.

### MATERIAL AND METHODS

### Cohorts of patients

*BC patients:* The study developed here is based on samples taken from surgical residues, available after histopathological analyses, and not required for diagnosis. There is no interference with clinical practice. Analysis of primary tumor samples was performed according to the relevant national law on the protection of people taking part in biomedical research. All patients hospitalized at Institut Curie (BC patients) received a welcome booklet explaining that their samples may be used for research purposes. All patients included in the study were thus informed by their referring oncologist that biological samples collected through standard clinical practice could be used for research purposes and that they could express opposition for such use, if they wished so. In case of patient refusal, that could be either orally expressed or written, residual tumor samples were not included in the study. Human experimental procedures for analyses of tumor micro-environment by F. Mechta-Grigoriou's lab were approved by the Institutional Review Board and Ethics committee of the Institut Curie Hospital group (approval February 12^{th}, 2014) and CNIL (Commission Nationale de I'informatique et des Libertés) (Nº approval: 1674356 delivered March 30^{th}, 2013). The 'Biological Resource Centre' (BRC) is part of to the Pathology Department, in the Diagnostic and Theragnostic Medicine Department headed by Dr. A. Vincent-Salomon. BRC is authorized to store and manage human biological samples according to French legislation. The BRC has declared defined sample collections that are continuously incremented as and when patient consent forms are obtained (declaration number # DC-2008-57). The BRC follows all currently required national and international ethical rules, including the declaration of Helsinki. The BRC has also been accredited with the AFNOR NFS-96-900 quality label (renewed and currently valid until 2021). Luminal (Lum) tumors were defined by positive immunostaining for ER (Estrogen receptor) and/or PR (Progesterone receptor). The cut-off used to define hormone receptor positivity was 10% of stained cells. Ki67 (proliferation) score further distinguishes Lum A from Lum B tumors (below 15%: Lum A, above: Lum B). HER2-amplified carcinomas have been defined according to ERBB2 immunostaining using ASCO's guideline. TN immunophenotype was defined as follows: ER⁻PR⁻ ERBB2⁻ with the expression of at least one of the following markers: KRT5/6⁺ or EGF-R⁺.

*NSCLC patients:* NSCLC samples were from the routine diagnostic samples stored in the Pathology Department of Bichat Hospital, from patients treated by immuno-oncology drugs in the Thoracic Oncology Department of Bichat hospital headed by Pr. G. Zalcman, MD. The de-identified clinical data were part of the thoracic oncology database of lung cancer patients from the Clinical Investigation Centre (co-headed by Pr. G. Zalcman) CIC-1425/CLIP₂ of Bichat Hospital (Regional Health Agency authorization #17-1381), in accordance with French regulatory rules for observational clinical research. Patients received checkpoint inhibitors, after progression upon chemotherapy-based 1^{st} or 2^{nd} line, according to the registration of immuno-oncology drugs. During the current study period, the anti-PD-1 Nivolumab monoclonal antibody represented the most frequent drug used in such setting. Efficacy of immuno-oncology treatment was assessed every 8 to 12 weeks by whole body CT-scan, by a weekly multidisciplinary tumor board, including thoracic specialized radiologists, thoracic oncologists and pulmonologists, according to RECIST v.1.1 criteria, defining objective responders (OR), patients with stable disease (SD) and patients showing tumor progression (Progr) with 20% of more increase in their tumor volume without any clinical benefit. The best response status observed at 4 months was used in the current study. SD patients, for whom immuno-oncology drug was given more than 6 months because of a clinical benefit at 4 months evaluation (n= 3), without any criteria for progressive disease (then long-lasting SD), were included in the group of responder patients in the current study. Date of the CT-scan assessed progression was recorded. Some patients showed an early clinical progression that required an early (before eight weeks) CT-scan assessment. In these series, according to the above-criteria. There were 22 responder patients, 48 progressive patients, who received less than 4 months treatment. Date of progression was retained as the date of CT-scan showing RECIST progression. Date and cause of death, or date of last news with vital status was systematically recorded. Post-progression 2^{nd} or 3^{rd}-line treatments were registered. There was no unbalance according to post-progression treatments. PD-L1 staining was performed and interpreted by A.G., on 4µm paraffin-embedded sections, from diagnosis, pre-treatment biopsy samples, containing at least 200 tumor cells, using Cell Signaling Technology E1L3N commercially available clone, on the Leica Bond platform. All but 5 patients (having less of 200 tumor cells in the remaining pathological block) had PD-L1 immunohistochemistry analysis.

### CAF-S1 RNA sequencing at single cell level

*Isolation of CAF-S1 from BC:* CAF-S1 fibroblasts were isolated from a total of 8 primary BC (surgical residues prior any treatment) (see Table S1 for details on the prospective cohort). 7 BC were initially studied. In addition, another BC sample was added for validating the CAF-S1 clusters by using the Label Transfer algorithm from Seurat R package. CAF-S1 fibroblasts were isolated from BC by using BDFACS ARIA III^{™} sorter (BD biosciences). Fresh human BC primary tumors were collected directly from the operating room, after surgical specimen's macroscopic examination and selection of areas of interest by a pathologist. Samples were cut into small pieces (around 1 mm³) and digested in CO₂-independent medium (Gibco #18045-054) supplemented with 150 µg/ml liberase (Roche #05401020001) and DNase I (Roche #11284932001) during 40 minutes (min) at 37°C with shaking (180 rpm). Cells were then filtrated through a 40 µm cell strainer (Fisher Scientific #223635447) and resuspended in PBS+ solution (PBS, Gibco #14190; EDTA 2 mM, Gibco #15575; Human Serum 1 %, BioWest #S4190-100) at a final concentration between 5 x 10⁵ and 10⁶ cells in 50 µl. To isolate CAF-S1 fibroblasts, the inventors first apply a selection to exclude epithelial (EPCAM⁺), hematopoietic (CD45⁺), endothelial (CD31⁺), and CD235a⁺ (red blood) cells and next use CAF-S1 markers (FAP and CD29). To do so, cells in suspension were then stained with an antibody mix containing anti-EpCAM-BV605 (BioLegend, #324224), anti-CD31-PECy7 (BioLegend, #303118), anti-CD45-APC-Cy7 (BD Biosciences, #BD-557833), anti-CD235a-PerCP/Cy5.5 (Biolegend, #349109), anti-CD29-Alexa Fluor 700 (BioLegend, #303020), anti-FAP-APC (primary antibody, R&D Systems, #MAB3715) for flow cytometry cell sorting in order to perform single cell RNA sequencing. All antibodies except FAP were purchased already conjugated with fluorescent dyes. Anti-FAP antibody was conjugated with fluorescent dye Zenon APC Mouse IgG1 labeling kit (ThermoFisher Scientific, #Z25051). Isotype control antibodies for each CAF marker used were: iso-anti-CD29 (BioLegend, #400144) and iso-anti-FAP (primary antibody, R&D Systems, #MAB002).

Cell suspensions were stained immediately after dissociation of BC tumor samples during 15 min at RT with the antibody mix in PBS+ solution. 2.5 µg/ml DAPI (ThermoFisher scientific, #D1306) was added just before flow cytometry sorting. Signals were acquired on the BDFACS ARIA III^{™}sorter (BD biosciences) for cell sorting. At least 5 x 10⁵ events were recorded. Compensations were performed using single staining on anti-mouse IgG and negative control beads (BD biosciences, #552843) for each antibody. Data analysis was performed using FlowJo version X 10.0.7r2. Cells were first gated based on forward (FSC-A) and side (SSC-A) scatters (measuring cell size and granularity, respectively) to exclude debris. Dead cells were excluded based on their positive staining for DAPI. Single cells were next selected based on SSC-A versus SSC-W parameters. Gating included EPCAM⁻, CD45⁻, CD31⁻, CD235a⁻ cells, to remove epithelial (EPCAM⁺), hematopoietic (CD45⁺), endothelial (CD31⁺) and red blood cells (CD235a⁺).

*Single cell CAF-S1 RNA sequencing:* Upon isolation, CAF-S1 cells were directly collected into RNase-free tubes (ThermoFisher Scientific, #AM12450) pre-coated with DMEM (GE Life Sciences, #SH30243.01) supplemented with 10% FBS (Biosera, #1003/500). At least 6 000 cells were collected per sample. In these conditions, cell concentration was checked in control samples and was of 200 000 cells/ml. Single cell capture, lysis, and cDNA library construction were performed using Chromium^{™} system from 10X Genomics, with the following kits: Chromium^{™} Single Cell 3' Library & Gel Bead Kit v2 kit (10X Genomics, #120237) and Chromium^{™} Single Cell A Chip Kits (10X Genomics, #1000009). Generation of Gel beads in Emulsion (GEM), barcoding, post GEM-RT (Reverse Transcription) cleanup and cDNA Amplification were performed according to manufacturer's instructions. Targeted cell recovery was 3 000 cells per sample to retrieve enough cells, while preserving a low multiplet rate. Cells were loaded accordingly on the Chromium Single cell A chips and 12 cycles were performed for cDNA amplification. cDNA quality and quantity were checked on Agilent 2100 Bioanalyzer using Agilent High Sensitivity DNA kit (Agilent, #5067-4626) and library construction followed according to 10X Genomics protocol. Libraries were next run on the Illumina HiSeq (for patients P5-7) and NovaSeq (for patients P1-4) with a depth of sequencing of 50 000 reads per cell. Processing of raw data, including demultiplexing of raw base call (BCL) files into FASTQ files, alignment, filtering, barcode and Unique Molecular Identifiers (UMI) counting, were performed using 10X Cell Ranger pipeline version 2.1.1. Reads were aligned to Homo sapiens (human) genome assembly GRCh38 (hg38).

### scRNA-seq data processing

*scRNAseq:* Pre-processing of raw data was initially performed using Cell Ranger software pipeline (version 2.1.1). This step included demultiplexing of raw base call (BCL) files into FASTQ files, reads alignment on human genome assembly GRCh38 using STAR and counting of unique molecular identifier (UMI). A first set of 18 805 CAF-S1 cells from 7 BC patients (corresponding to 7 sequencing runs, patients 1 to 7) were analyzed using Seurat R package (version 3.0.0) (Butler et al. Nat Biotechnol 2018;36(5):411-2). A second set of 1646 CAF-S1 cells from one BC patient (patient 8) was used for validation (see *#Label Transfer*) and analyzed using the same methodology.

*Quality control:* As a quality control step, it was first filtered out of low-quality cells, empty droplets and multiplet captures based on the distribution of the unique genes detected (non-zero count) in each cell for each patient. Cells with less than 200 genes detected and more than 6000 genes (for patient 1), more than 5000 genes detected (for patients 3, 5 and 6), more than 4500 genes detected (for patients 2, 7 and 8) or more than 4000 genes detected (for patient 4) were excluded. Distribution of cells based on the fraction of expressed mitochondrial genes was also plotted. Cells with fraction of mitochondrial genes higher than 5% were discarded in order to eliminate dying cells or low-quality cells with extensive mitochondrial contamination. For each patient, the mitochondrial fraction was computed using *PercentageFeatureSet* function from Seurat with argument pattern = "^MT-". Following these QC criteria, 18 296 CAF-S1 cells (patient1= 1825 cells; patient2= 3300 cells; patient3= 2810 cells; patient4= 3153 cells; patient5= 2486 cells; patient6= 3179 cells and patient7= 1543 cells) and 1582 CAF-S1 cells (patient8) were finally conserved in the first and second datasets respectively for downstream analyses.

*Normalization and data integration:* Integration of the 7 BC scRNA-Seq from the first dataset was done using Seurat functions *FindIntegrationAnchors* and *IntegrateData* after library-size normalization of each cell using *NormalizeData* function with default parameters. 30 dimensions were used for Canonical correlation analysis (CCA), 30 principal components (PCs) were used in the weighting procedure of *IntegrateData* function. Data were scaled using *ScaleData* function and variables 'nUMI' and 'percent.mt' were used for regression. Same parameters were used for the normalization of the second dataset. *Clustering and data visualization:* PCA dimensionality reduction was run using default parameters. Number of included components (PCs) was assessed using JackStraw procedure implemented in *JackStraw* and *ScoreJackStraw* functions. 30 PCs were conserved. Graph-based clustering approach was used to cluster the cells from the first dataset using *FindNeighbours (k = 20)* and *FindClusters* functions (res = 0.35). 10 CAF-S1 clusters were obtained at this resolution. For visualization of the data, non-linear dimensional reduction technique UMAP was applied using the *RunUMAP* function from Seurat. *Analysis of differential gene expression and signaling pathways:* Genes specifically up-regulated in each of the 10 clusters of the first dataset were identified using pairwise differential analysis. Although the median number of differentially expressed genes in each pairwise combination was 126 genes, 2 combinations gave a very limited number of differentially expressed genes, with only 9 genes between clusters 0 and 5 and 22 genes between clusters 3 and 6. Biological meaning of each cluster was also determined using Metascape tool (http://metascape.org) using all genes significantly up-regulated in each of the 10 initial clusters (one cluster *versus* all other clusters; function *FindAllMarkers* with following parameters: logfc.threshold = 0.25, test = wilcox for Wilcoxon Rank Sum test). Consistent with pairwise analysis, biological pathways identified for clusters 0/5 on the one hand, and 3/6 on the other hand, were redundant and thus combined. Clusters 0 and 5 were then defined as cluster 0/ecm-myCAF and clusters 3 and 6 as cluster 3/TGFβ-myCAF to finally identify 8 biologically distinct CAF-S1 clusters.

*Gene signatures of CAF-S1, CAF-S1 clusters and normal fibroblasts :* Specific gene signatures of CAF-S1 clusters 0 to 5 were defined by performing a differential analysis (Wilcoxon rank-sum test) between clusters 0 to 5. Differentially expressed genes between clusters (one cluster *versus* all other clusters) with an adjusted P value < 0.05 were selected. As these signatures were used for detecting CAF-S1 clusters in RNA-seq data from single cells and bulk of different cancer types including melanoma, NSCLC and HNSCC, the inventors excluded genes expressed in tumor cells by using scRNA-seq data from tumor cells of melanoma (27), NSCLC (31) and HNSCC (30). The inventors defined genes expressed in tumor cells (and thus excluded from CAF-S1 cluster signatures) if more than 10% of tumor cells show an expression level higher than 1 in any of the aforementioned scRNA-seq data. CAF-S1 global signature was initially published in Costa A, et al. Cancer Cell 2018;33(3):463-79 e10 and submitted to the same type of analysis, excluding genes detected in tumor cells, and thus adapted for bulk analysis. The first 100 most significant genes were considered for the CAF-S1-specific signature. The normal fibroblast signature was defined by the genes significantly up-regulated in normal fibroblasts (FAP^{Neg} CD29^{Med} SMA^{Neg}) isolated from healthy juxta-tumor tissues, compared to CAF-S1 fibroblasts isolated from BC. Genes that are expressed in tumor cells were excluded from the signature, following the same strategy as this one described above. Cytolytic index was defined as the geometric mean of granzyme A (GZMA) and perforin (PRF1) gene expression, as described in Rooney et al. Cell 2015;160(1-2):48-61.

### Label Transfer

In order to validate the CAF-S1 clusters identified in the first dataset, another set of data corresponding to 1582 CAF-S1 fibroblasts after quality control and collected from an additive BC sample was analyzed using Seurat pipeline. The Label Transfer algorithm, described in Stuart T, et al. Cell 2019;177(7):1888-902 e21 and implemented in Seurat V3.0 R package, was applied using functions *FindTransferAnchors* and *TransferData.* The first dataset of 18 296 CAF-S1 cells was used as reference, while the second dataset of 1582 CAF-S1 cells was used as query. When finding anchors, dimensional reduction was performed by projecting the PCA from the reference onto the query. 30 dimensions were used.

### Single-cell data integration from BC, HNSCC and NSCLC

Integration between BC and HNSCC or BC and NSCLC single-cell data was done using method described in Stuart T, et al. Cell 2019;177(7):1888-902 e21 and implemented in Seurat V3.0 R package. In brief, the identification of cell pairwise correspondences between single cells across datasets (called "anchors") allows transforming datasets into a shared space. Dimensionality reduction of both datasets was performed using diagonalized Canonical Correspondence Analysis (CCA) and L2-normalization was applied to the canonical correlation vectors prior to the identification of anchors. Default parameters were used for *FindintegrationAnchors* and *IntegrateData* function in Seurat V3.0 package.

### Flow cytometry analysis of the 5 most abundant CAF-S1 clusters and immune cells

44 BC were cut into small fragments and digested in CO₂-independent medium (Gibco, #18045-054) supplemented with 5% fetal bovine serum (FBS, PAA, #A11-151), 2 mg/ml collagenase I (Sigma-Aldrich, #C0130), 2 mg/ml hyaluronidase (Sigma-Aldrich, #H3506) and 25 mg/ml DNase I (Roche, #11284932001) for 45 min at 37°C with shaking (180 rpm). After tissue digestion, cells were filtered using a cell strainer (40 mm, Fischer Scientific, #223635447) and washed using PBS solution (Gibco, #14190) supplemented with 2 mM EDTA (Gibco, #15575) and 1% human serum (BioWest, #S4190-100). Cells were then separated on 2 groups for analyzing CAF-S1 clusters panel and immune cells panel, respectively.

*CAF-S1 clusters panel:* Cells were stained with Live Dead NIR (1:1000, BD Bioscience #565388) for 20 min in PBS. Cells were then washed and stained with an antibody cocktail for 45 min containing anti-CD235a-APC-Cy7 (1:20, BioLegend, #349115) anti-EpCAM-BV605 (1:25, Biolegend, #324224), anti-CD31-PECy7 (1:50, BioLegend, #303118), anti-CD45-BUV395 (1:25, BD Biosciences, #BD-563792), anti-CD29-Alexa Fluor 700 (1:50, BioLegend, #303020), anti-FAP (1:100, R&D Systems, #MAB3715) coupled using fluorescent dye Zenon APC Mouse IgG1 labeling kit (Thermo Fisher Scientific, #Z-25051), anti-ANTXR1-AF405 (1:33, Novus Biological, #NB-100-56585), anti-LAMP5-PE (1:10, Miltenyi Biotech, #130-109-156), anti-SDC1-BUV737 (1:25, BD Biosciences, #BD-564393), anti GPC3-AF594 (1:20, RnD, #FAB2119T,100UG), anti-DLK1-AF488 (1:25, RnD, #FAB1144G-100) and anti-CD9-BV711 (1:200, BD Biosciences, #BD-743050). Isotype control antibodies for each CAF cluster marker used were: mouse IgG1 isotype control - BV711 (1:200, BD Bioscience, #563044), mouse IgG1 isotype control - AF405 (1:3, Novus Biological, #IC002V), mouse IgG1 isotype control - BUV737 (1:25, BD Bioscience, #564299), mouse IgG2B isotype control - AF488 (1:12,5, RnD, #IC0041G), mouse IgG2A isotype control - AF594 (1:5, RnD, #IC003T), REA Control-PE (1:10, Miltenyi Biotech, #130-113-462). Cells were then washed and acquired using LSR FORTESSA analyzer (BD biosciences) the same day or fixed in 4% paraformaldehyde (PFA, Electron Microscopy Sciences, #15710) for 20 minutes then washed and kept in PBS+ solution overnight and acquired the next day. At least 5 x 10⁵ events were recorded. Compensations were performed using single staining on anti-mouse IgG and negative control beads (BD bioscience #552843) for each antibody and on cells for Live/Dead staining. Data analysis was performed using FlowJo version 10.4.2 (LLC, USA). Cells were first gated based on forward (FSC-A) and side (SSC-A) scatters (measuring cell size and granulosity, respectively) to exclude debris. Dead cells and red blood cells were excluded based on their positive staining for Live/Dead NIR and CD235a respectively. Single cells were next selected based on SSC-H versus SSC-A parameters. Cells were then gated on EpCAM⁻, CD45⁻, CD31⁻ cells, for excluding epithelial cells (EpCAM⁺), hematopoietic cells (CD45⁺) and endothelial cells (CD31⁺). DAPI⁻, EPCAM⁻, CD45⁻, CD31⁻ cells were separated on 4 subsets (CAF-S1 to CAF-S4) according to FAP and CD29. CAF-S1 subset was first gated on ANTXR1. ANTXR1⁺ cells were next gated according to SDC1 and LAMP5. ANTXR1⁺ SDC1⁺ LAMP5⁻ were defined as cluster 0/ecm-myCAF and ANTXR1⁺ SDC1⁻ LAMP5⁺ as cluster 3/TGFβ-myCAF. ANTXR1⁺ SDC1⁻ LAMP5⁻ were gated on CD9, and ANTXR1⁺ SDC1⁻ LAMP5⁻ CD9⁺ were defined as cluster 4/ wound-myCAF. ANTXR1^{- / Low} cells were gated on DLK1 and GPC3. Cluster 1/detox-iCAF was defined as ANTXR1⁻ GPC3⁺ DLK1^{-/+} and cluster 2 as ANTXR1⁻ GPC3⁻ DLK1⁺. ANTXR1⁻ GPC3⁻ DLK1⁻ and ANTXR1⁺ SDC1⁻ LAMP5⁻ CD9⁻ were designated as other clusters.

*Immune panel:* Among the 44 BC samples analyzed for CAF-S1 clusters, 37 were characterized at the meantime for immune content. Cell types were analyzed on the live dead negative fraction and defined as hematopoietic cell (CD45⁺), CD4⁺ / CD8⁺ T lymphocytes; B lymphocytes (CD45⁺ CD14⁻CD3⁻ CD19⁺); NK (CD45⁺ CD14⁻ CD3⁻ CD56⁺), cytotoxic NK (CD56⁺ CD16⁺) and non-cytotoxic NK (CD56⁺ CD16⁻); (CD45⁺ CD14⁻ CD3⁺ CD4⁺ / CD8⁺) and myeloid cells (CD45⁺ CD14⁺). On each identified population, the percentage of positive cells for the following checkpoints was also evaluated: PD-1, CTLA-4, NKG2A, TIGIT, CD244, CD158K, CD69, CD161. Cells were stained with Live dead (1:1000, Thermo Fisher Scientific, #L34955) for 20 min in PBS. Cells were then washed and stained with an antibody cocktail for 45 min containing anti-CD45-APC-cy7 (1:20, BD Biosciences, #BD-557833); anti-CD14-BV510 (1:50, BD Bioscience, #563079), anti-CD56-BUV395 (1:25, BD Bioscience, #563554), anti-CD16-BV650 (1:25, BD Bioscience, #563692), anti-PD-1-BUV 737 (1:20, BD Bioscience, #565299) anti-CD3-AF700 (1:25, BD Bioscience, #557943), anti-NKG2A-BV786 (1:20, BD Bioscience, #747917), anti-TIGIT-BV605 (1:20, BD Bioscience, #747841), anti-CD158K-PE (1:10, Miltenyi Biotech, #130-095-205) anti-CD244-FITC (1:10, BD Bioscience, #550815), anti-CTLA-4-Pe-cy5 (1:10, BD Bioscience, #555854), anti-CD19-Percp-cy5.5 (1:20, BD Bioscience, #561295), anti-CD4 APC (1:25, Miltenyi Biotec, #130-092-374), anti-CD8-PE-TexasRred (1:100, Life Technologies, #MHCD0817), anti-CD69-BV710 (1:25, BD Bioscience, #563836), anti-CD161-PE-VIO770 (1:100, Miltenyi, #130-113-597). Cells were then washed and acquired using LSR FORTESSA analyzer (BD biosciences) the same day or fixed in 4% paraformaldehyde (PFA, Electron Microscopy Sciences, #15710) for 20 minutes then washed and kept in PBS+ solution overnight and acquired the next day. At least 5 x 10⁵ events were recorded. Compensations were performed using single staining on anti-mouse IgG and negative control beads (BD bioscience, #552843) for each antibody and on cells for Live/Dead staining. Data analysis was performed using FlowJo version 10.4.2 (LLC, USA).

### RNA sequencing of CAF-S1 primary cell lines isolated from BC

RNAs were extracted from CAF-S1 fibroblasts with Qiagen miRNeasy kit (Qiagen, #217004) according to the manufacturer's instructions. Among the 7 CAF-S1 primary cell lines studied here, 3 were isolated by sorting and 4 by spreading. These 7 CAF-S1 primary cell lines were generated from 7 different BC patients. RNA integrity and quality were analyzed using the Agilent RNA 6000 Pico kit (Agilent Technologies, #5067-1513). cDNA libraries were prepared using the TruSeq Stranded mRNA kit (Illumina, #20020594) followed by sequencing on NovaSeq (Illumina). Reads were mapped on the human reference genome (hg38; Gencode release 26) and quantified using STAR (version 2.5.3a) with parameters "outFilterMultimapNmax = 20; alignSJoverhangMin = 8; alignSJDBoverhangMin = 1; outFilterMismatchNmax = 999; outFilterMismatchNoverLmax = 0.04; alignIntronMin = 20; alignIntronMax = 1000000; alignMatesGapMax = 1000000; outMultimapperOrder = Random". Only genes with one read in at least 5% of all samples were kept for further analyses. Normalization were conducted with DESeq2 R package and raw read matrix was log2 transformed. For identifying the identity of CAF-S1 primary cell lines, a score was computed by the mean of expression of the genes that compose the iCAF/myCAF signatures (as defined in Ohlund et al. J Exp Med 2017;214(3):579-96) and for each CAF-S1 cluster signature. P values are from DESeq2 analysis.

### Functional assays

*Isolation of CD4⁺ CD25⁺ T lymphocytes:* CD4⁺ *CD25⁺* T lymphocytes were isolated from peripheral blood of healthy donors obtained from the "Etablissement Français du Sang", Paris, Saint-Antoine Crozatier blood bank through a convention with the Institut Curie (Paris, France). Briefly, peripheral blood mononuclear cells (PBMCs) were isolated using Lymphoprep (Stemcell, #07861), as previously described in Costa A, et al. Cancer Cell 2018;33(3):463-79 e10. CD4⁺ CD25+ were purified from 5 x 10⁸ PBMC by using magnetic cell separation (MACS) with the human CD4⁺ *CD25⁺* Tregs isolation kit (Miltenyi Biotec, #130-091-301), according to manufacturer's instructions. The purity of CD4⁺ *CD25⁺* T lymphocytes was determined by flow cytometry, as described in Costa A, et al. Cancer Cell 2018;33(3):463-79 e10.

*Isolation of CAF-S1 clusters in culture:* To isolate the different CAF-S1 clusters, the inventors first started by sorting the cells according to their specific markers but they failed to keep them alive with their different identities. They then tested two distinct methods of isolation by spreading and sorting. For the "spreading" method, tumors were cut into small pieces and incubated in plastic dishes (Falcon, #353003) in DMEM (HyClone, #SH30243.01) supplemented with 10% FBS (Biosera, #FB-1003/500), streptomycin (100 µg/ml) and penicillin (100 U/ml) (Gibco #15140-122) in a humidified in 1.5% O₂ and 5% CO₂ incubator, to let fibroblasts spread and expand during at least 2-3 weeks at 37ºC. For isolating fibroblasts by the "sorting" method, tumors were digested using enzymatic cocktails described in *(# Isolation of CAF-S1 from BC)* and sorted by BDFACS ARIA III^{™}using the gating strategy detailed in *(#CAF-S1 RNA sequencing at single cell)* in 48-well plastic dishes (TPP plates, #192048) pre-coated with FBS for 2h. CAF-S1 sorted cells were next expanded during 3-4 weeks at 37ºC in plastic dishes (TPP plates, #192048) in pericyte medium (ScienCell, #1201) supplemented with 2% FBS (ScienCell, #0010) in a humidified in 1.5% O₂ and 5% CO₂ incubator. For comparing cellular identity of sorted and spread CAF-S1 fibroblasts in the exact same conditions used in functional assays, both types of fibroblasts (spread and sorted) were transferred into plastic dishes (Falcon, #353047) in DMEM medium (HyClone, #SH30243.01) in 20% O₂, as these culture conditions are compatible with co-culture with CD4+ CD25+ T lymphocytes which are applied for *in vitro* functional assays. Using these protocols, 7 different CAF-S1 cell lines from 7 different patients have been isolated, 3 by sorting and 4 by spreading. To avoid any *in vitro* activation, these CAF-S1 primary cell lines isolated by sorting and spreading were used no later than passage 5. Moreover, in each experiment, properties of spread and sorted cells were compared at the same passages.

*Treg - CAF-S1 clusters functional assays:* 5x10⁴ CAF-S1 cells (spread and sorted) were plated on 24-well plates (Falcon, #353047) in DMEM (HyClone, #SH30243.01) with 10% FBS (Biosera, # FB-1003/500) at 1.5% O₂ overnight for complete adherence. The medium was then removed and 5x10⁵ CD4⁺ *CD25⁺* T lymphocytes were added in 500µl of DMEM 1% FBS (ratio 1-10) and incubated overnight (for RNA analysis) or for 24h (for FACS) at 37°C, 20% O₂. For FACS analysis, non-adherent cells (CD4⁺ CD25⁺) were then harvested, washed and stained for 30 min at RT using the following markers: Live Dead (1:1000, BD Bioscience, #562247), anti-CD45-BUV395 (1:50, BD Biosciences, #BD-563792), anti-CD4-APC (1:50, Miltenyi Biotec, #130-092-374), anti-CD25 PE-cy7 (1:33, BD Bioscience, #557741), anti-FOXP3-FITC (1:33, ebioscience, #53-4776-42), anti-CTLA-4-Pe-cy5 (1:20, BD Bioscience,# 555854), anti-PD-1-BUV737 (BD Bioscience,# 565299), anti-TIGIT-BV605 (1:50, BD Bioscience, # 747841), anti-LAG3-BV510 (1:50, BD Bioscience, #744985), anti-TIM3-BV711 (1:50, BD Bioscience, #565566). Adherent cells were trypsinized, washed and stained for CAF-S1 clusters markers (ANTXR1, CD9, SDC1, LAMP5, GPC3, DLK1) in addition to Live Dead NIR to remove dead cells and CD45 to remove remaining Tregs. Cells (Treg Panel and CAF-S1 panel) were acquired using ZE5 cell analyzer (Bio-Rad) and analyzed by Flowjo v10.4.2. For RNA analysis, non-adherent cells (CD4⁺ CD25⁺) were harvested by pipetting, and spin down. RNA was then extracted using Single Cell RNA Purification Kit (Norgen Biotek corp., #51800) according to the manufacturer's recommendations. RNA integrity and quality were analyzed using the Agilent RNA 6000 Pico kit (Agilent Technologies, #5067-1513). cDNA libraries were prepared using the TruSeq RNA Exome kit (Illumina, #20020189) followed by sequencing on NovaSeq (illumina). Reads were mapped on the human reference genome (hg38; Gencode release 29) and quantified using STAR (version 2.6.1a) with parameters "outFilterMultimapNmax = 20; alignSJoverhangMin = 8; alignSJDBoverhangMin = 1; outFilterMismatchNmax = 999; outFilterMismatchNoverLmax = 0.04; alignIntronMin = 20; alignIntronMax = 1000000; alignMatesGapMax = 1000000; outMultimapperOrder = Random". Only genes with one read in at least 5% of all samples were kept for further analyses. Normalization was conducted with DESeq2 R package.

*Treg - CAF-S1 cluster intracellular staining:* 5x10⁴ CAF-S1 cells (spread) were plated on 24-well plates (Falcon, #353047) in DMEM (HyClone, #SH30243.01) with 10% FBS (Biosera, # FB-1003/500) at 1.5% O₂ overnight for complete adherence. The medium was then removed and 5x10⁵ CD4⁺ CD25⁺T lymphocytes were added in 500µl of DMEM 1% FBS (ratio 1-10) and incubated overnight at 37°C, 20% O₂. Non-adherent cells (CD4⁺ CD25⁺) were then harvested, washed and stained for 30 min at RT with Live Dead (1:1000, BD Bioscience, #562247),after washing, the cells were divided in 2 groups (one fixed and permeabilized and the second kept without fixation / permeabilization except for FOXP3 staining) and stained using the following markers: anti-CD45-BUV395 (1:50, BD Biosciences, #BD-563792), anti-CD4-APC (1:50, Miltenyi Biotec, #130-092-374), anti-CD25 PE-cy7 (1:33, BD Bioscience, #557741), anti-CTLA-4-Pe-cy5 (1:20, BD Bioscience,# 555854), anti-PD-1-BUV737 (BD Bioscience,# 565299), anti-TIGIT-BV605 (1:50, BD Bioscience, # 747841), anti-LAG3-BV510 (1:50, BD Bioscience, #744985), anti-TIM3-BV711 (1:50, BD Bioscience, #565566) anti-FOXP3-FITC (1:33, ebioscience, #53-4776-42)

### Comparison of fibroblasts from normal healthy tissue with CAF-S1 from BC

Primary fibroblasts were collected by using the spreading method (see above) from juxta-tumors, i.e. tissues defined as heathy by referent Pathologists. Juxta-tumors were cut into small pieces, put in plastic dishes (Falcon, #353003) and cultured in DMEM (HyClone, #SH30243.01) supplemented with 10% FBS (Biosera, #FB-1003/500), streptomycin (100 µg/ml) and penicillin (100 U/ml) (Gibco #15140122) for 2-3 weeks at 37ºC. Spread fibroblasts were next analyzed at early and late passages (passages 2 and 5, respectively) for verifying expression of CAF-S1 markers. Primary cells were trypsinized, resuspended in PBS and stained with LIVE/DEAD^{™} Fixable Aqua Dead Cell Stain dye (ThermoFisher Scientific, #L34957) diluted in PBS for 20 min at RT and fixed in PFA 4% for 20 min at RT. After a rapid wash in PBS+, the cells were stained with an anti-FAP antibody (1:100, R&D Systems, #MAB3715) or isotype control (1:100, R&D Systems, #MAB002) in PBS+ for 40 min at RT. Both antibody and isotype control were coupled using fluorescent dye Zenon APC Mouse IgG1 labeling kit (Thermo Fisher Scientific, #Z-25051). Cells were acquired using LSR FORTESSA analyzer (BD biosciences). 50 000 events per sample were recorded.

### RNA sequencing from NSCLC samples

Formalin Fixed Paraffin Embedded (FFPE) biopsies (N = 120) from NSCLC naïve from any treatment were processed for RNA extraction using high FFPET RNA Isolation Kit (Roche, #06650775001) following the manufacturer's instructions. RNA integrity and quality were analyzed using the Agilent RNA 6000 Pico kit (Agilent Technologies, #5067-1513). Samples with a DV200 higher than 40% were chosen for RNA sequencing (N = 70). cDNA library was prepared using the Nextera XT Sample Preparation kit (Illumina, #FC-131-10) followed by sequencing on NovaSeq (illumina). Reads were mapped on the human reference genome (release hg19/GRCh37) and quantified using STAR (version 2.5.3a) with parameters "outFilterMultimapNmax = 20; alignSJoverhangMin = 8; alignSJDBoverhangMin = 1; outFilterMismatchNmax = 999; outFilterMismatchNoverLmax = 0.04; alignIntronMin = 20; alignIntronMax = 1000000; alignMatesGapMax = 1000000; outMultimapperOrder = Random". Only genes with one read in at least 5% of all samples were kept for further analyses. Normalization, unsupervised analysis (PCA) and differential analysis between responders and non-responders patients were conducted with DESeq2 R package.

### Statistical analysis

All statistical analyses and graphical representation of data were performed in the R environment (https://cran.r-project.org, Versions 3.5.3) or using GraphPad Prism software (version 8.1.1). Statistical tests used are in agreement with data distribution: Normality was first checked using the Shapiro-Wilk test and parametric or non-parametric two-tailed tests were applied according to normality, as indicated in each Figure legend. scRNA-seq data presented in **Figs. 1****,** **2****,** **3****,** **Fig.** 7 and **Fig.** 8 were analyzed using *Seurat* R package (version 3.0). Correlation matrix shown in **Figs.** 2 and 4 were computed using *cor* function from *stats* R package with method = "pearson" and use = "pairwise.complete.obs". *Corrplot R* function was used for the clustering and the visualization of the correlation matrix with following parameters: *order =* "hclust" and *hclust.method =* "ward.D2". Quantifications from FACS analysis shown in Fig. 5 are shown using mean ± s.e.m. Differential analysis between CD4⁺ *CD25⁺* T cells cultured alone or in presence of ecm-myCAF in Fig. 10 were conducted using DESeq2 R package. Gene Set Enrichment Analysis (GSEA) software version 3.0 (Broad Institute) was used in Fig. 6. For melanoma RNA-seq data, the following parameters were applied: Enrichment statistic = 'weighted', Metric for ranking genes = 'Signal2Noise'. For NSCLC RNA-seq data, GSEAPreranked was used with log2 fold change from DESeq2 differential analysis as metric for ranking genes and 'classic' mode for enrichment score.

**Table 1. Description of prospective cohorts of BC**

| | | **Prospective cohorte** | | |
|---|---|---|---|---|
| | | **1 ssRNAseq - BC Patients** | **2 FACS - BC Patients** | **3 Primary cell lines - BC Patients** |
| **Number of cases** | | 8 | 44 | 7 |
| **Age at surgery** | **< 50 years** | 3 (37.5%) | 7 (15.9%) | 3 (42.9%) |
| | **> or = 50 years** | 5 (62.5%) | 37 (84.1%) | 4 (57.1%) |
| **Multifocality** | **no** | 6 (75%) | 37 (84.1%) | 4 (57.1%) |
| | **yes** | 2 (25%) | 7 (15.9%) | 2 (28.6%) |
| | **NA** | 0 (0%) | 0 (0%) | 1 (14.3%) |
| **Histological type** | **ductal** | 6 (75%) | 33 (75%) | 5 (71.4%) |
| | **lobular** | 0 (0%) | 4 (9.1%) | 0 (0%) |
| | **other** | 2 (25%) | 7 (15.9%) | 2 (28.6%) |
| **Grade** | **G1** | 0 (0%) | 9 (20.5%) | 0 (0%) |
| | **G2** | 6 (75%) | 18 (40.9%) | 5 (71.4%) |
| | **G3** | 2 (25%) | 16 (36.4%) | 2 (28.6%) |
| | **NA** | 0 (0%) | 1 (2.3%) | 0 (0%) |
| **Tumor size** | **pT1** | 4 (50%) | 21 (47.7%) | 4 (57.1%) |
| | **pT2** | 2 (25%) | 18 (40.9%) | 3 (42.9%) |
| | **pT3** | 2 (25%) | 4 (9.1%) | 0 (0%) |
| | **NA** | 0 (0%) | 1 (2.3%) | 0 (0%) |
| **Lymph node metastases** | **pN0** | 4 (50%) | 29 (65.9%) | 4 (57.1%) |
| | **pN1** | 3 (37.5%) | 11 (25%) | 1 (14.3%) |
| | **pN2** | 0 (0%) | 3 (6.8%) | 2 (28.63%) |
| | **pN3** | 1 (12.5%) | 1 (2.3%) | 0 (0%) |
| | **NA** | 0 (0%) | 0 (0%) | 0 (0%) |
| **Nottingham Prognostic Index** | **excellent** | 0 (0%) | 0 (0%) | 0 (0%) |
| | **good** | 2 (25%) | 15 (34.1%) | 2 (28.6%) |
| | **moderate** | 5 (62.5%) | 26 (59.1%) | 5 (71.4%) |
| | **poor** | 1 (12.5%) | 2 (4.5%) | 0 (0%) |
| | **NA** | 0 (0%) | 1 (2.3%) | 0 (0%) |
| **Estrogen receptor (RO)** | **no** | 2 (25%) | 9 (20.5%) | 1 (14.3%) |
| | **yes** | 6 (75%) | 34 (77.3%) | 6 (85.7%) |
| | **NA** | 0 (0%) | 1 (2.3%) | 0 (0%) |
| **Progesterone receptor (RP)** | **no** | 2 (25%) | 13 (29.5%) | 3 (42.9%) |
| | **yes** | 6 (75%) | 30 (68.2%) | 4 (57.1%) |
| | **NA** | 0 (0%) | 1 (2.3%) | 0 (0%) |
| **HER2 amplification/ overexpression** | **no** | 8 (100%) | 39 (88.6%) | 7 (100%) |
| | **yes** | 0 (0%) | 4 (9.1%) | 0 (0%) |
| | **NA** | 0 (0%) | 1 (2.3%) | 0 (0%) |
| **Surgical treatment** | **lumpectomy** | 4 (50%) | 26 (59.1%) | 5 (71.4%) |
| | **mastectomy** | 4 (50%) | 18 (40.9%) | 2 (28.6%) |
| **Hormone therapy** | **no** | 2 (25%) | 9 (20.4%) | 1 (14.3%) |
| | **yes** | 6 (75%) | 35 (79.5%) | 6 (85.7%) |
| | **NA** | 0 (0%) | 0 (0%) | 0 (0%) |
| **Adjuvant chemotherapy** | **no** | 2 (25%) | 22 (50%) | 3 (42.9%) |
| | **yes** | 6 (75%) | 21 (47.7%) | 4 (57.1%) |
| | **NA** | 0 (0%) | 1 (2.3%) | 0 (0%) |
| **Radiotherapy** | **no** | 0 (0%) | 9 (20.5%) | 0 (0%) |
| | **yes** | 8 (100%) | 33 (75%) | 7 (100%) |
| | **NA** | 0 (0%) | 2 (4.5%) | 0 (0%) |
| **Trastuzumab** | **no** | 8 (100%) | 41 (93.2%) | 7 (100%) |
| | **yes** | 0 (0%) | 3 (6.8%) | 0 (0%) |
| | **NA** | 0 (0%) | 0 (0%) | 0 (0%) |
| **Relapse** | **no** | 8 (100%) | 43 (97.7%) | 7 (100%) |
| | **yes** | 0 (0%) | 1 (2.3%) | 0 (0%) |
| **Survival** | **alive** | 8 (100%) | 44 (100%) | 7 (100%) |
| | **deceased** | 0 (0%) | 0 (0%) | 0 (0%) |

NSCLC samples were from the routine diagnostic samples stored in the Pathology Department of Bichat Hospital, from patients treated by immuno-oncology drugs. All patients received 2nd or 3rd line immunotherapy first cycle, according to drug registrations at that period of time and on-going clinical trials, from 28 July 2015 to 20 Feb. 2018. Efficacy of immune-oncology treatment was assessed every 8 to 12 weeks by whole body CT-scan, according to RECIST v.1.1 criteria. All clinical data were retrieved from patients electronic files. Histological subtypes of non-small cell lung cancer samples were determined according to the current World Health Organization 2015 classification, on formalin-fixed paraffin-embedded tissue sections, stained with haematoxylin, from bronchial endoscopy or CT-guided transthoracic biopsy samples. Squamous-Cell Cancer diagnosis was supported by p40 positive and TTF-1 (thyroid transcription factor 1) negative immunostaining. Non-squamous lung cancer included both adenocarcinoma showing Alcian blue positive staining (for mucin tumor cell content) and/or nuclear TTF-1 positive immunostaining, and large-cell carcinoma devoid of mucin secretion, with p40- and TTF-1-negative immunostaining. Two samples exhibited mixt features of squamous and adenocarcinoma differentiation. The two stage IIIB patients, according to the 8th TNM staging system, had non resectable lung tumor, and have contra-indication to radiation therapy. *PD-L1 staining was performed and interpreted by AG, on 4-µm paraffin-embedded sections, from diagnosis, pre-treatment biopsy samples, containing at least 200 tumor cells, using Cell Signaling Technology E1L3N commercially available clone, on the Leica Bond platform. **EOCG (Eastern Cooperative Oncology Group) performance status was assessed at time of first immunotherapy cycle according to Oken et al., Am J Clin Oncol 1982;5(6):649-55.

**Table 2. description of the retrospective cohort of NSCLC patient treated by immunotherapy (cohort 4).**

| | | **Patients** |
|---|---|---|
| **Number of cases** | | 70 |
| **Mean Age (year) (min-max)** | | 62.5 (40-83) |
| **Gender (%)** | **Female** | 20 (28.6%) |
| | **Male** | 50 (71.4%) |
| **Tumor type (%)** | **non-Squamous cell carcinoma** | 47 (67.1%) |
| | **Squamous cell carcinoma** | 21 (30.0%) |
| | **Adenocarcinoma / squamous** | 2 (2.9%) |
| **Stage (%)** | **IIIB** | 2 (2.9%) |
| | **IV** | 68 (97.1%) |
| **Smoking status (%)** | **Active smokers** | 37 (52.9%) |
| | **Former smokers** | 29 (41.4%) |
| | **Never smokers** | 4 (5.7%) |
| **Pack-Years (mean) (min-max)** | | 41.15 (0-150) |
| **Metastasis sites (%)** | **Brain** | 23 (32.9%) |
| | **Liver** | 9 (12.9%) |
| | **Bone** | 27 (38.6%) |
| | **Suprarenal gland** | 20 (28.6%) |
| | | |
| **nb. of metastastic sites (%)** | **0** | 2 (2.9%) |
| | **1** | 33 (47.1) |
| | **≥2** | 35 (50.0) |
| **PD-L1 staining (%)*** | **≥50%** | 30 (42.9) |
| | **1-49%** | 21 (30.0) |
| | **<1%** | 14 (20.0) |
| | **Not done** | 5 (7.1) |
| **i.o. drug (%)** | **Nivolumab** | 56 (80.0) |
| | **Pembrolizumab** | 5 (7.1) |
| | **Nivo+ipilumumab** | 8 (11.4) |
| | **Nivo+chemo** | 1(1.4) |
| **line of treatment** | **1L** | 20 (28.6) |
| | **2L** | 37 (52.9) |
| | **>3L** | 13 (18.6) |
| **ECOG performance status**** | **PS= 0-1** | 43 (61.4) |
| | **PS=2-3** | 27 (38.6) |

## Claims

1. An *in vitro* method for detecting immunosuppressive fibroblasts in a cancer sample from a subject suffering from cancer, wherein the method comprises detecting Anthrax Toxin Receptor 1 (ANTXR1) and Fibroblast Activation Protein (FAP) positive fibroblasts in the cancer sample from said patient, the presence of ANTXR1⁺FAP⁺ fibroblasts being indicative of immunosuppressive CAFs.

2. An *in vitro* method for predicting a response of a subject suffering from cancer to an immunotherapy treatment, wherein the method comprises detecting ANTXR1⁺FAP⁺ fibroblasts in a cancer sample from said patient, wherein ANTXR1⁺FAP⁺ fibroblasts in the cancer sample are predictive of the responsiveness of said patient to an immunotherapeutic agent.

3. An immune checkpoint inhibitor for use in the treatment of a cancer in a patient, wherein the patient presents a cancer sample having (a) low number or percentage of ANTXR1⁺FAP⁺ fibroblasts; or (b) no ANTXR1⁺FAP⁺ fibroblasts, and wherein the percentage of ANTXR1⁺FAP⁺ fibroblasts and the percentage of ANTXR1⁺FAP⁺ fibroblasts is preferably the number of ANTXR1⁺FAP⁺ fibroblasts on the total number of fibroblasts in the cancer sample or on the total number of cells in the cancer sample.

4. The method according to claim 2 or the immune checkpoint inhibitor for use according to claim 3, wherein the ANTXR1⁺FAP⁺ fibroblasts are selected from the group consisting of FAP⁺ ANTXR1⁺ CAFs, FAP⁺ ANTXR1⁺ LAMP5⁻ SDC1⁺ fibroblasts, preferably CAFs; FAP⁺ ANTXR1⁺ LAMP5⁺ SDC1^{+/-} fibroblasts, preferably CAFs; and FAP⁺ANTXR1⁺ SDC1⁻ LAMP5⁻ CD9⁺ fibroblasts, preferably CAFs.

5. In vitro use of ANTXR1 as biomarker for the identification of immunosuppressive FAP⁺CAFs.

6. In vitro use of ANTXR1 in FAP⁺CAFs populations, as biomarker in a tumor of a subject suffering from cancer, for predicting the response of the subject to an immunotherapeutic agent.

7. A FAP inhibitor for use in the treatment of a cancer in a patient, wherein the patient has ANTXR1⁺FAP⁺ fibroblasts, preferably CAFs, in a tumor sample from the patient; and wherein the FAP inhibitor is selected in the group consisting of talabostat, PT-100, linagliptin (Tradjenta), FAP inhibitors with a *N*-(4-quinolinoyl)-Gly-(2-cyanopyrrolidine) scaffold, such as FAPI-02, FAPI-04 and FAPI-46, a peptide-targeted radionuclide such as FAP-2286 and any combination thereof.

8. The FAP inhibitor for use according to claim 7, for use in combination with an immunotherapeutic agent.

9. The FAP inhibitor for use according to claim 7, for use in combination with radiotherapy, or chemotherapy.

10. Product or kit comprising a) a FAP inhibitor according to claim 7 and b) an immunotherapeutic agent, as a combined preparation for simultaneous, separate or sequential use in the treatment of a cancer in a patient, wherein the patient has ANTXR1⁺FAP⁺ fibroblasts, preferably CAFs, in a tumor sample from the patient.

11. The method according to any one of claims 2 and 4, the use according to claim 6, the FAP inhibitor for use according to claim 8 or the product or kit according to claim 10, wherein the immunotherapeutic agent is selected from the group consisting of therapeutic agents that stimulate the patient's immune system to attack the malignant tumor cells, immunization of the patient with tumoral antigens, molecules stimulating the immune system such as cytokines, therapeutic antibodies, adoptive T cell therapy, CAR cell therapy, immune checkpoint inhibitor, and any combination thereof, preferably an immune checkpoint inhibitor.

12. The method according to claim 11, the use according to claim 11, the FAP inhibitor for use according to claim 11, the product or kit according to claim 11, or the immune checkpoint inhibitor for use according to claim 3 or 4, wherein the checkpoint inhibitor is selected from the group consisting of an antibody directed against cytotoxic T lymphocyte associated protein (CTLA-4), programmed cell death protein 1 (PD-1), programmed cell death ligand (PD-L1), T cell immunoreceptor with Ig and ITIM domains (TIGIT), Lymphocyte-activation gene 3 (LAG-3), T-cell immunoglobulin and mucin-domain containing-3 (TIM-3), B- and T-lymphocyte attenuator (BLTA), IDO1, or any combination thereof, preferably selected from the group consisting of an antibody directed against CTLA-4, PD-1, PD-L1 and TIGIT or any combination thereof, more preferably an antibody directed against PD-1 or CTLA-4 and the combination thereof.

13. The method according to claim 11, the use according to claim 11, the FAP inhibitor for use according to claim 11, the product or kit according to claim 11, or the immune checkpoint inhibitor for use according to claim 3 or 4, wherein the immunotherapeutic agent is selected from the group consisting of ipilimumab, nivolumab, BGB-A317, pembrolizumab, atezolizumab, avelumab, or durvalumab, BMS-986016, and epacadostat, or any combination thereof.

14. The method according to any one of claims 2, 4 and 11-13, the immune checkpoint inhibitor for use according to any one of claims 3-4 and 11-13, the use according to any one of claims 6 and 11-13,the FAP inhibitor for use according to any one of claims 7-9 and 11-13, or the product or kit according to any one of claims 10-13, wherein the cancer is selected from the group consisting of prostate cancer, lung cancer, Non-Small Cell Lung Carcinoma (NSCLC), breast cancer, gastric cancer, kidney cancer, ovarian cancer, hepatocellular cancer, osteosarcoma, melanoma, hypopharynx cancer, esophageal cancer, endometrial cancer, cervical cancer, pancreatic cancer, liver cancer, colon or colorectal cancer, adenocarcinoma, sarcoma, non-Squamous cell carcinoma, Squamous cell carcinoma, neuroendocrine tumors, muscle cancer, adrenal cancer, thyroid cancer, uterine cancer, skin cancer, melanoma, metastatic melanoma, bladder cancer, head and neck cancer, preferably the cancer is selected from the group consisting of head and neck cancer, breast cancer, ovary cancer, NSCLC, melanoma and metastatic melanoma, more preferably the cancer is selected from the group consisting of head and neck cancer, NSCLC, melanoma and metastatic melanoma.

## Patentansprüche

1. Ein *in* vitro-Verfahren zum Nachweis immunsuppressiver Fibroblasten in einer Krebsprobe von einem an Krebs leidenden Subjekt, wobei das Verfahren das Nachweisen Anthrax Toxin Rezeptor 1 (ANTXR1) und Fibroblasten-Aktivierungsprotein (FAP) positive Fibroblasten in der Krebsprobe des Patienten umfasst, wobei das Vorhandensein von ANTXR1⁺FAP⁺ Fibroblasten ein Indikator für immunsuppressive CAFs ist.

2. Ein *in* vitro-Verfahren zur Vorhersage einer Antwort eines an Krebs leidenden Subjekts auf eine Immuntherapie-Behandlung, wobei das Verfahren das Nachweisen von ANTXR1⁺FAP⁺ Fibroblasten in einer Krebsprobe von dem Patienten umfasst, wobei ANTXR1⁺FAP⁺ Fibroblasten in der Krebsprobe prädiktiv für die Responsivität des Patienten auf ein immuntherapeutisches Mittel sind.

3. Ein Immun-Checkpoint-Inhibitor zur Verwendung in der Behandlung eines Krebs in einem Patienten, wobei der Patient eine Krebsprobe darstellt, die (a) eine geringe Anzahl oder Prozentsatz von ANTXR1⁺FAP⁺ Fibroblasten oder (b) keine ANTXR1⁺FAP⁺ Fibroblasten aufweist und wobei der Prozentsatz an ANTXR1⁺FAP⁺ Fibroblasten und der Prozentsatz an ANTXR1⁺FAP⁺ Fibroblasten bevorzugt die Anzahl der ANTXR1⁺FAP⁺ Fibroblasten auf die Gesamtzahl der Fibroblasten in der Krebsprobe oder auf die Gesamtzahl der Zellen in der Krebsprobe ist.

4. Das Verfahren nach Anspruch 2 oder der Immun-Checkpoint-Inhibitor zur Verwendung nach Anspruch 3, wobei die ANTXR1⁺FAP⁺ Fibroblasten ausgewählt sind aus der Gruppe bestehend aus FAP⁺ ANTXR1⁺ CAFs, FAP⁺ ANTXR1⁺ LAMP5⁻ SDC1⁺ Fibroblasten, bevorzugt CAFs; FAP⁺ ANTXR1⁺ LAMP5⁺ SDC1^{+/-} Fibroblasten, bevorzugt CAFs und FAP⁺ ANTXR1⁺ SDC1⁻ LAMP5⁻ CD9⁺ Fibroblasten, bevorzugt CAFs.

5. *In vitro*-Verwendung von ANTXR1 als Biomarker zur Identifizierung immunsuppressiver FAP⁺ CAFs.

6. *In vitro*-Verwendung von ANTXR1 in FAP⁺ CAFs Populationen als Biomarker in einem Tumor eines an Krebs leidenden Subjekts, zur Vorhersage der Antwort des Subjekts auf ein immuntherapeutisches Mittel.

7. Ein FAP-Inhibitor zur Verwendung in der Behandlung eines Krebs in einem Patienten, wobei der Patient ANTXR1⁺FAP⁺ Fibroblasten, bevorzugt CAFs hat, in einer Tumorprobe des Patienten und wobei der FAP-Inhibitor ausgewählt ist aus der Gruppe bestehend aus Talabostat, PT-100, Linagliptin (Tradjenta), FAP-Inhibitoren mit einem N-(4-quinolinoyl)-Gly-(2-cyanopyrrolidin)-Gerüst, wie beispielsweise FAPI-02, FAPI-04 und FAPI-46, ein Peptid-zielgerichtetes Radionuklid, wie beispielsweise FAP-2286 und jede Kombination davon.

8. Der FAP-Inhibitor zur Verwendung nach Anspruch 7 zur Verwendung in Kombination mit einem immuntherapeutischen Mittel.

9. Der FAP-Inhibitor zur Verwendung nach Anspruch 7 zur Verwendung in Kombination mit Radiotherapie oder Chemotherapie.

10. Produkt oder Kit umfassend a) ein FAP-Inhibitor nach Anspruch 7 und b) ein immuntherapeutisches Mittel als eine kombinierte Zubereitung zur gleichzeitigen, separaten oder aufeinanderfolgenden Verwendung in der Behandlung eines Krebs in einem Patienten, wobei der Patient ANTXR1⁺FAP⁺ Fibroblasten, bevorzugt CAFs, in einer Tumorprobe des Patienten hat.

11. Das Verfahren nach einem der Ansprüche 2 und 4, zur Verwendung nach Anspruch 6, der FAP-Inhibitor zur Verwendung nach Anspruch 8, oder das Produkt oder Kit nach Anspruch 10, wobei das immuntherapeutische Mittel ausgewählt ist aus der Gruppe bestehend aus therapeutischen Mittel, die das Immunsystem des Patient stimulieren die malignen Tumorzellen zu attackieren, Immunisierung des Patienten mit Tumor-Antigenen, das Immunsystem stimulierende Moleküle, wie beispielsweise Cytokine, therapeutische Antikörper, Adoptive T-Zell-Therapie , CAR Zell-Therapie, Immun-Checkpoint-Inhibitor und jede Kombination davon, bevorzugt ein Immun-Checkpoint-Inhibitor.

12. Das Verfahren nach Anspruch 11, die Verwendung nach Anspruch 11, der FAP-Inhibitor nach Anspruch 11, das Produkt oder Kit nach Anspruch 11, oder der Immun-Checkpoint-Inhibitor zur Verwendung nach Anspruch 3 oder 4, wobei der Checkpoint-Inhibitor ausgewählt ist aus der Gruppe bestehend aus einem Antikörper gerichtet gegen Zytotoxisches T-Lymphozyten-assoziiertes Protein (CTLA-4), Programmed Cell Death Protein (PD-1), Programmed Cell Death Ligand (PD-L1), T-Zell-Immunrezeptor mit Ig und ITIM Domänen (TIGIT), Lymphozytenaktivierungsgen 3 (LAG-3), T-Zell-Immunglobulin und Mucin-Domäne enthaltend 3 (TIM-3), B- und T-Lymphozyten-Attenuator (BLTA), IDO1 oder jede Kombination davon, bevorzugt ausgewählt aus der Gruppe bestehend aus einem Antikörper gerichtet gegen CTLA-4, PD-1, PD-L1 und TIGIT oder jede Kombination davon, mehr bevorzugt ausgewählt aus einem Antikörper gerichtet gegen PD-1 oder CTLA-4 und Kombination davon.

13. Das Verfahren nach Anspruch 11, die Verwendung nach Anspruch 11, der FAP-Inhibitor zur Verwendung nach Anspruch 11, das Produkt oder Kit nach Anspruch 11, oder der Immun-Checkpoint-Inhibitor zur Verwendung nach Anspruch 3 oder 4, wobei das immuntherapeutische Mittel ausgewählt ist aus der Gruppe bestehend aus Ipilimuimab, Nivolumab, BGB-A317, Pembrolizumab, Atezolizumab, Avelumab oder Durvalumab, BMS-986016 und Epacadostat oder jede Kombination davon.

14. Das Verfahren nach einem der Ansprüche 2, 4 und 11 bis 13, der Immun-Checkpoint Inhibitor zur Verwendung nach einem der Ansprüche 3 bis 4 und 11 bis 13, die Verwendung nach einem der Ansprüche 6 und 11 bis 13, der FAP-Inhibitor zur Verwendung nach einem der Ansprüche 7 bis 9 und 11 bis 13, oder das Produkt oder Kit nach einem der Ansprüche 10 bis 13, wobei der Krebs ausgewählt ist aus der Gruppe bestehend aus Prostatakrebs, Lungenkrebs, nicht-kleinzelliges Lungenkarzinom (NSCLC), Brustkrebs, Magenkrebs, Nierenkrebs, Eierstockkrebs, Leberzellkarzinom, Osteosarkom, Melanom, Hypopharynxkarzinom, Ösophaguskarzinom, Endometriumkrebs, Gebärmutterhalskrebs, Pankreaskrebs, Leberkrebs, Darm- oder Dickdarmkrebs, Adenokarzinom, Sarkom, Nicht-Plattenepithelkarzinom, Plattenepithelkarzinom, neuroendokrine Tumoren, Muskelkrebs, Nebennierenkrebs, Schilddrüsenkrebs, Gebärmutterkrebs, Hautkrebs, Melanom, metastasierendes Melanom, Blasenkrebs, Kopf-Hals-Krebs, bevorzugt wird der Krebs ausgewählt aus der Gruppe bestehend aus Kopf-Hals-Krebs, Brustkrebs, Eierstockkrebs, NSCLC, Melanom und metastasierendem Melanom, mehr bevorzugt ist der Krebs ausgewählt aus der Gruppe bestehend aus Kopf-Hals-Krebs, NSCLC, Melanom und metastasierendem Melanom.

## Revendications

1. Méthode *in vitro* pour détecter des fibroblastes immunosuppresseurs dans un échantillon cancéreux provenant d'un sujet souffrant d'un cancer, laquelle méthode comprend la détection de fibroblastes positifs au récepteur 1 de la toxine de l'anthrax (ANTXR1) et à la protéine d'activation des fibroblastes (FAP) dans l'échantillon cancéreux provenant dudit patient, la présence de fibroblastes ANTXR1⁺FAP⁺ étant indicative de CAF immunosuppresseurs.

2. Méthode *in vitro* pour prédire la réponse d'un sujet souffrant d'un cancer à un traitement par immunothérapie, laquelle méthode comprend la détection de fibroblastes ANTXR1⁺FAP⁺ dans un échantillon cancéreux provenant dudit patient, dans lequel des fibroblastes ANTXR1⁺FAP⁺ dans l'échantillon cancéreux sont prédictifs de la réponse dudit patient à un agent immunothérapeutique.

3. Inhibiteur de point de contrôle immunitaire pour une utilisation dans le traitement d'un cancer chez un patient, dans lequel le patient présente un échantillon cancéreux (a) ayant un faible nombre ou pourcentage de fibroblastes ANTXR1⁺FAP⁺ ; ou (b) n'ayant pas de fibroblastes ANTXR1⁺FAP⁺, et dans lequel le pourcentage de fibroblastes ANTXR1⁺FAP⁺ est de préférence le nombre de fibroblastes ANTXR1⁺FAP⁺ par rapport au nombre total de fibroblastes dans l'échantillon cancéreux ou par rapport au nombre total de cellules dans l'échantillon cancéreux.

4. Méthode selon la revendication 2 ou inhibiteur de point de contrôle immunitaire pour une utilisation selon la revendication 3, dans lequel les fibroblastes ANTXR1⁺FAP⁺ sont choisis dans le groupe constitué par les CAF FAP⁺ ANTXR1⁺, les fibroblastes, de préférence les CAF, FAP⁺ ANTXR1⁺ LAMP5⁻ SDC1⁺ ; les fibroblastes, de préférence les CAF, FAP⁺ ANTXR1⁺ LAMP5⁺ SDC1^{+/-} ; et les fibroblastes, de préférence les CAF, FAP⁺ ANTXR1⁺ SDC1⁻ LAMP5⁻ CD9⁺.

5. Utilisation *in vitro* d'ANTXR1 en tant que biomarqueur pour l'identification de CAF FAP⁺ immunosuppresseurs.

6. Utilisation *in vitro* d'ANTXRI dans des populations de CAF FAP⁺, en tant que biomarqueur dans une tumeur d'un sujet souffrant d'un cancer, pour prédire la réponse du sujet à un agent immunothérapeutique.

7. Inhibiteur de FAP pour une utilisation dans le traitement d'un cancer chez un patient, dans lequel le patient a des fibroblastes, de préférence des CAF, ANTXR1⁺FAP⁺, dans un échantillon tumoral provenant du patient ; et lequel inhibiteur de FAP est choisi dans le groupe constitué par le talabostat, PT-100, la linagliptine (Tradjenta), les inhibiteurs de FAP ayant une charpente de *N*-(4-quinolinoyl)-Gly-(2-cyanopyrrolidine), tels que FAPI-02, FAPI-04 et FAPI-46, un radionucléide ciblant un peptide tel que FAP-2286, et l'une quelconque de leurs combinaisons.

8. Inhibiteur de FAP pour une utilisation selon la revendication 7, pour son utilisation en combinaison avec un agent immunothérapeutique.

9. Inhibiteur de FAP pour une utilisation selon la revendication 7, pour son utilisation en combinaison avec une radiothérapie ou une chimiothérapie.

10. Produit ou trousse comprenant a) un inhibiteur de FAP selon la revendication 7 et b) un agent immunothérapeutique, sous la forme d'une préparation combinée pour une utilisation simultanée, séparée ou séquentielle dans le traitement d'un cancer chez un patient, dans lequel le patient a des fibroblastes, de préférence des CAF, ANTXR1⁺FAP⁺, dans un échantillon tumoral provenant du patient.

11. Méthode selon l'une quelconque des revendications 2 et 4, utilisation selon la revendication 6, inhibiteur de FAP pour une utilisation selon la revendication 8, ou produit ou trousse selon la revendication 10, dans lequel l'agent immunothérapeutique est choisi dans le groupe constitué par les agents thérapeutiques qui stimulent le système immunitaire du patient afin qu'il attaque les cellules tumorales malignes, l'immunisation du patient avec des antigènes tumoraux, les molécules stimulant le système immunitaire telles que les cytokines, les anticorps thérapeutiques, une thérapie adoptive par cellules T, une thérapie par cellules CAR, un inhibiteur de point de contrôle immunitaire, et l'une quelconque de leurs combinaisons, de préférence un inhibiteur de point de contrôle immunitaire.

12. Méthode selon la revendication 11, utilisation selon la revendication 11, inhibiteur de FAP pour une utilisation selon la revendication 11, produit ou trousse selon la revendication 11, ou inhibiteur de point de contrôle immunitaire pour une utilisation selon la revendication 3 ou 4, dans lequel l'inhibiteur de point de contrôle est choisi dans le groupe constitué par un anticorps dirigé contre la protéine associée aux lymphocytes T cytotoxiques (CTLA-4), la protéine 1 de mort cellulaire programmée (PD-1), le ligand de mort cellulaire programmée (PD-L1), l'immunorécepteur des cellules T avec domaines Ig et ITIM (TIGIT), le gène 3 d'activation des lymphocytes (LAG-3), l'immunoglobuline des cellules T et protéine à domaine mucine-3 (TIM-3), l'atténuateur des lymphocytes B et T (BTLA), IDO1, ou l'une quelconque de leurs combinaisons, de préférence choisi dans le groupe constitué par un anticorps dirigé contre CTLA-4, PD-1, PD-L1 et TIGIT ou l'une quelconque de leurs combinaisons, de manière préférée un anticorps dirigé contre PD-1 ou CTLA-4 et leur combinaison.

13. Méthode selon la revendication 11, utilisation selon la revendication 11, inhibiteur de FAP pour une utilisation selon la revendication 11, produit ou trousse selon la revendication 11, ou inhibiteur de point de contrôle immunitaire pour une utilisation selon la revendication 3 ou 4, dans lequel l'agent immunothérapeutique est choisi dans le groupe constitué par l'ipilimumab, le nivolumab, BGB-A317, le pembrolizumab, l'atézolizumab, l'avélumab, et le durvalumab, BMS-986016, et l'épacadostat, ou l'une quelconque de leurs combinaisons.

14. Méthode selon l'une quelconque des revendications 2, 4 et 11 à 13, inhibiteur de point de contrôle immunitaire pour une utilisation selon l'une quelconque des revendications 3, 4 et 11 à 13, utilisation selon l'une quelconque des revendications 6 et 11 à 13, inhibiteur de FAP pour une utilisation selon l'une quelconque des revendications 7 à 9 et 11 à 13, ou produit ou trousse selon l'une quelconque des revendications 10 à 13, dans lequel le cancer est choisi dans le groupe constitué par un cancer de la prostate, un cancer du poumon, un carcinome pulmonaire non à petites cellules (NSCLC), un cancer du sein, un cancer gastrique, un cancer rénal, un cancer ovarien, un cancer hépatocellulaire, un ostéosarcome, un mélanome, un cancer de l'hypopharynx, un cancer de l'œsophage, un cancer de l'endomètre, un cancer du col, un cancer du pancréas, un cancer du foie, un cancer du côlon ou colorectal, un adénocarcinome, un sarcome, un carcinome non à cellules squameuses, un carcinome à cellules squameuses, les tumeurs neuroendocriniennes, un cancer musculaire, un cancer surrénal, un cancer de la thyroïde, un cancer de l'utérus, un cancer de la peau, un mélanome, un mélanome métastasique, un cancer de la vessie, un cancer de la tête et du cou, de préférence le cancer est choisi dans le groupe constitué par un cancer de la tête et du cou, un cancer du sein, un cancer ovarien, un NSCLC, un mélanome et un mélanome métastasique, mieux encore le cancer est choisi dans le groupe constitué par un cancer de la tête et du cou, un NSCLC, un mélanome et un mélanome métastasique.
